# EUROPÄISCHE PATENTSCHRIFT

(11) **EP 3 881 802 B1**
(45) Veröffentlichungstag und Bekanntmachung des Hinweises auf die Patenterteilung: **11.05.2022**
(21) Anmeldenummer: 20164541.3
(22) Anmeldetag: 20.03.2020
(51) Int. Cl.: A61F 2/30, A61F 2/38, F16F 5/00, F16K 99/00

(54) **VORRICHTUNG UND VERFAHREN ZUR HERSTELLUNG VON KNIESPACER-KOMPONENTEN**
DEVICE AND METHOD FOR PRODUCING KNEE SPACER COMPONENTS
DISPOSITIF ET PROCÉDÉ DE FABRICATION DE COMPOSANTS DE L'ESPACEUR DE GENOU

(43) Veröffentlichungstag der Anmeldung: 22.09.2021
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- JP-A- 2018 118 056
- US-A1- 2010 101 989
- US-A1- 2010 102 484
- US-A1- 2016 139 094

## Beschreibung

Die Erfindung betrifft eine Vorrichtung zum Herstellen einer Kniespacer-Komponente eines Kniespacers, also einer Femur-Komponente oder einer Tibia-Komponente, durch Aushärten von Knochenzementteig, wie in Ansprüchen 1-9 beschrieben.

Der Kniespacer beziehungsweise die Tibia-Komponente und die Femur-Komponente sind als temporärer Platzhalter dazu vorgesehen, im medizinischen Anwendungsbereich ein Kniegelenk oder einen Teil eines Kniegelenks umfassend eine artikulierende Oberfläche des Kniegelenks temporär zu ersetzen. Bevorzugt sind die Tibia-Komponente und die Femur-Komponente zum temporären Ersetzen eines Kniegelenks geeignet und vorgesehen. Die Erfindung betrifft auch ein Verfahren zur Herstellung einer Kniespacer-Komponente eines Kniespacers, das heißt einer Femur-Komponente oder einer Tibia-Komponente, mit einer solchen Vorrichtung, wie in Ansprüchen 10-15 beschrieben.

Gegenstand der Erfindung sind somit eine Vorrichtung und ein Verfahren zur Herstellung von Kniespacer-Komponenten. Die Vorrichtung ist zur Fertigung von Kniespacer-Komponenten durch das OP-Personal vor dem Einsetzen des Kniespacers, also vor der eigentlichen Operation bestimmt. Unter dem Begriff Kniespacer-Komponenten werden die Tibia-Komponente und die Femur-Komponente von artikulierenden Kniespacern verstanden.

Kniegelenk-Endoprothesen werden in großem Umfang weltweit implantiert. Leider kommt es in einem geringen Prozentsatz vor, dass Kniegelenk-Endoprothesen von mikrobiellen Keimen, besonders Gram-positiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Gelenk-Endoprothesen erreichen. Bei einer Besiedlung von Gelenk-Endoprothesen mit mikrobiellen Keimen wird das umliegende Knochen- und Weichgewebe mit infiziert und von den mikrobiellen Keimen geschädigt. Stand der Technik sind vor allem zwei Behandlungsverfahren für infizierte Gelenk-Endoprothesen, die einzeitige septische Revision und die zweizeitige septische Revision. Bei der einzeitigen Revision wird innerhalb einer OP zuerst die infizierte Gelenk-Endoprothesen entfernt, anschließend radikal debridiert und dann wird eine Revisions-Gelenk-Endoprothese implantiert.

Bei zweizeitigen septischen Revisionen wird in einer ersten OP zuerst die infizierte Kniegelenk-Endoprothese entfernt, dann wird debridiert und anschließend erfolgt die Implantation eines Kniespacers. Ein Kniespacer hat eine Tibia-Komponente und eine Femur-Komponente und ist grundsätzlich den Kniegelenk-Endoprothesen in Form und Größe nachgebildet. Die Tibia-Komponente weist einen Schaft und ein Tibia-Plateau auf, wobei das Tibia-Plateau eine Gleitfläche beziehungsweise Abrollfläche des Kniegelenks bildet und der Schaft in der Tibia verankert werden kann. Die Femur-Komponente weist einen Schaft zur Verankerung im Femur und zwei Kondylen zum Abrollen auf dem Tibia-Plateau auf. Die Tibia-Komponente und die Femur-Komponente werden mit Knochenzement an dem jeweiligen Knochen verankert, also beispielsweise bei der Femur-Komponente am distalen Femur beziehungsweise im Femurkanal verankert und bei der Tibia-Komponente mit dem Schaft an der proximalen Tibia beziehungsweise im Tibiakanal verankert. Der Kniespacer verbleibt bis zu mehreren Wochen im Patienten bis die Entzündung abgeklungen ist und die klinischen Entzündungsmarker zurückgegangen sind. Dann wird in einer zweiten OP der Kniespacer entfernt und nach erneutem Debridement eine Revisions-Kniegelenk-Endoprothese implantiert.

Bei Kniespacer-Komponenten werden dem Zementpulver vor der eigentlichen Kniespacerherstellung Antibiotika zugesetzt. Mit diesem antibiotisch modifizierten Knochenzementpulver wird anschließend durch Beimischen von Monomerflüssigkeit ein Knochenzementteig hergestellt und aus diesem Knochenzementteig werden Kniespacer-Komponenten gegossen, die dann durch Polymerisation mit Hilfe der dem Zementpulver zugesetzten Monomerflüssigkeit aushärten. Der Knochenzementteig schließt dabei die Antibiotika im Wesentlichen ein. Die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab.

In der WO 2016/205077 A1 und der US 8 900 322 B2 werden Kniespacer mit einer Spülfunktion beschrieben. Es ist ferner bekannt, mit Antibiotika ausgerüstete Kniespacer zu verwenden. Kniespacer können PMMA-Knochenzementpulver, Antibiotika und Monomerflüssigkeit hergestellt werden, zum Beispiel mit einer Spacerform, wie sie beispielsweise in den Patenten DE 10 2015 104 704 B4, US 7 789 646 B2, US 8 801 983 B2 oder EP 2 617 393 B1 beschrieben sind. Andererseits ist es auch üblich, industriell aus Knochenzement vorgefertigte Kniespacer einzusetzen.

Das Patent EP 2 931 180 B1 offenbart eine Zementform zur Herstellung einer Tibia-Komponente eines Kniespacers, der zur Abgabe von Antibiotika geeignet ist. Das Patent EP 3 143 963 B1 offenbart eine dreiteilige Spacerform, mit einer Tibia-Spacerform, einer Femur-Spacerform und einer dritten Form zur Herstellung einer, einen intramedullären Kanal füllenden Komponente.

Aus der US 10 071 511 B2 sind Gießformen für die Herstellung von Tibia-Komponenten von Kniespacern bekannt. Mit diesen Gießformen könne unterschiedlich hohe Tibia-Komponenten gefertigt werden. Die Gießform besteht aus einem Unterteil und einem Oberteil, welche einen Hohlraum bilden, in den ein Polymethylmethacrylat-Knochenzementteig über einen Port eingepresst werden kann. Die Höhe der zu gießenden Tibiakomponente wird durch einen Rastmechanismus mit Zähnen definiert, der den Abstand zwischen dem Unterteil und dem Oberteil der Gießform festlegt.

Ein ähnliches Konzept ist aus der US 9 433 506 B2 bekannt, die eine Form für einen Kniespacer beansprucht. Dabei enthält die Gießform einen Port, der nach der Entformung im intramedullären Raum als Schaft vorliegt. Die Gießform wird über den Port mit Hilfe einer Zementkartusche befüllt.

US2010/102484 offenbart eine Vorrichtung zum Herstellen einer Kniespacer-Komponente durch Aushärten von Knochenzementteig, aufweisend eine Gießform zum Formen der Kniespacer-Komponente. In die Gießform mündet eine Öffnung durch die der Zement eingespritzt werden kann; während sich der eingespritzte Zement in der Gießform befindet, kann ein Stopfen oder eine andere Art von Einsatz vorgesehen werden, um die Öffnung zu schließen, nachdem das härtbare Material injiziert wurde.

Die Aufgabe der vorliegenden Erfindung besteht also darin, die Nachteile des Stands der Technik zu überwinden. Insbesondere besteht die Aufgabe der Erfindung in der Entwicklung einer kostengünstigen Vorrichtung zum Herstellen einer Kniespacer-Komponente eines Kniespacers durch Aushärten von Knochenzementteig und in der Entwicklung eines einfach und kostengünstig durchführbaren Verfahrens zum Herstellen einer Kniespacer-Komponente eines Kniespacers durch Aushärten von Knochenzementteig, mit dem vom medizinischen Personal im OP unter Verwendung von Knochenzementteig, insbesondere von Polymethylmethacrylat-Knochenzement, einteilige Kniespacer-Komponenten, also Tibia-Komponenten und Femur-Komponenten, hergestellt werden können. Tibia-Komponenten und Femur-Komponenten sind ähnlich aufgebaut. Sie bestehen aus einem Schaft und einem Kopf oder einem Plateau.

Aufgabe der Erfindung ist es auch, eine Vorrichtung zu entwickeln, mit der vom medizinischen Personal Kniespacer-Komponenten intraoperativ unter Verwendung von Knochenzementteig, insbesondere von Polymethylmethacrylat-Knochenzementteig (PMMA-Knochenzementteig) hergestellt werden können. Der grundsätzliche Aufbau der zu entwickelnden Vorrichtung soll prinzipiell gleich sein für die Tibia-Komponente als auch für die Femur-Komponente von Kniespacern. Die Vorrichtung soll daher in einer ersten Variante zur Herstellung der Tibia-Komponente und in einer zweiten Variante für die Herstellung der Femur-Komponente von Kniespacern geeignet sein. Es soll möglich sein, sowohl mit Polymethylmethacrylat-Knochenzementteig normaler Viskosität als auch mit hoher Viskosität die Kniespacer-Komponenten der Kniespacer herzustellen.

Für die vollständige Füllung einer Gießform mit einem hochviskosem Knochenzementteig ist ein hoher Einpressdruck erforderlich. Die zu entwickelnde Vorrichtung soll deshalb so beschaffen sein, dass (Polymethylmethacrylat-)Knochenzementteig aus einer Knochenzementkartusche in die Vorrichtung eingepresst werden kann. Bei Verwendung von nicht hochviskosem Knochenzementteig darf nach vollständiger Füllung der Gießform der Knochenzementteig nicht aus dem Anguss ausfließen. Dazu ist es notwendig, die Gießform so zu gestalten, dass ein Auslaufen auch von niedriger-viskosem Knochenzementteig aus der Gießform während der Trennung der Gießform von der Knochenzementkartusche sicher verhindert wird. Dieses Verschließen soll möglich sein, ohne dass in der Wandung der Gießform Öffnungen für komplex aufgebaute Ventile notwendig sind. Öffnungen in der Wandung der Gießform können zur Undichtigkeit der Gießform führen, wenn der Knochenzementteig unter hohem Druck in die Gießform eingepresst wird. Weiterhin soll der Angussbereich der Gießform so gestaltet sein, dass einerseits eine leichte Befüllung der Gießform mit Knochenzementteig möglich ist und dass andererseits nach erfolgter Aushärtung des Knochenzementteigs die Angussreste leicht entfernt werden können.

Weiterhin soll es in einer Ausgestaltungsform der Vorrichtung möglich sein, Kniespacer-Komponenten auch ohne die Verwendung von Knochenzementkartuschen mit (Polymethylmethacrylat-)Knochenzementteig herzustellen, der in geeigneten Mischschüsseln manuell gemischt wird.

Die Aufgaben der Erfindung werden gelöst durch eine Vorrichtung zum Herstellen einer Kniespacer-Komponente durch Aushärten von Knochenzementteig, wobei die Kniespacer-Komponente dazu vorgesehen ist, im medizinischen Bereich einen Teil eines Kniegelenks umfassend eine artikulierende Oberfläche des Kniegelenks temporär zu ersetzen, die Vorrichtung aufweisend
eine Gießform zum Formen der Kniespacer-Komponente aus Knochenzementteig;
einen Ventilsitz, der mit der Gießform verbunden ist, wobei der Ventilsitz eine bereichsweise geschlossene Kopfseite mit mindestens einer ersten Durchführung aufweist, wobei die mindestens eine erste Durchführung in die Gießform hineinmündet;
einen Ventilkörper, der drehbar gegen den Ventilsitz gelagert ist und der eine Dichtfläche aufweist, wobei die Dichtfläche in Richtung der bereichsweise geschlossenen Kopfseite des Ventilsitzes ausgerichtet ist, wobei in der Dichtfläche mindestens eine zweite Durchführung angeordnet ist;
wobei der Ventilsitz und der Ventilkörper zusammen ein Ventil bilden, wobei das Ventil durch Drehen des Ventilkörpers gegen den Ventilsitz reversibel in eine geöffnete Stellung und reversibel in eine geschlossene Stellung überführbar ist, wobei in der geöffneten Stellung des Ventils die mindestens eine erste Durchführung des Ventilsitzes und die mindestens eine zweite Durchführung des Ventilkörpers zumindest bereichsweise übereinanderliegen und eine für Knochenzementteig durchlässige Verbindung durch das Ventil bereitstellen, wobei in der geschlossenen Stellung des Ventils die mindestens eine erste Durchführung des Ventilsitzes durch die Dichtfläche des Ventilkörpers abgedeckt ist, wobei die Gießform in der geschlossenen Stellung des Ventils für Knochenzementteig flüssigkeitsdicht verschlossen ist.

Flüssigkeitsdicht bedeutet, dass der nicht ausgehärtete also fluide Knochenzementteig und bevorzugt auch eine flüssige Monomerflüssigkeit als Ausgangskomponente des Knochenzements nicht durch die Verbindung am Anschluss ausfließen oder hindurchdringen können.

Für Knochenzementteig abgedeckt bedeutet, dass der Knochenzementteig im Ventil zumindest derart stark am Fließen gehindert wird, dass er nicht durch das Ventil hindurchfließen kann, bevor er aushärtet. Hierzu reicht es für normal viskose Knochenzementteige bereits aus, wenn der Knochenzementteig nicht in gerader Linie durch das Ventil strömen kann und die freien Leitungsquerschnitte kleiner als 1 mm sind. Knochenzementteige sind viskose oder hochviskose Fluide, wie durch den Zusatz "Teig" angedeutet. Die Viskosität eines Knochenzementteigs beträgt mindestens 10 Pa s, was der Viskosität von flüssigem Honig entspricht. Zudem härtet der Knochenzementteig innerhalb von wenigen Minuten aus, so dass anschließend kein Durchtritt mehr möglich ist. Es kann bevorzugt vorgesehen sein, dass der Knochenzementteig eine Viskosität von mindestens 10 Pa s aufweist.

Unter einem Knochenzementteig beziehungsweise einem fluiden Knochenzementteig ist ein gemischter (also zum Einsatz bereiter) Knochenzementteig zu verstehen, der eine zähflüssige Konsistenz hat. Die Viskosität eines Knochenzements entspricht vorzugsweise der von Honig oder hat eine noch zähflüssigere, das heiß höhere Viskosität. Die Begriffe fluider Knochenzement und Knochenzementteig werden synonym verwendet.

Bevorzugt ist die Gießform innen hohl.

Es kann vorgesehen sein, dass die Dichtfläche bis auf die mindestens eine zweite Durchführung geschlossen ist.

Es kann bevorzugt auch vorgesehen sein, dass die Gießform einen Innenraum aufweist, der eine Negativform eines Tibia-Plateaus oder von Kondylen nachbildet.

Es kann auch vorgesehen sein, dass der Ventilsitz flüssigkeitsundurchlässig mit einer Gießformwand der Gießform verbunden ist.

Ferner kann vorgesehen sein, dass der Ventilsitz an einer Stirnseite eines durch die Gießform begrenzten Hohlraums als Scheibe ausgebildet ist, insbesondere als ebene Scheibe ausgebildet ist.

Bevorzugt kann auch vorgesehen sein, dass der Ventilsitz und der Ventilkörper hohlzylinderförmig sind.

Es kann ferner vorgesehen sein, dass die Gießform zweiteilig oder mehrteilig ist, wobei vorzugsweise die Teile der Gießform ineinander schiebbar sind. Besonders bevorzugt ist die Gießform zweiteilig.

Die Gießform soll einem Druck von 10 N/cm² standhalten, um auch hochviskosen Knochenzement verwenden zu können.

In der vorliegenden Patentanmeldung werden die Richtungsbezeichnungen "proximal", "distal" und "lateral" sowie die Ebenen-bezeichnungen "Sagittalebene", "Frontalebene" und "Transversalebene" in Bezug auf den Kniespacer, die Kniespacer-Komponenten und die Gießform so verwendet, wie dies bei dem im Patienten eingesetzten Zustand als anatomische Hauptrichtung oder als Körperebene zu verstehen wäre. Dabei bedeutet "proximal" zum Körperzentrum hin und "distal" vom Körperzentrum entfernt.

Der Schaft ist zur Verbindung mit einem Knochen (im Fall von Tibia-Komponenten mit der Tibia und im Fall von Femur-Komponenten mit dem Femur) vorgesehen und ist vorzugsweise zu diesem Zweck in ein proximales oder distales Ende des präparierten Knochens beziehungsweise in den Knochenkanal einführbar.

Bevorzugt kann vorgesehen sein, dass die Vorrichtung zur Herstellung einer Kniespacer-Komponente eines Kniespacers zur Anwendung zumindest eines antibiotischen und/oder antimykotischen Wirkstoffs geeignet ist.

Bevorzugt ist die Kniespacer-Komponente einteilig aus einem biokompatiblen Knochenzementteig, wie Polymethylmethacrylat (PMMA), zu fertigen, wobei besonders bevorzugt das PMMA wenigsten ein aus dem PMMA lösbares Antibiotikum und/oder Antimykotikum enthält.

Es kann bevorzugt vorgesehen sein, dass die bereichsweise geschlossene Kopfseite des Ventilsitzes und die Dichtfläche des Ventilkörpers Scheiben sind oder scheibenförmig sind.

Es kann bevorzugt vorgesehen sein, dass der Ventilsitz die Gießform begrenzt.

Die Begriffe "geöffneter Zustand" und "geschlossener Zustand" des Ventils beziehungsweise des Ventilkörpers relativ zum Ventilsitz und die Begriffe "geöffnete Stellung" und "geschlossene Stellung" des Ventils beziehungsweise des Ventilkörpers relativ zum Ventilsitz werden synonym verwendet.

Es kann vorgesehen sein, dass der Ventilsitz nicht verdrehbar gegen die Gießform mit der Gießform verbunden ist, bevorzugt der Ventilsitz fest und/oder starr mit der Gießform verbunden ist oder einteilig mit der Gießform ausgeführt ist.

Hierdurch kann auf konstruktiv einfache und damit kostengünstige Weise eine Abdichtung beziehungsweise eine flüssigkeitsdichte Verbindung des Ventilsitzes gegen die Gießform erreicht werden.

Ferner kann vorgesehen sein, dass das Ventil manuell bedienbar ist, bevorzugt manuell von außerhalb der Vorrichtung manuell bedienbar ist, wobei besonders bevorzugt der Ventilkörper manuell gegen den Ventilsitz drehbar ist und durch die Drehung das Ventil von der geschlossenen Stellung in die geöffnete Stellung und von der geöffneten Stellung in die geschlossene Stellung überführbar ist.

Hierdurch kann das Ventil der Vorrichtung bequem von außen bedient werden, um eine Knochenzementkartusche zu wechseln oder diese zu lösen.

Des Weiteren kann vorgesehen sein, dass die mindestens eine erste Durchführung des Ventilsitzes in der geschlossenen Stellung des Ventils mit der Dichtfläche des Ventilkörpers abgedeckt ist, wobei bevorzugt die bereichsweise geschlossene Kopfseite des Ventilsitzes und die Dichtfläche des Ventilkörpers maximal 2 mm voneinander beabstandet sind, besonders bevorzugt maximal 1 mm voneinander beabstandet sind, ganz besonders bevorzugt maximal 0,5 mm voneinander beabstandet sind.

Hierdurch kann sichergestellt werden, dass der in die Gießform eingefüllte Knochenzementteig (der fluide Knochenzement) nicht wieder aus der Gießform durch das Ventil herausgedrückt werden kann, wenn das Ventil geschlossen ist. Wenn der Knochenzementteig mit diesen Dicken beziehungsweise mit diesen Querschnitten im Bereich des Angusses aushärtet, kann er nach dem Aushärten des Spacers leicht manuell abgebrochen oder durchgeschnitten werden und muss nicht mit einer Säge aufgetrennt werden. Daher sind Angüsse mit solchen Dicken unschädlich, da sie den OP-Betrieb während einer OP nicht nennenswert aufhalten.

Gemäß einer bevorzugten Weiterbildung der vorliegenden Erfindung kann vorgesehen sein, dass der Ventilkörper um eine Drehachse gegen den Ventilsitz drehbar gelagert ist, wobei die Drehachse senkrecht zur Dichtfläche des Ventilkörpers verläuft oder wobei die Drehachse entlang einer Rotationssymmetrieachse der Dichtfläche des Ventilkörpers verläuft.

Hiermit wird erreicht, dass der durch das Ventil fließende Knochenzementteig von dem Ventilkörper durch die Drehung abgeschnitten beziehungsweise abgedreht werden kann. Dies ermöglicht eine glatte Schnittfläche und einen geringen Kraftaufwand beim Abscheren. Ferner kann auch eine Drehung der Knochenzementkartusche zum Abscheren des Knochenzementteigs verwendet werden. Es wird bevorzugt, dass die Drehachse entlang der Rotationssymmetrieachse der Dichtfläche des Ventilkörpers verläuft. Wenn die Drehachse senkrecht zur Dichtfläche des Ventilkörpers verläuft, kann das Ventil nach Art eines Zapfhahns (beispielsweise für Bier) aufgebaut sein.

Auch kann vorgesehen sein, dass der Ventilkörper einen Anschluss zum flüssigkeitsdichten Verbinden einer Knochenzementkartusche oder eines Griffstücks der Vorrichtung aufweist oder mit einem solchen Anschluss fest verbunden ist.

Ferner kann vorgesehen sein, dass der Ventilkörper den Anschluss zum flüssigkeitsdichten Verbinden einer Knochenzementkartusche aufweist oder mit einem solchen Anschluss fest verbunden ist.

Hierdurch kann der Ventilkörper mit Hilfe einer angeschlossenen Knochenzementkartusche bedient oder mit dem Griffstück bedient werden.

Dabei kann vorgesehen sein, dass die Vorrichtung ein Adapterelement aufweist, das mit einer Knochenzementkartusche verbunden ist oder verbindbar ist, wobei das Adapterelement lösbar und formschlüssig mit dem Anschluss verbunden oder verbindbar ist, so dass ein Innenraum einer Knochenzementkartusche über das Adapterelement für Knochenzementteig durchlässig mit der mindestens einen zweiten Durchführung im Ventilkörper verbunden oder verbindbar ist.

Hierdurch wird erreicht, dass der Knochenzementteig aus der Knochenzementkartusche problemlos durch das Ventil in seiner geöffneten Stellung hindurch in die Gießform eingefüllt werden kann.

Ferner kann dabei vorgesehen sein, dass der Anschluss zum flüssigkeitsdichten Verbinden einer Knochenzementkartusche oder eines Griffstücks ein Innengewinde im Ventilkörper oder ein Außengewinde am Ventilkörper umfasst, wobei vorzugsweise ein Adapterelement der Knochenzementkartusche oder an der Knochenzementkartusche ein zu dem Innengewinde oder zu dem Außengewinde passendes Gegengewinde aufweist oder das Griffstück der Vorrichtung ein zu dem Innengewinde oder zu dem Außengewinde passendes Gegengewinde aufweist.

Hierdurch kann zum Einen eine stabile und flüssigkeitsdichte Verbindung am Anschluss hergestellt werden und zum anderen kann die Drehung bei der Schraubbewegung genutzt werden, um zu Beginn oder nach Ende der Schraubbewegung den Ventilkörper gegen den Ventilsitz zu drehen und somit das Ventil vom geöffneten in den geschlossenen Zustand zu überführen oder um das Ventil vom geschlossenen in den geöffneten Zustand zu überführen.

Durch die Verwendung geeigneter Gewinde kann insbesondere eine zusätzliche Sicherungsfunktion der Vorrichtung dadurch erzielt werden, dass ein Lösen einer Knochenzementkartusche oder des Griffstücks nur bei geschlossenem Ventil möglich ist und ein Öffnen des Ventils nur bei angeschlossener Knochenzementkartusche oder angeschlossenem Griffstück möglich ist.

Es kann vorgesehen sein, dass das Innengewinde im Ventilkörper oder das Außengewinde am Ventilkörper ein Rechtsgewinde ist und das Ventil durch eine gleichsinnige Rechtsdrehung des Ventilkörpers von der geschlossenen in die geöffnete Stellung überführbar ist und das Ventil durch eine gegensinnige Linksdrehung des Ventilkörpers von der geöffneten in die geschlossene Stellung überführbar ist oder
das Innengewinde im Ventilkörper oder das Außengewinde am Ventilkörper ein Linksgewinde ist und das Ventil durch eine gleichsinnige Linksdrehung des Ventilkörpers von der geschlossenen in die geöffnete Stellung überführbar ist und das Ventil durch eine gegensinnige Rechtsdrehung des Ventilkörpers von der geöffneten in die geschlossene Stellung überführbar ist oder
das Innengewinde des Ventilsitzes und das Innengewinde und das Außengewinde des Ventilkörpers alle Linksgewinde oder alle Rechtsgewinde sind, wobei vorzugsweise auch ein Außengewinde eines Adapterelements oder ein Außengewinde des Griffstücks zum flüssigkeitsdichten Verbinden einer Knochenzementkartusche an den Anschluss den gleichen Drehsinn hat.

Auch diese Maßnahmen zielen darauf ab, dass ein Schließen des Ventils beim Ausschrauben der Knochenzement-Kartusche oder des Griffstücks automatisch erfolgt und ein Öffnen des Ventils bei Einschrauben der Knochenzement-Kartusche oder des Griffstücks automatisch erfolgt.

Es kann auch vorgesehen sein, dass der Ventilkörper um eine Drehachse gegen den Ventilsitz drehbar gelagert ist, wobei die Drehachse in Richtung der mindestens einen ersten Durchführung ausgerichtet ist.

Es kann ferner vorgesehen sein, dass sich der Ventilkörper um einen Winkel von maximal 280° gegen den Ventilsitz verdrehen lässt, bevorzugt von maximal 180°, besonders bevorzugt von maximal 100° gegen den Ventilsitz verdrehen lässt, ganz besonders bevorzugt von bis zu 90° gegen den Ventilsitz verdrehen lässt.

Bevorzugt können jeweils zwei Durchführungen in dem Ventilsitz und in dem Ventilkörper angeordnet sein, wobei zwei Durchführungen bevorzugt um 180° versetzt um den Mittelpunkt von Scheiben des Ventilsitzes und des Ventilkörpers angeordnet sind, wobei die Scheiben die bereichsweise geschlossene Kopfseite des Ventilsitzes und die Dichtfläche des Ventilkörpers bilden.

Des Weiteren kann vorgesehen sein, dass die Vorrichtung eine Knochenzementkartusche zum Mischen von Knochenzement-Ausgangskomponenten und zum Austragen von gemischtem Knochenzementteig aus der Knochenzementkartusche heraus aufweist, bevorzugt eine Knochenzementkartusche zum Mischen von Polymethylmethacrylat-Knochenzement-Ausgangskomponenten und zum Austragen von gemischtem Polymethylmethacrylat-Knochenzementteig aus der Knochenzementkartusche heraus aufweist, wobei besonders bevorzugt die Knochenzementkartusche die Knochenzement-Ausgangskomponenten zur Herstellung des Knochenzementteigs in voneinander getrennten Bereichen beinhaltet.

Hierdurch wird die Vorrichtung weiter komplettiert, da mit der Vorrichtung dann auch der Knochenzementteig bereitgestellt werden kann, der zum Ausformen der Kniespacer-Komponente des Kniespacers in die Gießform gefüllt wird.

Alternativ zur Knochenzementkartusche kann auch vorgesehen sein, dass die Vorrichtung einen Monomerflüssigkeitsbehälter enthaltend Monomerflüssigkeit, ein Knochenzementpulver (vorzugsweise ein abgepacktes Knochenzementpulver) und einen Mischbecher zum manuellen Herstellen und Mischen eines Knochenzementteigs aus der Monomerflüssigkeit und dem Knochenzementpulver aufweist, wobei besonders bevorzugt die Vorrichtung zusätzlich einen Mischspatel aufweist.

Auch kann vorgesehen sein, dass die Vorrichtung ein Griffstück aufweist, das mit dem Ventilkörper verbindbar ist und mit dem der Ventilkörper gegen den Ventilsitz drehbar ist, wobei bevorzugt das Griffstück einen Hohlraum zur Aufnahme von Knochenzementteig aufweist, der mit der mindestens einen zweiten Durchführung flüssigkeitsdurchlässig verbunden ist.

Hiermit kann das Ventil mit Hilfe des Griffstücks manuell vom geschlossenen in den geöffneten Zustand überführt werden.

Bevorzugt kann auch vorgesehen sein, dass die Summe aller freien Öffnungen der mindestens einen ersten Durchführung in der geschlossenen Kopfseite höchstens so groß ist wie die geschlossene Oberfläche der Kopfseite und dass die Summe aller freien Öffnungen der mindestens einen zweiten Durchführung in der Dichtfläche höchstens so groß ist wie die geschlossene Oberfläche der Dichtfläche.

Hierdurch wird sichergestellt, dass ein stabiles und für den Knochenzementteig undurchlässiges Verschließen des Ventils durch Drehen des Ventilkörpers gegen den Ventilsitz möglich ist.

Es kann auch vorgesehen sein, dass der Ventilsitz ein Innengewinde an der Innenseite aufweist und der Ventilkörper ein passendes Außengewinde an der Außenseite aufweist, so dass der Ventilkörper in den Ventilsitz schraubbar ist.

Durch diese Maßnahme kann eine hohe Dichtwirkung an der Verbindung zwischen dem Ventilkörper und dem Ventilsitz erreichet werden. Zudem kann das Ventil so einfach und kostengünstig zusammengebaut werden.

Ferner kann vorgesehen sein, dass die Gießform eine trogförmige Form mit einem Hohlraum und einen Stempel aufweist, wobei der Stempel in den Hohlraum der trogförmigen Form einsetzbar oder eingesetzt ist und der Stempel in dem Hohlraum axial verschiebbar ist, und wobei vorzugsweise die trogförmige Form einen Hohlraumboden aufweist, wobei der Hohlraumboden die Kontur einer oder mehrerer artikulierender Gleitflächen der Kniespacer-Komponente bildet.

Hierdurch kann die Kniespacer-Komponente durch Anpressen des Knochenzementteigs in der Gießform mit Hilfe des Stempels geformt werden. Zudem wird so die Möglichkeit eröffnet, die Höhe der Kniespacer-Komponente variable einzustellen.

Vorteilhaft kann dabei vorgesehen sein, dass die Unterseite des Stempels konkav geformt ist. Hierdurch kann Luft aus der Gießform in Richtung eines Spalts zwischen dem Stempel und der Form entweichen.

Dabei kann vorgesehen sein, dass der Ventilsitz in dem Stempel angeordnet ist, vorzugsweise am Ende eines einen Schaft der Kniespacer-Komponente formenden Teils des Stempels angeordnet ist.

Hiermit kann ein überschüssiger Knochenzementteig aus der Gießform beim Einpressen des Stempels durch den Stempel herausgedrückt werden. Hierdurch lässt sich die Höhe der Kniespacer-Komponente einstellen.

Ein Schaft beziehungsweise eine Schaftform ist nicht notwendig. Die Kniespacer-Komponente kann auch ohne Schaft ausgeführt werden. Der das Ventil beziehungsweise Anguss oder Abguss können dabei direkt auf der Ebene auf einer Seite der Tibia-Plattform angeordnet sein.

Bevorzugt kann auch vorgesehen sein, dass die mindestens eine erste Durchführung in der bereichsweise geschlossenen Kopfseite die gleiche Größe und Gestalt hat wie die mindestens eine zweite Durchführung in der Dichtfläche.

Ebenfalls bevorzugt kann vorgesehen sein, dass die mindestens eine erste Durchführung in der bereichsweise geschlossenen Kopfseite zwei erste Durchführungen sind und die mindestens eine zweite Durchführung in der Dichtfläche zwei zweite Durchführungen sind, wobei bevorzugt die zwei ersten Durchführungen in gegenüberliegend angeordneten Kreissegmentvierteln bezüglich der Drehachse des Ventilkörpers im Ventilsitz angeordnet sind und die zwei zweiten Durchführungen in gegenüberliegend angeordneten Kreissegmentvierteln bezüglich der Drehachse des Ventilkörpers in der Dichtfläche angeordnet sind.

Durch diese beiden Maßnahmen kann ein ausreichender Strömungsquerschnitt für den zähflüssigen Knochenzementteig bereitgestellt werden und es können einseitige Belastungen des Ventils vermieden werden, die ansonsten zu einer Undichtigkeit des Ventils führen könnten.

Mit der vorliegenden Erfindung wird auch vorgeschlagen, dass an der Dichtfläche des Ventilkörpers ein Bund angeordnet ist, der sich auf einem Rand des Ventilsitzes abstützt oder an der bereichsweise geschlossenen Kopfseite des Ventilsitzes ein Bund angeordnet ist, der sich auf einem Rand des Ventilkörpers abstützt.

Hierdurch kann eine stabile Führung des Ventilkörpers am Ventilsitz erreicht werden. Ferner kann dadurch exakt die Position der mindestens einen zweiten Durchführung bei gegebener Gewindelänge des Ventilkörpers bezüglich der mindestens einen ersten Durchführung definiert werden.

Des Weiteren kann vorgesehen sein, dass an dem Ventilkörper ein Griff befestigt oder befestigbar ist, der zumindest eine radiale Erstreckung bezüglich der Drehachse des Ventilkörpers aufweist, wobei vorzugsweise der Griff an einer der Dichtfläche gegenüberliegenden Seite des Ventilkörpers befestigt oder befestigbar ist, wobei besonders bevorzugt sich der Ventilkörper maximal um 90° gegen den Ventilsitz drehen lässt.

Hiermit kann das Ventil bequem von außen manuell bedient werden. Mit diesem Griff kann der Ventilkörper von der geöffneten Stellung in die geschlossene Stellung des Ventils gedreht werden.

Ferner kann vorgesehen sein, dass der Ventilkörper und der Ventilsitz aus Kunststoff gefertigt sind, insbesondere aus thermoplastischem Kunststoff gefertigt sind, wobei bevorzugt der Ventilsitz mit der Gießform einteilig ausgeführt ist.

Hierdurch ist das Ventil und damit die Vorrichtung kostengünstig und als hygienisches Wegwerfprodukt zu fertigen.

Bevorzugt kann auch vorgesehen sein, dass ein Rastelement an der Dichtfläche des Ventilkörpers angeordnet ist und an der Kopfseite des Ventilsitzes ein Gegenrastelement angeordnet ist, wobei das Rastelement mit dem Gegenrastelement in Eingriff bringbar ist, wobei das Rastelement am Ventilkörper so positioniert ist, dass ein Drehen oder ein Herausdrehen des Ventilkörpers aus dem Ventilsitz auf einen Drehwinkel von maximal 90° begrenzt ist, wenn der Ventilkörper maximal in den Ventilsitz eingesteckt oder eingeschraubt ist.

Hiermit kann sichergestellt werden, dass der Ventilkörper im eingerasteten Zustand nicht mehr als 90° gegen den Ventilsitz gedreht wird. Dadurch kann verhindert werden, dass der Ventilkörper zu weit aus dem Ventilsitz herausgedreht wird und dadurch ein zu großer Spalt zwischen dem Ventilkörper und dem Ventilsitz entsteht und das Ventil auch in einer geschlossenen Stellung für den Knochenzementteig durchlässig wird.

Des Weiteren kann vorgesehen sein, dass die Gießform zweitteilig oder mehrteilig ist, wobei zwischen zumindest zwei der Teile der zusammengesetzten Gießform ein Spalt vorhanden ist, durch den Luft oder Gas aus dem Inneren der Gießform entweichen kann.

Hiermit wird verhindert, dass Lufteinschlüsse in der Gießform zurückbleiben, die die Oberfläche der aus dem Knochenzementteig hergestellten Kniespacer-Komponente beeinträchtigen könnten.

Auch kann vorgesehen sein, dass die Vorrichtung einen Auffangbehälter aufweist, wobei das Ventil auf der von der Gießform abgewandten Seite mit dem Auffangbehälter zur Aufnahme von durch das Ventil in der geöffneten Stellung aus der Gießform austretenden überschüssigem Knochenzementteig verbunden oder verbindbar ist oder das Ventil einteilig mit dem Auffangbehälter ausgeführt ist.

Hierdurch kann verhindert werden, dass austretender Knochenzementteig den OP-Saal verschmutzt und kontaminiert.

Die der vorliegenden Erfindung zugrundeliegenden Aufgaben werden auch gelöst durch ein Verfahren zur Herstellung einer Kniespacer-Komponente zum temporären Ersetzen eines Teils eines Kniegelenks umfassend eine artikulierende Oberfläche des Kniegelenks, wobei das Verfahren mit einer erfindungsgemäßen Vorrichtung durchgeführt wird, das Verfahren aufweisend die folgenden chronologischen Schritte:
A) Flüssigkeitsdichtes Verbinden einer Knochenzementkartusche mit der Vorrichtung;
B) Einpressen von Knochenzementteig aus der Knochenzementkartusche durch das Ventil in der geöffneten Stellung hindurch in die Gießform;
C) Drehen des Ventilkörpers gegen den Ventilsitz und dadurch Überführen des Ventils in die geschlossene Stellung und Abscheren des Knochenzementteigs an der mindestens einen ersten Durchführung in der bereichsweise geschlossenen Kopfseite des Ventilsitzes durch die Drehung des Ventilkörpers gegen den Ventilsitz;
D) Lösen der Knochenzementkartusche von der Gießform;
E) Aushärten des Knochenzementteigs in der Gießform; und
F) Entnehmen der so geformten und ausgehärteten Kniespacer-Komponente aus der Gießform.

Der Kniespacer ist für medizinische Anwendungen vorgesehen. Das erfindungsgemäße Verfahren umfasst nicht die Implantation bei einem Patienten sondern lediglich das Ausformen des Kniespacers. Nach Schritt F) kann der Kniespacer entgratet, geglättet, geschliffen, gereinigt, poliert und/oder bereichsweise aufgeraut werden.

Zum Entnehmen des geformten und ausgehärteten Kniespacers aus der Gießform in Schritt F) kann die Gießform nach Schritt E) geöffnet werden.

Es kann erfindungsgemäß vorgesehen sein, dass die Gießform eine trogförmige Form und einen Stempel aufweist. Hierdurch kann die Kniespacer-Komponente durch einen Druck des Stempels geformt werden, um eine möglichst glatte und Fehlstellenfreie Gleitfläche zu erhalten.

Es kann dabei bevorzugt vorgesehen sein, dass der Stempel in die trogförmige Form eingedrückt wird, um die Kniespacer-Komponente zu formen. Hierzu kann besonders bevorzugt vorgesehen sein, dass das Eindrücken des Stempels in die trogförmige Form nach Schritt B) erfolgt und besonders bevorzugt vor Schritt C). Es kann so ein Teil des Knochenzementteigs wieder durch das Ventil aus der Gießform herausgedrückt werden.

Es kann vorgesehen sein, dass nach Schritt D) und vor Schritt E) die folgenden Zwischenschritte erfolgen:
D2) Flüssigkeitsdichtes Verbinden einer neuen Knochenzementkartusche mit der Vorrichtung, wobei in der neuen Knochenzementkartusche Knochenzementteig oder Ausgangskomponenten zur Herstellung des Knochenzementteigs enthalten ist oder sind;
D3) Drehen des Ventilkörpers gegen den Ventilsitz und dadurch Überführen des Ventils in die geöffnete Stellung;
D4) Einpressen des Knochenzementteigs aus der neuen Knochenzementkartusche durch das Ventil in der geöffneten Stellung hindurch in die Gießform;
D5) Drehen des Ventilkörpers gegen den Ventilsitz und dadurch Überführen des Ventils in die geschlossene Stellung und Abscheren des Knochenzementteigs an der mindestens einen ersten Durchführung in der bereichsweise geschlossenen Kopfseite des Ventilsitzes durch die Drehung des Ventilkörpers gegen den Ventilsitz; und
D6) Lösen der neuen Knochenzementkartusche von der Gießform;
   wobei vorzugsweise die Schritte D2) bis D6) einmal oder mehrfach mit jeweils neuen Knochenzementkartuschen, die Knochenzementteig oder dessen Ausgangskomponenten enthalten, wiederholt werden, bis die Gießform vollständig oder nach Bedarf mit Knochenzementteig gefüllt ist.

Hierdurch kann eine Gießform mit großem Volumen mit mehreren Knochenzementkartuschen enthaltend geringe Volumina des Knochenzementteigs gefüllt werden. Dies ist beispielsweise für die Herstellung von großvolumigen Kniespacern vorteilhaft.

Des Weiteren kann vorgesehen sein, dass zum flüssigkeitsdichten Verbinden der Knochenzementkartusche und/oder der neuen Knochenzementkartusche diese mit einem Anschluss an dem Ventilkörper der Vorrichtung verbunden wird, wobei die Knochenzementkartusche oder die neue Knochenzementkartusche in den Anschluss hineingedreht oder eingeschraubt wird und zum Lösen der Knochenzementkartusche und/oder der neuen Knochenzementkartusche von dem Anschluss die Knochenzementkartusche oder die neue Knochenzementkartusche aus dem Anschluss herausgedreht oder herausgeschraubt wird, und/oder

Neben dem Schrauben kann die Knochenzementkartusche beispielsweise mit einem Bajonettverschluss mit dem Ventilkörper verbunden werden.

Durch das Eindrehen oder Einschrauben der Knochenzementkartusche in den Ventilkörper kann eine flüssigkeitsdichte Verbindung zwischen dem Ventilkörper und der Knochenzementkartusche bereitgestellt werden. Zudem kann durch die Drehung auch eine Drehung des Ventilkörpers gegen den Ventilsitz erfolgen beziehungsweise induziert werden.

Ferner kann vorgesehen sein, dass das Einpressen des Knochenzementteigs aus der Knochenzementkartusche oder der neuen Knochenzementkartusche durch Eindrücken eines Kolbens in einen Innenraum der Knochenzementkartusche erfolgt.

Hierdurch kann der Knochenzementteig auf einfache Weise aus der Knochenzementkartusche durch das geöffnete Ventil in die Gießform eingepresst werden.

Auch kann vorgesehen sein, dass das Drehen des Ventilkörpers gegen den Ventilsitz durch ein Schrauben des Ventilkörpers in dem Ventilsitz erfolgt oder durch ein manuelles Drehen des Ventilkörpers gegen Ventilsitz erfolgt, wobei bevorzugt das manuelle Drehen durch Bedienen eines Griffs erfolgt, der mit dem Ventilkörper verbunden ist.

Hiermit kann das Ventil auf einfache Art und Weise vom Anwender bedient werden.

Des Weiteren kann vorgesehen sein, dass die Gießform eine trogförmige Form und einen Stempel aufweist, wobei vor Schritt E) der Stempel in die trogförmige Form gepresst wird und dadurch die Kniespacer-Komponente aus dem Knochenzementteig in der Gießform geformt wird, wobei bevorzugt beim Einpressen des Stempels in die trogförmige Form ein Teil des Knochenzementteigs durch das Ventil in der geöffneten Stellung aus der Gießform herausgedrückt wird und wobei besonders bevorzugt der Teil des Knochenzementteigs in einen Auffangbehälter gedrückt wird, der mit dem zumindest einen zweiten Durchgang des Ventils verbunden ist.

Hierdurch kann eine Kniespacer-Komponente mit variabler Höhe geformt werden. Zudem kann durch Andrücken des Stempels auch die formgebende innere Oberfläche der Gießform vollständig mit Knochenzementteig benetzt werden, um so eine vollständige Kniespacer-Komponente ohne Fehlstellen zu erhalten.

Es ist hierbei zu beachten, dass das Ventil hier nicht mehr dasselbe Ventil sein muss, durch das der Knochenzementteig in die Gießform eingefüllt wurde. Beim Entfernen der Knochenzementkartusche kann der Ventilkörper mit entfernt werden und ein Griffstück mit einem neuen Ventilkörper kann in den Ventilsitz eingesetzt werden, so dass der Ventilsitz an der Gießform und der Ventilkörper des Griffstücks ein neues Ventil bilden, das aber dem Ventil gleicht, dass aus dem Ventilsitz der Gießform und dem Ventilkörper zum Verbinden der Knochenzementkartusche aufgebaut ist.

Die der vorliegenden Erfindung zugrundeliegenden Aufgaben werden ferner gelöst durch ein Verfahren zur Herstellung einer Kniespacer-Komponente zum temporären Ersetzen eines Teils eines Kniegelenks umfassend eine artikulierende Oberfläche des Kniegelenks, wobei das Verfahren mit einer erfindungsgemäßen Vorrichtung durchgeführt wird, das Verfahren aufweisend die folgenden chronologischen Schritte:
A) Herstellen eines Knochenzementteigs;
B) Einfüllen eines gemischten Knochenzementteigs in die Gießform;
C) Komprimieren der Gießform und dadurch Herausdrücken eines Teils des Knochenzementteigs aus der Gießform durch das Ventil in der geöffneten Stellung hindurch;
D) Drehen des Ventilkörpers gegen den Ventilsitz und dadurch Überführen des Ventils in die geschlossene Stellung und Abscheren des Knochenzementteigs an der mindestens einen ersten Durchführung in der bereichsweise geschlossenen Kopfseite des Ventilsitzes durch die Drehung des Ventilkörpers gegen den Ventilsitz;
E) Aushärten des Knochenzementteigs in der Gießform; und
F) Entnehmen der so geformten und ausgehärteten Kniespacer-Komponente aus der Gießform.

Der Kniespacer ist für medizinische Anwendungen vorgesehen. Das erfindungsgemäße Verfahren umfasst nicht die Implantation bei einem Patienten sondern lediglich das Ausformen des Kniespacers. Nach Schritt F) kann der Kniespacer entgratet, geglättet, geschliffen, gereinigt, poliert und/oder bereichsweise aufgeraut werden.

Es kann vorgesehen sein, dass zwischen den Schritten B) und C) ein Schritt B1) erfolgt: B1) Verschließen der Gießform bis auf das Ventil.

Hierdurch wird sichergestellt, dass der Knochenzementteig nur durch das Ventil aus der Gießform austreten kann.

Ferner kann vorgesehen sein, dass die Gießform eine trogförmige Form und einen Stempel aufweist, wobei in Schritt C) der Stempel in die trogförmige Form gepresst wird und dadurch die Kniespacer-Komponente aus dem Knochenzementteig in der Gießform geformt wird, wobei vorzugsweise beim Einpressen des Stempels in die trogförmige Form ein Teil des Knochenzementteigs durch das Ventil in der geöffneten Stellung aus der Gießform herausgedrückt wird.

Hierdurch kann eine Kniespacer-Komponente mit variabler Höhe geformt werden. Zudem kann durch Andrücken des Stempels auch die formgebende innere Oberfläche der Gießform vollständig mit Knochenzementteig benetzt werden, um so eine vollständige Kniespacer-Komponente ohne Fehlstellen zu erhalten.

Auch kann vorgesehen sein, dass vor Schritt B) der Knochenzementteig in der Knochenzementkartusche aus einer Monomerflüssigkeit und aus einem Zementpulver gemischt wird, wobei bevorzugt gegebenenfalls vor Schritt D3), vorzugsweise vor Schritt D2), der Knochenzementteig in der neuen Knochenzementkartusche aus einer Monomerflüssigkeit und aus einem Zementpulver gemischt wird.

Hierdurch kann zur Herstellung des Kniespacers ein frisch gemischter Knochenzementteig verwendet werden. Insbesondere PMMA-Knochenzementteige lassen sich im gemischten Zustand nur schwer oder gar nicht über Zeiträume von mehr als wenigen Minuten lagern. Zudem können dem Knochenzementteig so auch kurz vor der Herstellung des Kniespacers dem Knochenzementteig zur Behandlung geeignete pharmazeutische Wirkstoffe, wie Antibiotika und Antimykotika, beigemengt werden.

Des Weiteren kann vorgesehen sein, dass das Drehen des Ventilkörpers gegen den Ventilsitz durch ein Schrauben des Ventilkörpers in dem Ventilsitz erfolgt oder durch ein manuelles Drehen des Ventilkörpers gegen Ventilsitz erfolgt, wobei bevorzugt das manuelle Drehen durch Bedienen eines Griffs am Ventilkörper erfolgt.

Hiermit kann die Vorrichtung auf einfache Weise bedient werden.

Der Erfindung liegt die überraschende Erkenntnis zugrunde, dass es durch einen in einem Ventilsitz drehbaren Ventilkörper gelingt, eine Vorrichtung mit einer Gießform bereitzustellen, bei der der Anguss oder der Abguss mit dem Ventilkörper abgeschert beziehungsweise weitgehend abgeschert werden kann und gleichzeitig die Gießform derart zu verschließen oder die verbleibenden Kanäle zumindest so weit einzuschnüren, dass der Knochenzementteig in der Gießform weiter unter Druck gehalten werden kann, um sich an die Innenseiten der Gießform anzupressen, wobei gleichzeitig verhindert wird, dass Knochenzementteig aus der Gießform durch die Öffnung wieder oder weiter herausgedrückt wird. Die Vorrichtung ermöglicht auch, die Gießform mit den Inhalten mehrerer Knochenzementkartuschen nacheinander zu befüllen, ohne dass der Knochenzementteig wieder durch die Öffnung aus der Gießform herausfließen kann. Hierdurch gelingt es, auch mit Knochenzementkartuschen, die nur geringe Volumina des Knochenzements bereitstellen, Kniespacer beziehungsweise Kniespacer-Komponenten mit großem Volumen herzustellen. Ebenso kann auch ein Stempeln der Kniespacer-Komponente zum Einstellen der Höhe der Kniespacer-Komponente und zum Anpressen des Knochenzementteigs an die formgebenden Oberflächen in der Gießform angewendet werden, wobei überschüssiger Knochenzementteig durch das geöffnete Ventil aus der Gießform herausgedrückt werden kann, wobei der so entstehende Abguss mit dem Ventilkörper abgeschert beziehungsweise weitgehend abgeschert werden kann, bevor der Knochenzementteig aushärtet.

Knochenzementteig, insbesondere nicht hochviskoser Knochenzementteig, kann durch den Verschluss beziehungsweise das geschlossene Ventil nicht aus der Gießform fließen. Eine Ausbildung von Fehlstellen im Kniespacer beziehungsweise in den Kniespacer-Komponenten infolge des Auslaufens von Knochenzementteig wird dadurch verhindert. Weiterhin wird durch die erfindungsgemäßen Maßnahmen der noch in der Knochenzementkartusche befindliche Rest des Knochenzementteigs (Anguss) und/oder der aus der Gießform herausgedrückte Knochenzementteig (Abguss) vom Knochenzementteig in der Gießform getrennt. Nach beendeter Aushärtung des Knochenzementteigs ist es daher nicht mehr notwendig, den Anguss beziehungsweise den Abguss mechanisch beispielsweise durch Sägen abzutrennen. Eventuell verbliebene dünne Verbindungen können leicht abgebrochen oder abgeschnitten werden. Dadurch wird der Arbeits- und Zeitaufwand für das OP-Personal gesenkt.

Der Anguss beziehungsweise der Abguss der Kniespacer-Komponente wird durch die Öffnung mit dem Ventilsitz und dem Ventilkörper gebildet. Durch das Verdrehen des Ventilkörpers gegenüber dem Ventilsitz aus der geöffneten Stellung in die geschlossene Stellung des Ventils wird die Gießform für Knochenzementteig undurchlässig verschlossen. Das bedeutet, der aus dem Ventilsitz und dem Ventilkörper gebildete Anguss/Abguss beziehungsweise die dem Anguss/Abguss formgebende Teile haben gleichzeitig eine Ventilfunktion. Komplexe zusätzliche Ventile sind nicht notwendig.

Die manuelle Drehung des Ventilkörpers gegen den Ventilsitz kann durch einen Griff an dem Ventilkörper erfolgen. Vorteilhaft kann die Drehung auch dadurch erfolgen, dass beim Herausdrehen der Knochenzementkartusche der Ventilkörper von der Knochenzementkartusche mit gedreht wird. Dabei ist es jedoch notwendig, dass ein Anschlag die Drehbewegung des Ventilkörpers gegenüber dem Ventilsitz begrenzt, damit der Verschluss sicher erfolgen kann und damit der Ventilkörper nicht aus dem Ventilsitz komplett herausgedreht werden kann.

Um die Gießform zu füllen, sollte ein Gegendruck vom Anwender aufgebracht werden. Die Gießform kann soweit mit Knochenzementteig gefüllt werden, bis die gewünschte Höhe der Kniespacer-Komponente in der Gießform eingestellt ist. Dabei kann der Stempel der Gießform aus der trogförmigen Form der Gießform herausgedrückt werden, je mehr Knochenzementteig in die Gießform gefüllt wird. Der Anwender muss dazu über den Stempel einen Druck auf die Gießform aufrechterhalten. Eingeschlossene Luft kann aus dem Spalt zwischen dem Stempel und der trogförmigen Form entweichen. Wenn die gewünschte Höhe der Kniespacer-Komponente in der Gießform erreicht ist, kann der Anwender einfach Aufhören weiteren Knochenzementteig in die Gießform zu pressen.

Alternativ kann der Knochenzementteig im Überschuss in die Gießform gefüllt werden und die Höhe wird eingestellt, indem die Gießform nach dem Einfüllen des Knochenzementteigs so weit komprimiert wird, bis die gewünschte Höhe der Kniespacer-Komponente in der Gießform erreicht ist. Hierzu kann der Stempel (beispielsweise mit dem Griffstück) so tief in die trogförmige Form eingedrückt werden, bis die gewünschte Höhe der Kniespacer-Komponente eingestellt ist. Ausfließender überschüssiger Knochenzementteig kann durch das geöffnete Ventil abfließen. Bevorzugt wird der überschüssige Knochenzementteig in einem Auffangbehälter aufgenommen. Der Auffangbehälter kann in dem Griffstück angeordnet sein, mit dem der Stempel und das Ventil bedient wird.

Die Höhe der Kniespacer-Komponente kann auf diese Weise stufenlos eingestellt werden. Eine Rastung oder Befestigungsmittel zum Einstellen der Höhe an der Gießform werden für das Einstellen der Höhe der Kniespacer-Komponente mit der erfindungsgemäßen Vorrichtung und dem erfindungsgemäßen Verfahren folglich nicht benötigt.

Die Teile der Gießform müssen auch nicht druckdicht aneinander befestigt werden. Es werden keine Clips, Schrauben oder andere Befestigungsmittel benötigt, um die Teile der Gießform aneinander zu befestigen oder um einen Druck aufzunehmen. Diese Teile können folglich nicht im OP-Saal verloren gehen und können daher den Ablauf einer OP nicht stören.

Erfindungsgemäß bevorzugt ist, dass ein Innengewinde oder Teile eines Innengewindes an der Innenseite eines Hohlzylinder-förmigen Ventilkörpers angeordnet sind, wobei das Innengewinde mit dem Außengewinde eines Kartuschenkopfs einer Knochenzementkartusche reversibel durch Verschrauben verbindbar ist. Mit dieser Vorrichtung können Kniespacer-Komponenten in der Weise hergestellt werden, dass zuerst in einer Knochenzementkartusche ein Polymethylmethacrylat-Knochenzementteig aus Zementpulver und Monomerflüssigkeit durch Vermischen hergestellt wird, dass in einem zweiten Schritt die Knochenzementkartusche in den Ventilkörper eingeschraubt wird, wobei das Ventil beziehungsweise der Ventilkörper in die geöffnete Stellung gedreht wird, dass in einem dritten Schritt ein Stempel der Gießform mit dem Ventilsitz, dem Ventilkörper und mit der Knochenzementkartusche in eine trogförmige Form der Gießform geschoben wird, bis die gewünschte Höhe über dem Boden der trogförmigen Form erreicht wird, dass in einem vierten Schritt mit einer Austragsvorrichtung der Polymethylmethacrylat-Knochenzementteig aus der Knochenzementkartusche in den Hohlraum der Gießform unter dem Stempel gepresst wird bis der gewünschte Füllstand unter dem Stempel erreicht wird, wobei die Luft aus dem Hohlraum der Gießform über einen Zwischenraum zwischen der trogförmigen Form und dem Stempel entwicht, dass in einem fünften Schritt die Knochenzementkartusche aus dem Ventilkörper herausgeschraubt wird, wobei das Ventil beziehungsweise der Ventilkörper gegenüber dem Ventilsitz in die geschlossene Stellung gedreht wird, wodurch der Polymethylmethacrylat-Knochenzementteig im Hohlraum der Gießform von überschüssigem Polymethylmethacrylat-Knochenzementteig im Ventilkörper getrennt wird, dass in einem sechsten Schritt nach erfolgter Aushärtung des Polymethylmethacrylat-Knochenzementteigs der Stempel aus der trogförmigen Form herausgezogen wird, wobei die Kniepacer-Komponente vom Stempel abgelöst wird, und in einem siebten Schritt die Kniespacer-Komponente aus der Gießform beziehungsweise der trogförmigen Form der Gießform entnommen wird.

Es ist dabei zeit- und arbeitssparend, dass die Kniespacer-Komponente keinen Anguss oder Abguss aus überschüssigen Polymethylmethacrylat-Knochenzement enthält, der mechanisch abgetrennt werden muss.

In einer zweiten Ausgestaltungsvariante, ist der Ventilkörper als Hohlzylinder ausgebildet, an dessen der Dichtfläche gegenüberliegenden Schmalseite ein Griff angeordnet ist. Die Handhabung der Vorrichtung erfolgt in der Weise, das in einem ersten Schritt Polymethylmethacrylat-Knochenzementpulver mit Monomerflüssigkeit in einer Mischschüssel oder auch in einer Knochenzementkartusche miteinander zu einem homogenen Polymethylmethacrylat-Knochenzementteig vermischt werden, dass in einem zweiten Schritt ein Überschuss an Knochenzementteig im Vergleich zum Volumen der herzustellenden Kniespacer-Komponente durch Gießen, Einspritzen oder mit einem Spatel in eine trogförmige Form der Gießform eingebracht wird, dass in einem dritten Schritt ein Stempel der Gießform mit dem Ventilsitz und dem Ventilkörper in den Hohlraum der Gießform eingesteckt wird, wobei das Ventil beziehungsweise der Ventilkörper gegenüber dem Ventilsitz durch Drehen des Griffs in die geöffnete Stellung gedreht wird, dass in einem vierten Schritt mit dem Griff der Stempel in Richtung des Bodens der trogförmigen Form gedrückt wird, bis die gewünschte Höhe des Knochenzementteigs in der Gießform erreicht wird, wobei der überschüssige Knochenzementteig durch den Ventilsitz und den Ventilkörper (beziehungsweise durch den mindestens einen ersten Durchgang im Ventilsitz und durch den mindestens einen zweiten Durchgang im Ventilkörper) tritt, dass in einem fünften Schritt der Griff am Ventilkörper so gedreht wird, dass das Ventil beziehungsweise der Ventilkörper gegenüber dem Ventilsitz in die geschlossene Stellung überführt wird, wodurch der überschüssige Polymethylmethacrylat-Knochenzementteig im Ventilkörper vom Polymethylmethacrylat-Knochenzementteig im Hohlraum der Gießform abgetrennt wird, dass in einem siebten Schritt der Polymethylmethacrylat-Knochenzementteig in der Gießform aushärtet, dass in einem achten Schritt der Stempel aus der trogförmigen Form gezogen wird, wobei sich der Stempel von der ausgehärteten Kniespacer-Komponente trennt und dass in einem neunten Schritt die Kniespacer-Komponente aus der Gießform beziehungsweise der trogförmigen Form der Gießform entnommen wird.

Es ist dabei zeit- und arbeitssparend, dass die Kniespacer-Komponente keinen Abguss aus überschüssigem Polymethylmethacrylat-Knochenzement aufweist, der mechanisch aufwendig abgetrennt werden muss. Vorteilhaft ist es weiterhin, wenn die Unterseite des Stempels konkav geformt ist, damit die Luft in Richtung des Ventilsitzes geleitet wird und durch den Ventilsitz und den Ventilkörper aus dem Hohlraum der Gießform ausgeleitet wird.

Ein mit der Vorrichtung hergestellter Kniespacer beziehungsweise die Kniespacer-Komponenten können vorteilhaft im Rahmen von zweizeitigen septischen Revisionen verwendet werden, bei denen eine Infektion mit zwei oder mehreren mikrobiellen Keimen und insbesondere mit problematischen Keimen vorliegt.

Eine beispielhafte erfindungsgemäße Vorrichtung kann zusammengesetzt sein aus
a) einer trogförmigen Form als Teil der Gießform, wobei die trogförmige Form einen Hohlraum aufweist, wobei der Hohlraumboden die Kontur einer oder mehrerer artikulierenden Gleitflächen besitzt,
b) einem Stempel als Teil der Gießform, der axial in dem Hohlraum der trogförmigen Form verschiebbar ist, wobei der Stempel die Öffnung enthält, welche die dem Hohlraumboden der trogförmigen Form zugewandte Unterseite des Stempels mit der gegenüberliegenden Oberseite des Stempels flüssigkeitsdurchlässig verbindet,
c) einem formstabilen hohlzylinderförmigen Ventilsitz,
   c1) der in der Öffnung des Stempels verdrehsicher angeordnet ist,
   c2) wobei der Ventilsitz an seiner Kopfseite als Scheibe ausgebildet ist, in der die mindestens eine erste Durchführung angeordnet ist, welche die Unterseite des Stempels mit der Oberseite des Stempels flüssigkeitsdurchlässig verbindet, wobei die Fläche der mindestens einen ersten Durchführung nur maximal 50 Prozent der Scheibenfläche des Ventilsitzes einnimmt, und
   c3) wobei der Ventilsitz ein Innengewinde an der Innenseite aufweist,
d) einem formstabilen, hohlzylinderförmigen Ventilkörper,
   d1) welcher ein Außengewinde an der Außenseite des Hohlzylinders aufweist, das mit dem Innengewinde des Ventilsitzes verschraubbar ist,
   d2) wobei der Ventilkörper an seiner Dichtfläche, die dem Hohlraum der trogförmigen Form zugewandt ist, als Scheibe ausgebildet ist, die mindestens eine zweite Durchführung aufweist, wobei die Fläche der mindestens einen zweiten Durchführung nur maximal 50 Prozent der Scheibenfläche des Ventilkörpers einnimmt, und
   d4) wobei die mindestens eine zweite Durchführung eine annähernd gleiche Größe und Gestalt wie die mindestens eine erste Durchführung des Ventilsitzes hat,
   d5) wobei der Ventilkörper beim Einschrauben in den Ventilsitz in eine geöffnete Stellung gedreht wird, so dass die mindestens eine zweite Durchführung über der mindestens einen ersten Durchführung liegt und eine flüssigkeitsdurchlässige Verbindung vom Innenraum der Gießform mit der Umgebung hergestellt wird und in eine geschlossene Stellung gedreht werden kann, so dass die mindestens eine zweite Durchführung nicht die mindestens eine erste Durchführung überdeckt beziehungsweise abdeckt, und
e) dass in den Hohlraum der trogförmigen Form eingebrachter Polymethylmethacrylat-Knochenzementteig, durch die Unterseite des Stempels begrenzt wird und dass überschüssiger Polymethylmethacrylat-Knochenzementteig im Hohlraum des Ventilkörpers angeordnet ist, wobei durch Drehung des Ventilkörpers in die geschlossene Stellung der überschüssige Polymethylmethacrylat-Knochenzementteig vom Polymethylmethacrylat-Knochenzementteig im Hohlraum der Gießform abgetrennt oder abgeschert oder weitgehend abgeschert wird.

Nach Beendigung des Einpressens des Knochenzementteigs in den Hohlraum der Gießform wird der Ventilkörper manuell so gedreht, dass die mindestens eine zweite Durchführung des Ventilkörpers nicht mehr über der mindestens einen ersten Durchführung des formstabilen Ventilsitzes steht beziehungsweise fluchtet. Dadurch wird der Hohlraum der Gießform flüssigkeitsundurchlässig verschlossen. Auch ein nicht hochviskoser Knochenzementteig kann dadurch nicht aus der Gießform fließen.

Ein beispielhaftes erfindungsgemäßes Verfahren zur Herstellung von Kniespacern mit der erfindungsgemäßen Vorrichtung kann die folgenden nacheinander ablaufenden Schritte umfassen:
a) Bereitstellen der Gießform und der trogförmigen Form der Gießform,
b) Mischen des Knochenzementpulvers mit der Monomerflüssigkeit in einer Knochenzementkartusche bis sich ein homogener Knochenzementteig gebildet hat,
c) Flüssigkeitsdurchlässiges Verbinden der Knochenzementkartusche mit der Gießform beziehungsweise mit dem Ventil an der Gießform durch Einschrauben des Adapterelements in ein Innengewinde des Ventilkörpers, wobei der Ventilkörper in die geöffnete Stellung gedreht wird,
d) Einpressen des Knochenzementteigs aus der Knochenzementkartusche in die Gießform mit Hilfe einer Auspressvorrichtung, wobei der Knochenzementteig die Luft im Hohlraum der Gießform verdrängt und vorzugsweise die verdrängte Luft durch einen Spalt zwischen der trogförmigen Form und dem Stempel aus dem Hohlraum der Gießform in die Umgebung austritt,
e) Herausdrehen der Knochenzementkartusche aus dem Ventilkörper, wobei der Ventilkörper aus der geöffneten Stellung in die geschlossene Stellung des Ventils gedreht wird, wodurch die mindestens eine erste Durchführung und die mindestens eine zweite Durchführung nicht mehr übereinanderstehen, so dass kein Knochenzementteig aus dem Hohlraum der Gießform durch das Ventil in die Umgebung austreten kann,
f) Trennen der Knochenzementkartusche von der Gießform
g) Aushärten des Knochenzementteigs in der Gießform,
h) Öffnen der Gießform nach erfolgter Aushärtung des Knochenzementteigs durch Herausnahme des Stempels aus der trogförmigen Form und
i) Entnahme der Kniespacer-Komponente.

Ein alternatives beispielhaftes erfindungsgemäßes Verfahren zur Herstellung von Kniespacern mit der erfindungsgemäßen Vorrichtung kann die folgenden nacheinander ablaufenden Schritte umfassen:
a) Bereitstellen der Gießform und der trogförmigen Form der Gießform,
b) Mischen eines Knochenzementpulvers mit einer Monomerflüssigkeit bis sich ein Knochenzementteig gebildet hat (beispielsweise in einer Mischschüssel oder auch in einer Knochenzementkartusche),
c) Einfüllen des Knochenzementteigs in die trogförmige Form,
d) gegebenenfalls Öffnen des Ventils durch Drehen des Ventilkörpers gegen den Ventilsitz in die geöffnete Stellung,
e) Einsetzen eines Stempels der Gießform mit dem Ventil in der geöffneten Stellung in den Hohlraum der trogförmigen Form,
f) Drücken eines Stempels der Gießform in Richtung des Hohlraumbodens der trogförmigen Form bis die gewünschte Höhe der Kniespacer-Komponente erreicht ist, unter gleichzeitigem Verdrängen der Luft und des überschüssigen Polymethylmethacrylat-Knochenzementteigs durch den Ventilsitz und den Ventilkörper in der geöffneten Stellung in einen Hohlraum des Ventilkörpers,
g) Nach Erreichen der der gewünschten Füllhöhe des Polymethylmethacrylat-Knochenzementteigs manuelles Verdrehen des Ventilkörpers gegen den Ventilsitz in die geschlossene Stellung, so dass der überschüssige Polymethylmethacrylat-Knochenzementteig vom Polymethylmethacrylat-Knochenzementteig im Hohlraum der Gießform abgetrennt ist,
h) Aushärten des Knochenzementteigs in der Gießform,
i) Öffnen der Gießform nach erfolgter Aushärtung des Knochenzementteigs durch Herausnahme des Stempels aus der trogförmigen Form und
i) Entnahme der Kniespacer-Komponente.

Im Folgenden werden weitere Ausführungsbeispiele der Erfindung anhand von dreiunddreißig schematisch dargestellten Figuren erläutert, ohne jedoch dabei die Erfindung zu beschränken. Dabei zeigt:
Figur 1: eine schematische perspektivische Querschnittansicht einer beispielhaften ersten erfindungsgemäßen Vorrichtung zum Herstellen einer Tibia-Komponente mit offenem Ventil;
Figur 2: eine schematische Außenansicht auf die Teile der ersten erfindungsgemäßen Vorrichtung nach Figur 1;
Figur 3: eine schematische perspektivische Außenansicht der ersten erfindungsgemäßen Vorrichtung nach den Figuren 1 und 2 mit eingesetztem Stempel;
Figur 4: eine schematische perspektivische Außenansicht der ersten erfindungsgemäßen Vorrichtung nach den Figuren 1 bis 3 mit eingesetztem Stempel;
Figur 5: eine schematische perspektivische Querschnittansicht der ersten erfindungsgemäßen Vorrichtung mit geschlossenem Ventil;
Figur 6: eine schematische perspektivische Querschnittansicht der ersten erfindungsgemäßen Vorrichtung mit offenem Ventil während dem Ausformen der Tibia-Komponente;
Figur 7: eine schematische perspektivische Querschnittansicht der ersten erfindungsgemäßen Vorrichtung mit offenem Ventil während dem Ausformen der Tibia-Komponente, wobei die Schnittebene gegenüber Figur 6 um 90° gedreht ist;
Figur 8: eine schematische Querschnittansicht der ersten erfindungsgemäßen Vorrichtung mit offenem Ventil und an die Gießform angeschlossener Knochenzementkartusche;
Figur 9: eine schematische Querschnittansicht der ersten erfindungsgemäßen Vorrichtung mit geschlossenem Ventil mit von der Gießform gelöster Knochenzementkartusche;
Figur 10: eine schematische Querschnittansicht der ersten erfindungsgemäßen Vorrichtung mit offenem Ventil und an die Gießform angeschlossenem Griffstück während dem Ausformen einer Tibia-Komponente;
Figur 11: eine schematische perspektivische Ansicht einer beispielhaften zweiten erfindungsgemäßen Vorrichtung zum Herstellen einer Femur-Komponente;
Figur 12: eine schematische perspektivische Querschnittansicht durch die Teile der zweiten erfindungsgemäßen Vorrichtung nach Figur 11 mit einer damit hergestellten Femur-Komponente;
Figur 13: das Einfüllen von Knochenzementteig in einen Teil der Gießform der zweiten erfindungsgemäßen Vorrichtung;
Figur 14: eine schematische perspektivische Außenansicht auf die Teile der zweiten erfindungsgemäßen Vorrichtung vor dem Ausformen der Femur-Komponente;
Figur 15: eine schematische perspektivische Querschnittansicht auf die geschlossene zweite erfindungsgemäßen Vorrichtung nach den Figuren 11 bis 14 und der darin enthaltenen Femur-Komponente;
Figur 16: eine perspektivische Ansicht auf eine Femur-Komponente, die mit einer zweiten erfindungsgemäßen Vorrichtung nach den Figuren 11 bis 15 hergestellt wurde;
Figur 17: eine schematische perspektivische Außenansicht einer beispielhaften dritten erfindungsgemäßen Vorrichtung zum Herstellen einer Tibia-Komponente;
Figur 18: eine schematische perspektivische Querschnittansicht der dritten erfindungsgemäßen Vorrichtung mit geschlossenem Ventil;
Figur 19: eine schematische perspektivische Querschnittansicht der dritten erfindungsgemäßen Vorrichtung mit offenem Ventil und eingeschobenem Stempel;
Figur 20: eine schematische perspektivische Querschnittansicht der dritten erfindungsgemäßen Vorrichtung nach den Figuren 17 bis 19 während dem Ausformen einer Tibia-Komponente;
Figur 21: eine schematische perspektivische Querschnittansicht der dritten erfindungsgemäßen Vorrichtung nach den Figuren 17 bis 20 nach dem Ausformen der Tibia-Komponente;
Figur 22: eine perspektivische Ansicht auf eine Tibia-Komponente Knochenzementrest, die mit einer dritten erfindungsgemäßen Vorrichtung nach den Figuren 17 bis 21 hergestellt wurde, zusammen mit einem Knochenzementrest;
Figur 23: eine schematische perspektivische Ansicht auf ein Ventil für eine erfindungsgemäße Vorrichtung im geöffneten Zustand;
Figur 24: eine schematische perspektivische Teilquerschnittansicht auf das Ventil nach Figur 23 im geöffneten Zustand;
Figur 25: eine schematische perspektivische Querschnittansicht durch das Ventil nach den

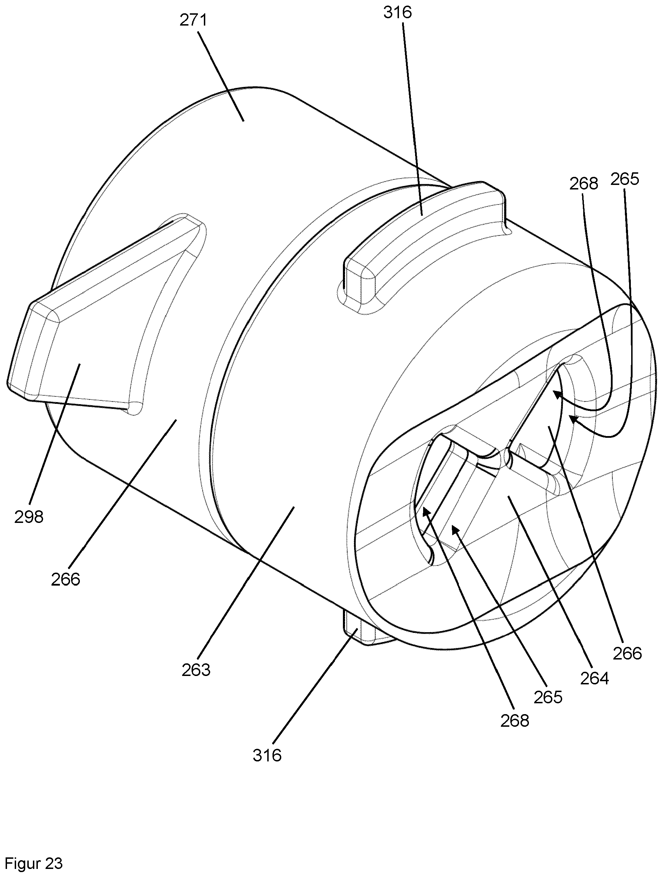

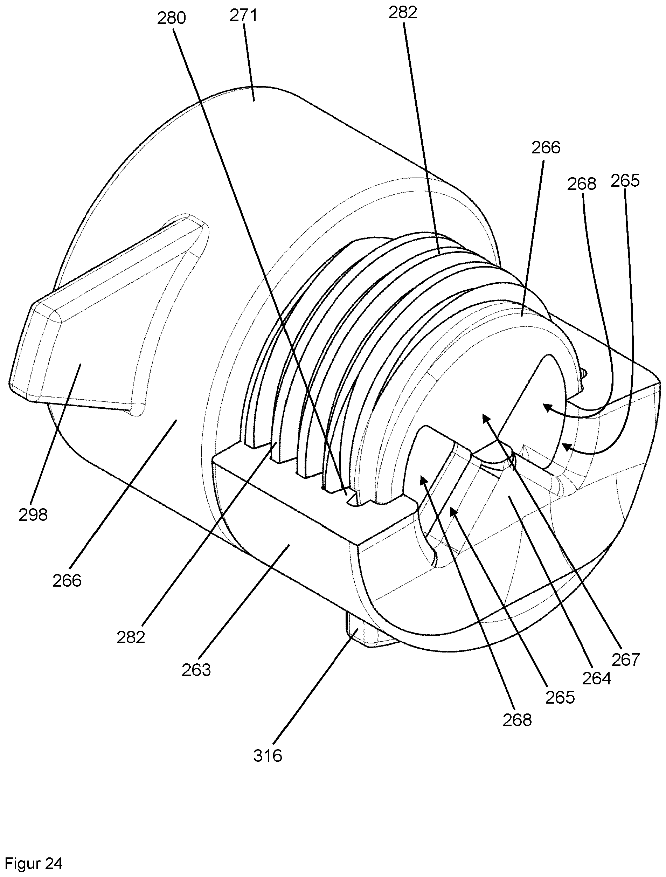

Figuren 23 und 24 im geöffneten Zustand;

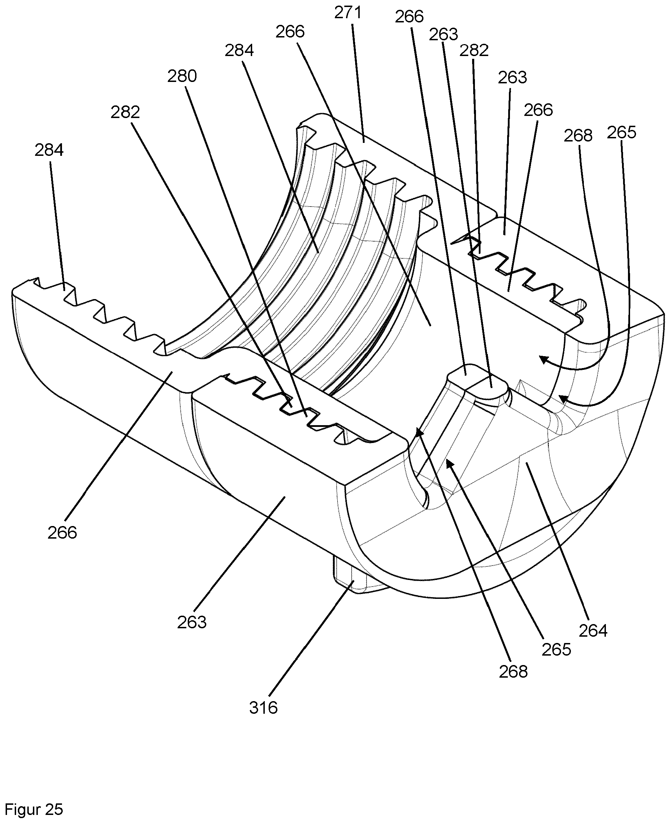

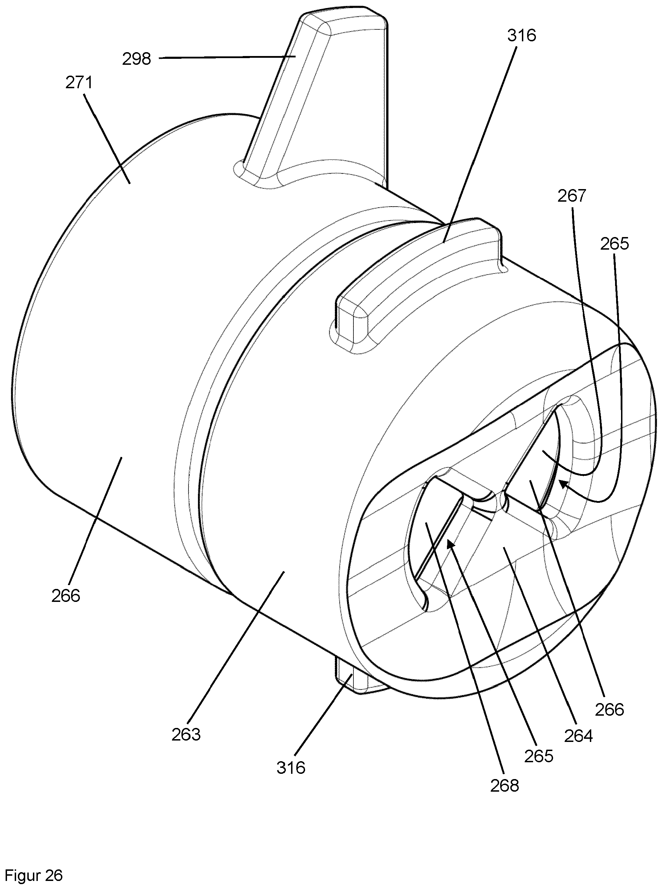

Figur 26: eine schematische perspektivische Ansicht auf das Ventil nach den Figuren 23 bis 25 im geschlossenen Zustand;

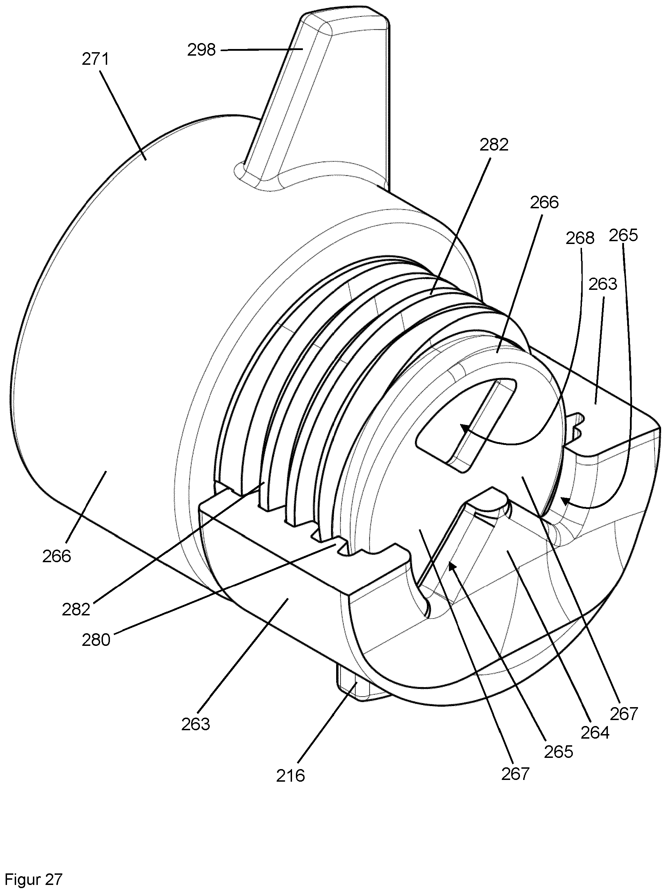

Figur 27: eine schematische perspektivische Teilquerschnittansicht auf das Ventil nach den
Figuren 23 bis 26 im geschlossenen Zustand;

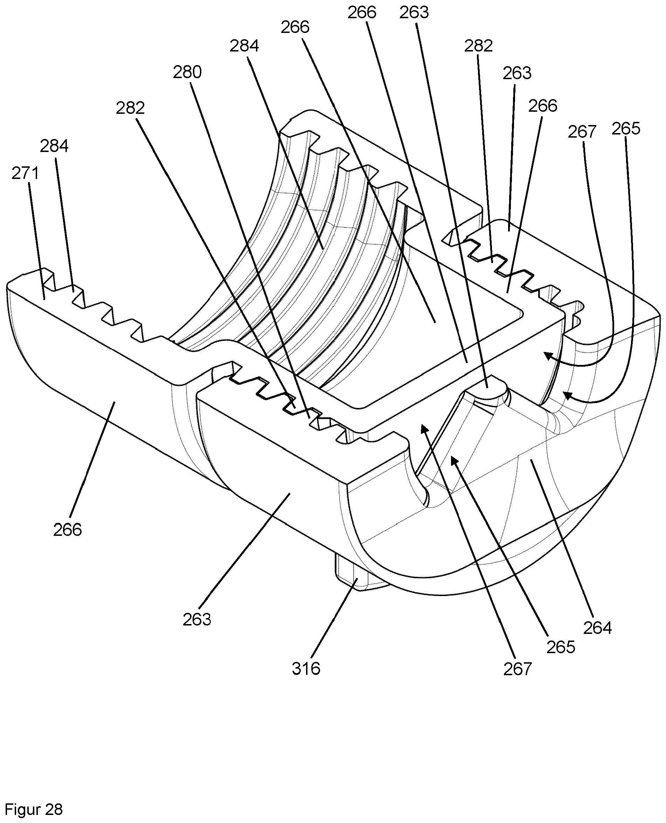

Figur 28: eine schematische perspektivische Querschnittansicht auf das Ventil nach den
Figuren 23 bis 27 im geschlossenen Zustand;

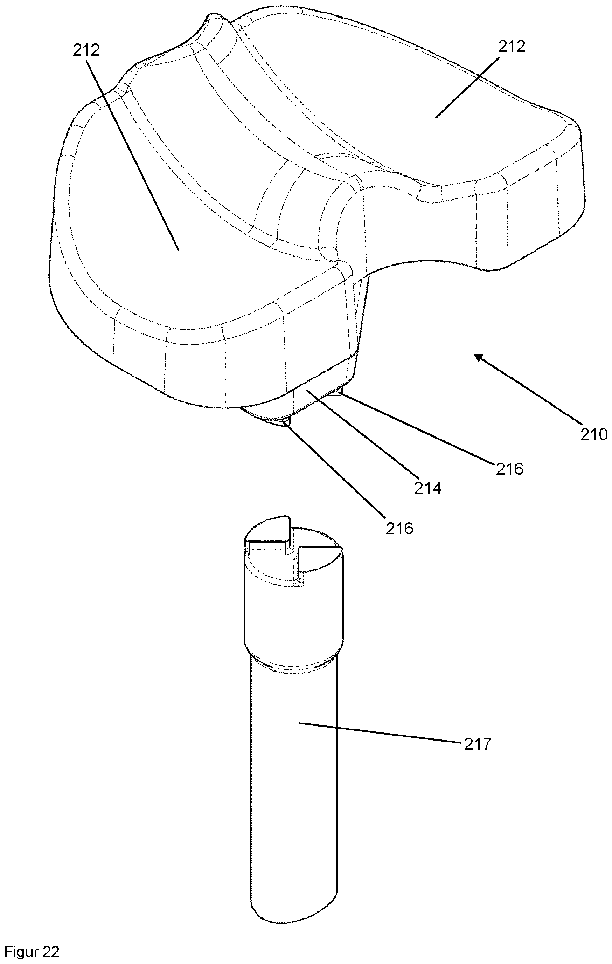

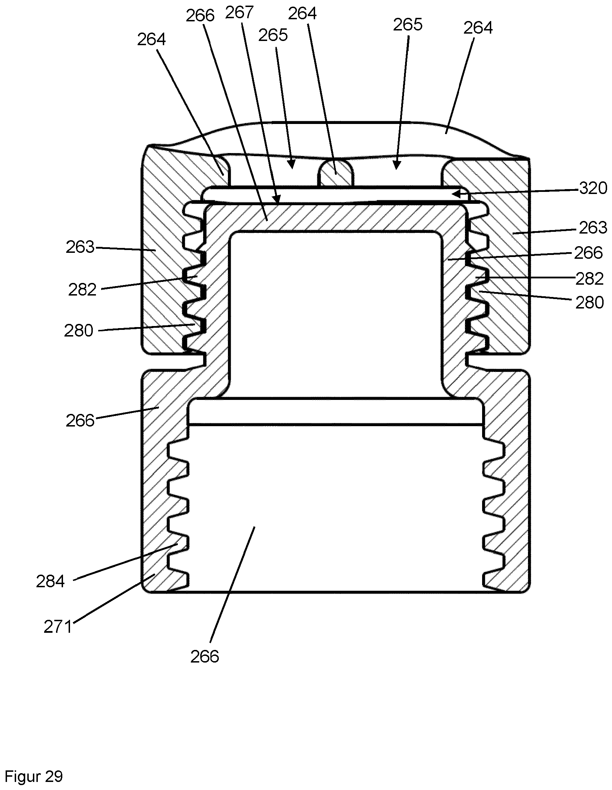

Figur 29: eine schematische Querschnittansicht des Ventils nach den Figuren 22 bis 27 im geschlossenen Zustand;

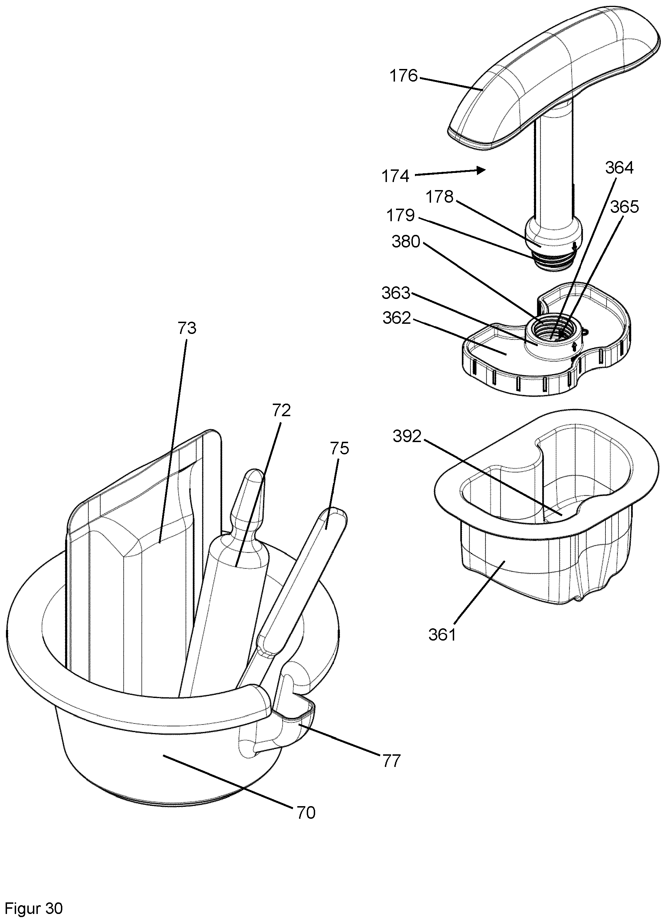

Figur 30: eine schematische perspektivische Außenansicht einer beispielhaften vierten erfindungsgemäßen Vorrichtung zum Herstellen einer Tibia-Komponente;

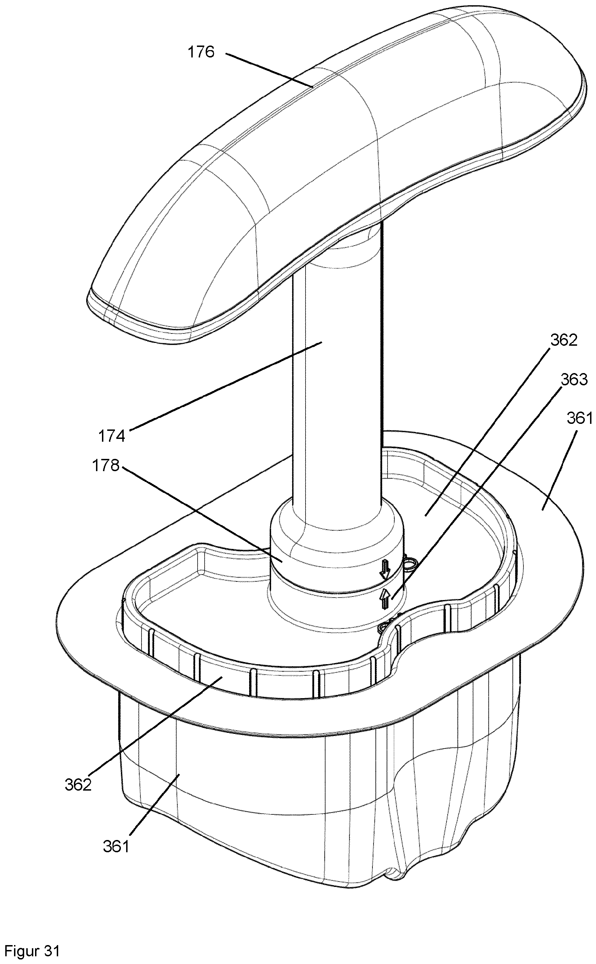

Figur 31: eine schematische perspektivische Außenansicht der vierten erfindungsgemäßen Vorrichtung mit eingesetztem Stempel;

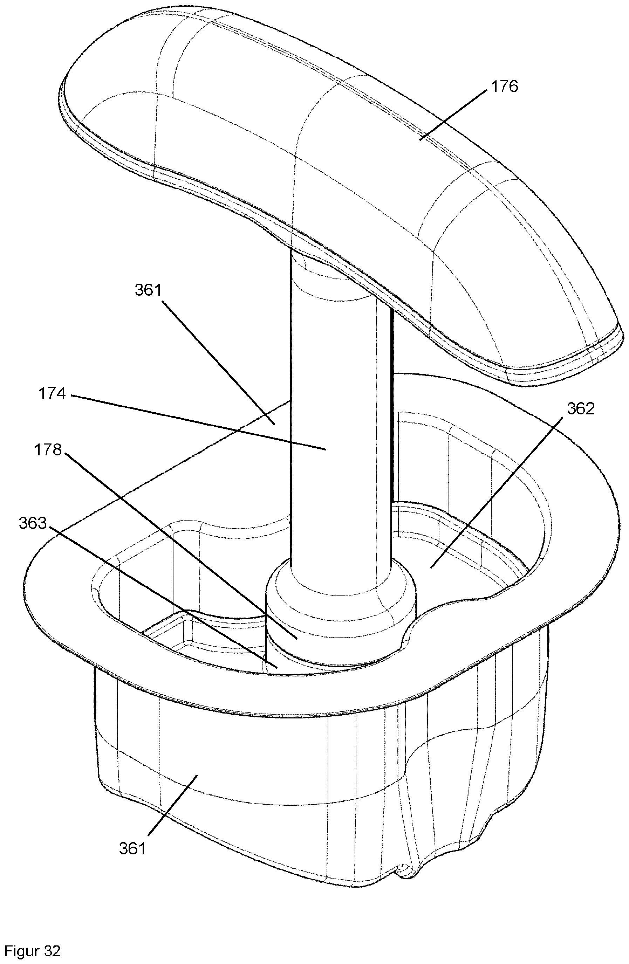

Figur 32: eine schematische perspektivische Außenansicht der vierten erfindungsgemäßen Vorrichtung mit eingeschobenem Stempel; und

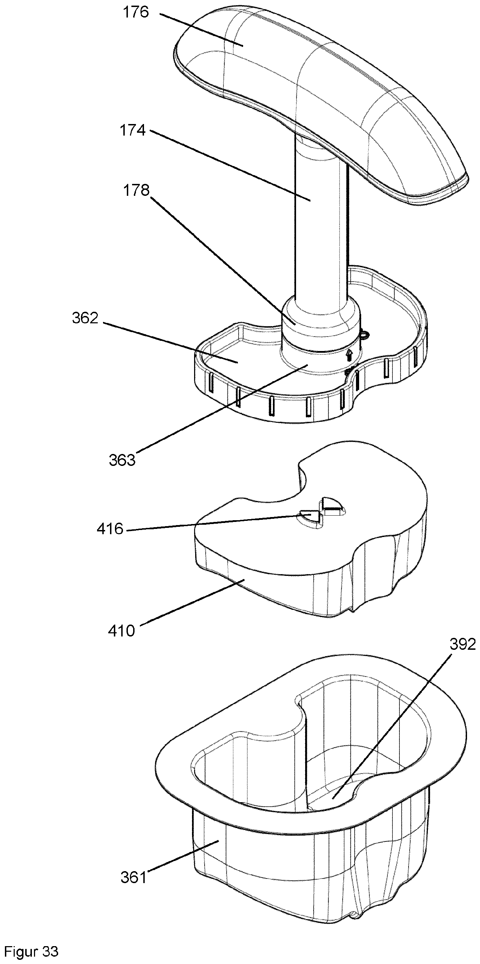

Figur 33: eine schematische perspektivische Querschnittansicht der vierten erfindungsgemäßen Vorrichtung nach den Figuren 30 bis 32 nach dem Ausformen der Tibia-Komponente.

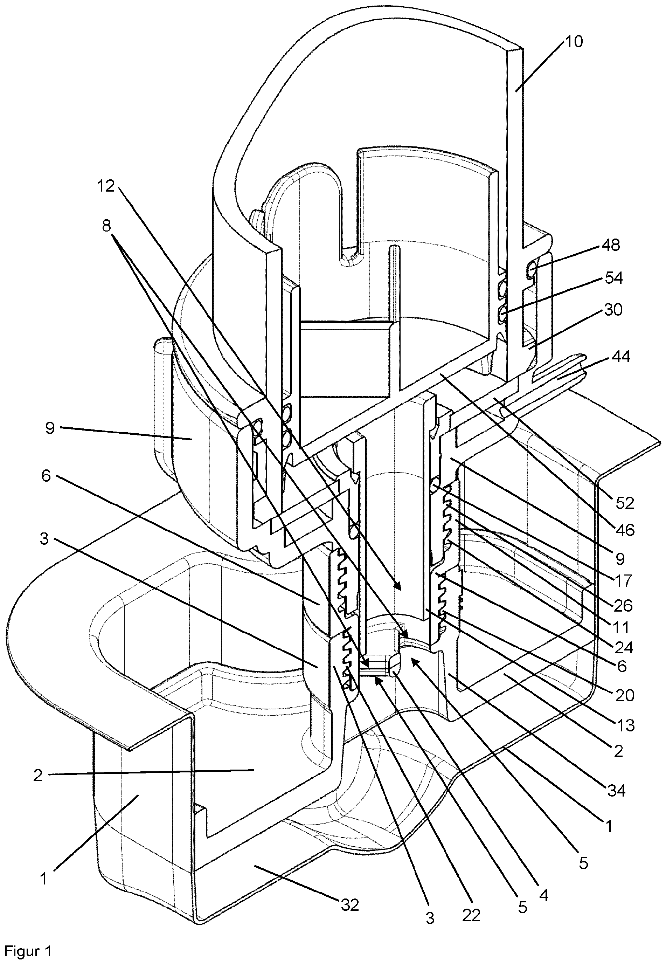

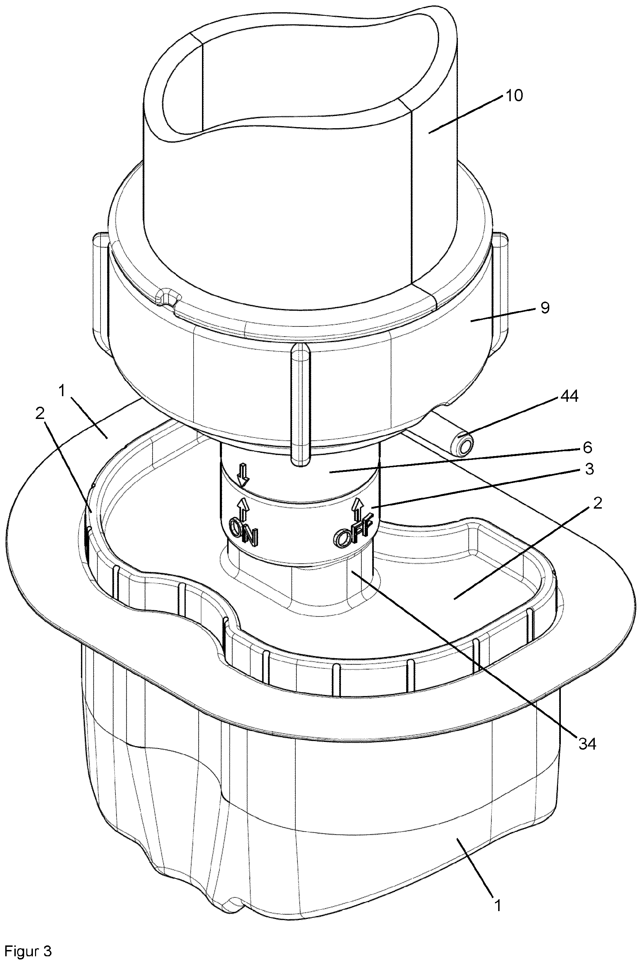

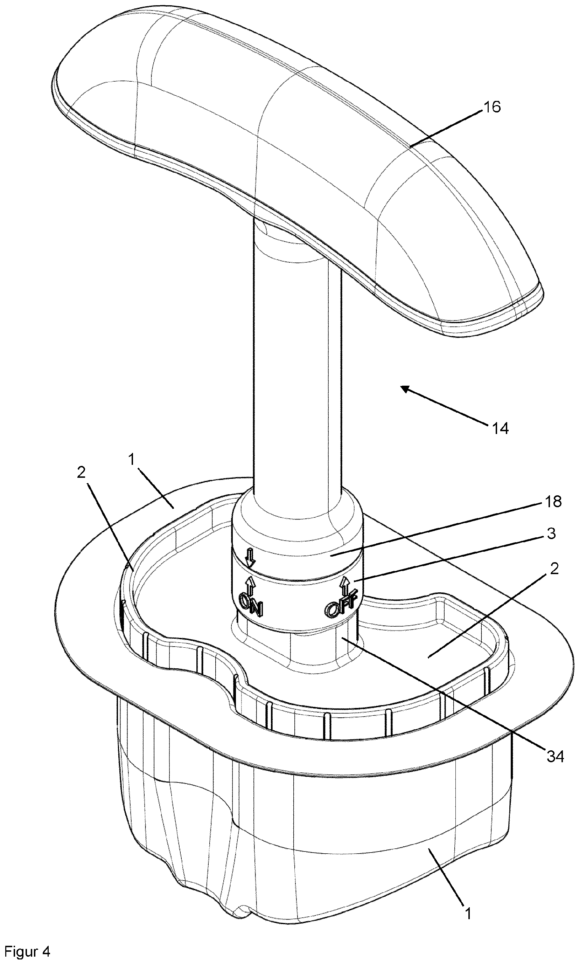

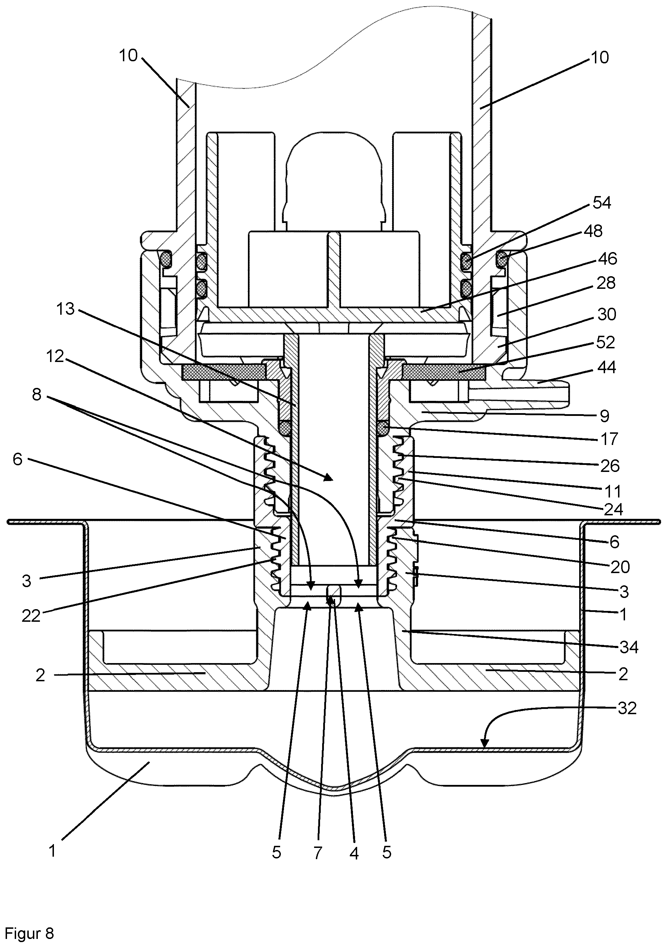

In den Figuren 1 bis 10 sind Abbildungen eines ersten Ausführungsbeispiels einer erfindungsgemäßen Vorrichtung zur Herstellung von Kniespacer-Komponenten eines Kniespacers und Teile davon in verschiedenen Ansichten gezeigt.

Die erste erfindungsgemäße Vorrichtung ist zur Herstellung einer Tibia-Komponente eines Kniespacers geeignet und vorgesehen. Die Vorrichtung umfasst eine Gießform, die sich aus zwei Teilen zusammensetzt. Die Gießform kann eine trogförmige Form 1 und einen Stempel 2 aufweisen. Der Stempel 2 kann in die trogförmige Form 1 eingesetzt werden und kann in die trogförmige Form 1 eingeschoben und vorzugsweise auch wieder herausgezogen werden. Die trogförmige Form 1 kann kostengünstig aus Kunststofffolie gefertigt werden. Die Kunststofffolie kann mehrere Schichten aufweisen. An einer Seite des Stempels 2 kann eine Öffnung zum Durchfließen von Knochenzementteig 50 ausgeformt sein, die durch eine zylindrische Wandung des Stempels 2 begrenzt sein kann. In dieser Öffnung kann ein Ventilsitz 3 angeordnet sein. Der Ventilsitz 3 kann mit dem Stempel 2 der Gießform fest verbunden sein oder sogar einteilig ausgeführt sein, so wie das in den Figuren 1 bis 10 dargestellt ist.

Der Ventilsitz 3 kann als ein Hohlzylinder ausgeführt sein, der auf einer in Richtung der Öffnung im Stempel 2 ausgerichteten Kopfseite 4 bis auf zwei erste Durchführungen 5 geschlossen ist. Die beiden ersten Durchführungen 5 können viertelkreisförmig geformt sein und können vorzugsweise um 180° bezüglich der Zylinderachse des Ventilsitzes 3 gegeneinander verdreht beziehungsweise versetzt angeordnet sein. Im Inneren des Ventilsitzes 3 kann ein gegen den Ventilsitz 3 axial verdrehbarer Ventilkörper 6 angeordnet sein. Der Ventilkörper 6 kann eine in Richtung der Kopfseite 4 des Ventilsitzes 3 ausgerichtete Dichtfläche 7 beziehungsweise Oberfläche aufweisen. Der Ventilkörper 6 kann als abgestufter Hohlzylinder aufgebaut sein, dessen vorderer Teil in den Ventilsitz 3 eingeschraubt oder eingesteckt sein kann.

In der Dichtfläche 7 können zwei zweite Durchführungen 8 angeordnet sein. Die beiden zweiten Durchführungen 8 können analog den ersten Durchführungen 5 viertelkreisförmig geformt sein und können vorzugsweise um 180° bezüglich der Zylinderachse des Ventilkörpers 6 gegeneinander verdreht angeordnet sein. Der Ventilsitz 3 und der Ventilkörper 6 bilden zusammen ein Ventil der Vorrichtung. In den Ventilkörper 6 kann ein Adapterelement 9 zum flüssigkeitsdichten Verbinden einer Knochenzementkartusche 10 eingeschraubt sein. Die Knochenzementkartusche 10 und das Adapterelement 9 können Teil der erfindungsgemäßen Vorrichtung sein. Der Ventilkörper 6 kann an seiner offenen Seite, die von der Dichtfläche 7 abgewandt ist, als Anschluss 11 zum Verbinden des Adapterelements 9 ausgeformt sein.

Die Knochenzementkartusche 10 kann an ihrer Vorderseite eine Austragsöffnung 12 zum Austragen des Knochenzementteigs 50 aus der Knochenzementkartusche 10 aufweisen. Die Austragsöffnung 12 der Knochenzementkartusche 10 kann in der Vorderseite eines Austragsrohrs 13 der Knochenzementkartusche 10 angeordnet sein. Die Austragsöffnung 12 kann auch in dem Adapterelement 9 angeordnet sein und durch dieses begrenzt werden. Das Adapterelement 9 kann die Knochenzementkartusche 10 an ihrer Vorderseite bis auf die Austragsöffnung 12 und gegebenenfalls bis auf einen Vakuumanschluss 44 verschließen, wobei das Austragsrohr 13 durch das Adapterelement 9 hindurchragen kann.

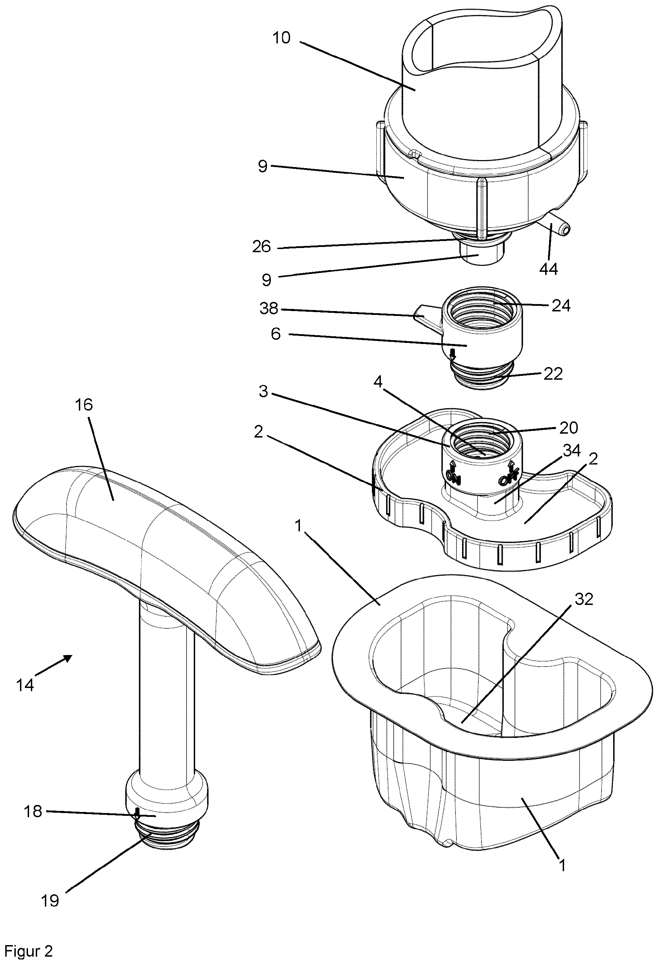

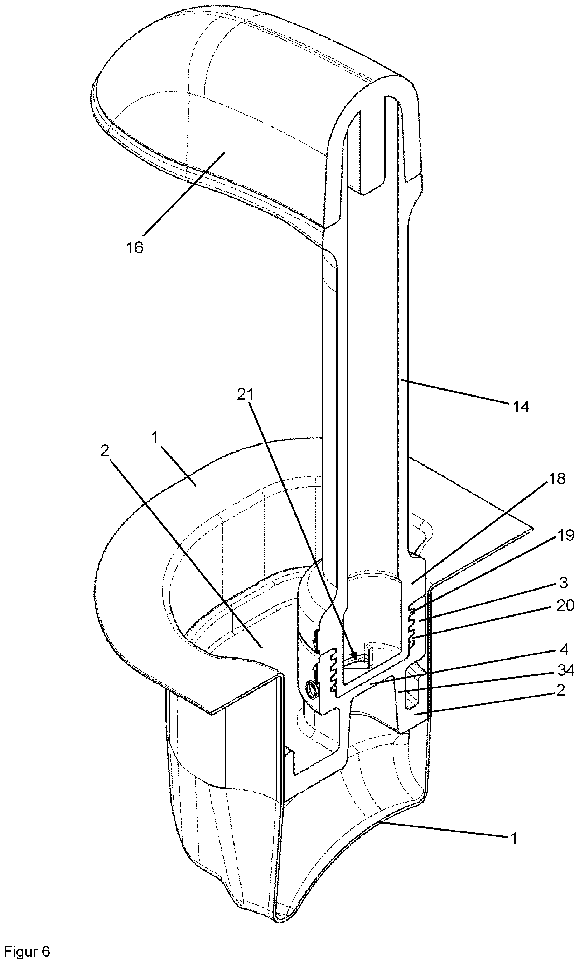

Die Vorrichtung kann ein Griffstück 14 aufweisen, das an einem Ende einen Griff 16 formt (siehe Figuren 2, 4, 6, 7 und 10). Am gegenüberliegenden Ende des Griffstücks 14 kann ein Ventilkörper 18 mit einem Außengewinde 19 angeordnet sein. Der Ventilkörper 18 des Griffstücks 14 kann in dem Ventilsitz 3 und gegen den Ventilsitz 3 axial verdrehbar angeordnet sein beziehungsweise werden (siehe Figuren 6, 7und 10). Der Ventilkörper 18 des Griffstücks 14 kann eine in Richtung der Kopfseite 4 des Ventilsitzes 3 ausgerichtete Dichtfläche beziehungsweise Oberfläche aufweisen. Der Ventilkörper 18 kann in den Ventilsitz 3 eingeschraubt oder eingesteckt sein.

In der Dichtfläche können zwei zweite Durchführungen 21 des Griffstücks 14 angeordnet sein. Die beiden zweiten Durchführungen 21 können analog den ersten Durchführungen 5 viertelkreisförmig geformt sein und können vorzugsweise um 180° bezüglich der Zylinderachse des Ventilkörpers 18 des Griffstücks 14 gegeneinander verdreht angeordnet sein. Auch der Ventilsitz 18 des Griffstücks 14 und der Ventilkörper 6 bilden zusammen ein Ventil der Vorrichtung. Das Griffstück 14 kann im inneren Hohl ausgeführt sein. Der Hohlraum im Inneren des Griffstücks 14 kann mit den beiden zweiten Durchführungen 21 des Griffstücks 14 verbunden sein. Dadurch kann der Hohlraum im Inneren des Griffstücks einen Auffangbehälter zur Aufnahme von überschüssigem Knochenzementteig 50 bilden, wobei der überschüssige Knochenzementteig 50 aus der Gießform durch die zwei zweiten Durchführungen 21 in den Auffangbehälter strömen kann, wenn das durch den Ventilkörper 18 und den Ventilsitz 3 gebildete Ventil in einer geöffneten Stellung ist, in der die beiden ersten Durchführungen 5 im Ventilsitz 3 für den Knochenzementteig 50 flüssigkeitsdurchlässig mit den beiden zweiten Durchführungen 21 des Griffstücks 14 verbunden sind beziehungsweise übereinanderliegend angeordnet sind.

Der Stempel 2 kann in die trogförmige Form 1 hineingeschoben werden. Durch Einstecken des Stempels 2 in die trogförmige Form 1 kann die Gießform nach außen verschlossen werden. Zwischen der trogförmigen Form 1 und dem Stempel 2 kann im ineinandergesteckten Zustand ein Spalt zur Entlüftung des Innenraums der Gießform vorhanden sein (in den Figuren 1 bis 10 nicht zu sehen). Durch den Spalt kann Luft oder Gas aus dem Inneren der geschlossenen Gießform entweichen, wenn ein Knochenzementteig 50 in die Gießform eingefüllt wird.

Der Ventilsitz 3 kann an seiner Innenseite ein Innengewinde 20 aufweisen. Das Außengewinde 19 des Griffstücks 14 passt zu dem Innengewinde 20 des Ventilsitzes 3, so dass der Ventilkörper 18 des Griffstücks 14 in den Ventilsitz 3 eingeschraubt werden kann.

An der der Dichtfläche 7 zugewandten vorderen Hälfte des Ventilkörpers 6 kann der Ventilkörper 6 an seiner Außenseite ein zum Innengewinde 20 des Ventilsitzes 3 passendes Außengewinde 22 aufweisen. Der Ventilkörper 6 kann mit seinem Außengewinde 22 in das Innengewinde 20 des Ventilsitzes 3 eingeschraubt werden.

Durch Einschrauben des Ventilkörpers 6 in den Ventilsitz 3 bis zum Anschlag können die ersten Durchführungen 5 und die zweiten Durchführungen 8 in Überdeckung zueinander gebracht werden. Ebenso können durch das Einschrauben des Ventilkörpers 18 des Griffstücks 14 in den Ventilsitz 3 bis zum Anschlag die ersten Durchführungen 5 und die zweiten Durchführungen 21 in Überdeckung zueinander gebracht werden. Das Ventil befindet sich dann im geöffneten Zustand. Ein Knochenzementteig 50 kann in diesem geöffneten Zustand durch die ersten Durchführungen 5 und durch die zweiten Durchführungen 8 aus der Knochenzementkartusche 10 in die Gießform fließen, beziehungsweise ein Knochenzementteig 50 kann in diesem geöffneten Zustand durch die ersten Durchführungen 5 und durch die zweiten Durchführungen 21 des Griffstücks 14 aus der Gießform in den Auffangbehälter im Griffstück 14 fließen.

Durch eine Vierteldrehung (um 90°) des Ventilkörpers 6 oder des Ventilkörpers 18 gegen den Ventilsitz 3, das heißt durch ein Herausschrauben des Ventilkörpers 6 oder des Ventilkörpers 18 aus dem Ventilsitz 3, können die ersten Durchführungen 5 und die zweiten Durchführungen 8 oder die zweiten Durchführungen 21 gegeneinander versetzt werden, so dass die Dichtfläche 7 des Ventilkörpers 6 die ersten Durchführungen 5 des Ventilsitzes 3 abdeckt und die geschlossenen Bereiche der Kopfseite 4 des Ventilsitzes 3 die zweiten Durchführungen 8 des Ventilkörpers 6 oder die zweiten Durchführungen 21 des Ventilkörpers 18 abdeckt. Das Ventil befindet sich dann im geschlossenen Zustand. Durch den geringen Hub des Ventilkörpers 6 oder des Ventilkörpers 18 gegen den Ventilsitz 3 bei einer Vierteldrehung, ist der zwischen dem Ventilkörper 6 oder dem Ventilkörper 18 und dem Ventilsitz 3 entstehende Spalt so schmal (weniger als 1 mm breit), dass ein normaler und erst recht ein hochviskoser Knochenzementteig 50 nicht durch den Spalt dringen können. Dies gilt insbesondere, weil der Knochenzementteig 50 im Spalt von seiner eigentlichen Flussrichtung um 90° abgelenkt wird.

In der Rückseite des Ventilkörpers 6 kann im Anschluss 11 ein Innengewinde 24 angeordnet sein. Das Adapterelement 9 weist an seiner Vorderseite ein zum Innengewinde 24 passendes Außengewinde 26 auf. Das Adapterelement 9 kann so in den Anschluss 11 des Ventilkörpers 6 eingeschraubt werden. Hierdurch kann eine flüssigkeitsdichte Verbindung der Knochenzementkartusche 10 zum Ventilkörper 6 und damit in die Gießform erzeugt werden.

Das Innengewinde 20 des Ventilsitzes 3, das Außengewinde 19 des Ventilkörpers 18 des Griffstücks 14, das Außengewinde 22 des Ventilkörpers 6, das Innengewinde 24 des Ventilkörpers 6 und das Außengewinde 26 des Adapterelements 9 können alle den gleichen Drehsinn aufweisen, das heißt, dass alle diese Gewinde Rechtsgewinde oder Linksgewinde sind. Dadurch kann das Ventil durch Einschrauben des Adapterelements 9 in den Anschluss 11 und gleichsinniges Weiterdrehen des Adapterelements 9 geöffnet werden. Gleichzeitig dichtet auch der Ventilkörper 6 gegen den Ventilsitz 3 ab. Ebenso kann dadurch das Ventil durch Einschrauben des Griffstücks 14 in den Ventilsitz 3 geöffnet werden. Gleichzeitig dichtet auch der Ventilkörper 18 gegen den Ventilsitz 3 ab.

Das Adapterelement 9 kann über ein Rastmittel 28 am Adapterelement 9 mit einer Gegenrastung 30 an einer zylindrischen Wandung der Knochenzementkartusche 10 verbunden werden oder sein. Zur Abdichtung kann eine umlaufende Dichtung 48 vorgesehen sein, die die zylindrische Wandung der Knochenzementkartusche 10 gegen das Adapterelement 9 abdichtet.

Die Gießform kann eine Bodenplatte 32 zum Formen einer Gleitfläche einer mit der Gießform geformten Kniespacer-Komponente (nicht gezeigt) aufweisen. Für den vorliegenden Fall kann dies ein Tibia-Plateau sein. Die Bodenplatte 32 kann den Grund beziehungsweise den Boden der trogförmigen Form 1 bilden. Der Stempel 2 kann in Richtung der Bodenplatte 32 in die trogförmige Form 1 hineingeschoben werden. Dadurch kann ein in die Gießform gefüllter Knochenzementteig 50 gegen die Bodenplatte 32 der trogförmigen Form 1 gedrückt werden. Ferner kann die Gießform und insbesondere der Stempel 2 der Gießform ein Schaft-Formteil 34 zum Ausformen eines Schafts einer Tibia-Komponente aufweisen.

Ein Handgriff 38 kann mit dem Ventilkörper 6 verbunden sein. Der Ventilkörper 6 kann mit dem Handgriff 38 im Ventilsitz 3 manuell gedreht werden.

In dem Adapterelement 9 kann ein Vakuumanschluss 44 angeordnet sein, mit dem ein Innenraum der Knochenzementkartusche 10, in dem der Knochenzementteig 50 gemischt wird, evakuiert werden kann. Dadurch kann der Knochenzementteig 50 unter Vakuum gemischt werden.

Im zylindrischen Innenraum der Knochenzementkartusche 10 kann ein Kolben 46 zum Austreiben des Knochenzementteigs 50 aus der Knochenzementkartusche 10 durch den Ventilkörper 6 und den Ventilsitz 3 in die Gießform 1 angeordnet sein. Hierzu kann der Kolben 46 an seiner Außenseite zylindrisch geformt und über zwei umlaufende Dichtungen 54 gegen den zylindrischen Innenraum abgedichtet sein. Durch Vortreiben des Kolbens 46 kann der Knochenzementteig 50 aus der Austragsöffnung 12 der Knochenzementkartusche 10 in beziehungsweise durch das geöffnete Ventil bestehend aus dem Ventilkörper 6 und dem Ventilsitz 3 gepresst werden.

In dem Adapterelement 9 kann eine Porenscheibe 52 angeordnet sein. Die Porenscheibe 52 ist für den Knochenzementteig 50 und seine Ausgangskomponenten undurchlässig. Der Vakuumanschluss 44 kann von der Porenscheibe 52 abgedeckt sein. Hierdurch wird verhindert, dass ein Knochenzementpulver als Ausgangskomponente des Knochenzementteigs 50 in den Vakuumanschluss 44 eindringen kann.

Der Ablauf eines erfindungsgemäßen Verfahrens kann anhand der Figuren 1 bis 10 anhand der ersten erfindungsgemäßen Vorrichtung erläutert werden. Ein Knochenzementteig 50 kann in der Knochenzementkartusche 10 unter Vakuum gemischt werden. Anschließend kann die Knochenzementkartusche 10 mit dem Adapterelement 9 in den Anschluss 11 des Ventilkörpers 6 geschraubt werden. Beim Einschrauben der Adapterelements 9 kann das Ventil in die geöffnete Stellung überführt werden, indem der Ventilkörper 6 bis zum Anschlag in den Ventilsitz 3 geschraubt wird. Diese Situation ist in den Figuren 1, 3 und 8 dargestellt.

Anschließend wird durch Vortreiben des Kolbens 46 der Knochenzementteig 50 aus der Knochenzementkartusche 10 durch das Ventil und durch die sich in Deckung befindlichen ersten Durchführungen 5 und zweiten Durchführungen 8 in die Gießform gepresst. Dabei kann der Stempel 2 aus der trogförmigen Form 1 herausgedrückt werden. Um die Kniespacer-Komponente durch Anpressen des Knochenzementteigs 50 an die Innenwände der Gießform zu formen, sollte der Anwender einen Druck auf den Stempel 2 über die Knochenzementkartusche 10 beim Einpressen des Knochenzementteigs 50 aufrechterhalten. Durch Schließen des Ventils durch manuelles Bedienen des Handgriffs 38 und dadurch Drehen des Ventilkörpers 6 um eine Vierteldrehung gegen den Ventilsitz 3 kann zwischendurch eine neue Knochenzementkartusche 10 angesetzt werden, falls das Volumen des Knochenzementteigs 50 einer einzigen Knochenzementkartusche 10 nicht ausreicht, um die Gießform ausreichend zu füllen. Dabei kann der in der Gießform enthaltene Knochenzementteig 50 nicht wieder ausfließen, das die ersten Durchgänge 5 und die zweiten Durchgänge 8 in der geschlossenen Stellung des Ventils abgedeckt sind und der Spalt dazwischen nicht ausreicht, um den viskosen Knochenzementteig 50 hindurchfließen lassen zu können.

Irgendwann ist die Gießform ausreichend mit dem Knochenzementteig 50 gefüllt. Luft oder Gas aus der Gießform kann durch den Spalt zwischen der trogförmigen Form 1 und dem Stempel 2 aus der Gießform entweichen. Wenn die gewünschte Höhe der Kniespacer-Komponente erreicht ist, indem der Stempel 2 genau richtig weit aus der trogförmigen Form herausgedrückt ist, kann das Ventil geschlossen bleiben und der Knochenzementteig 50 in der Gießform aushärten, um die Kniespacer-Komponente zu erzeugen. Durch Schließen des Ventils mit dem Handgriff 38 wird der Knochenzementteig 50 abgeschert beziehungsweise abgeschnitten. Die Knochenzementkartusche 10 kann abgeschraubt und entfernt werden.

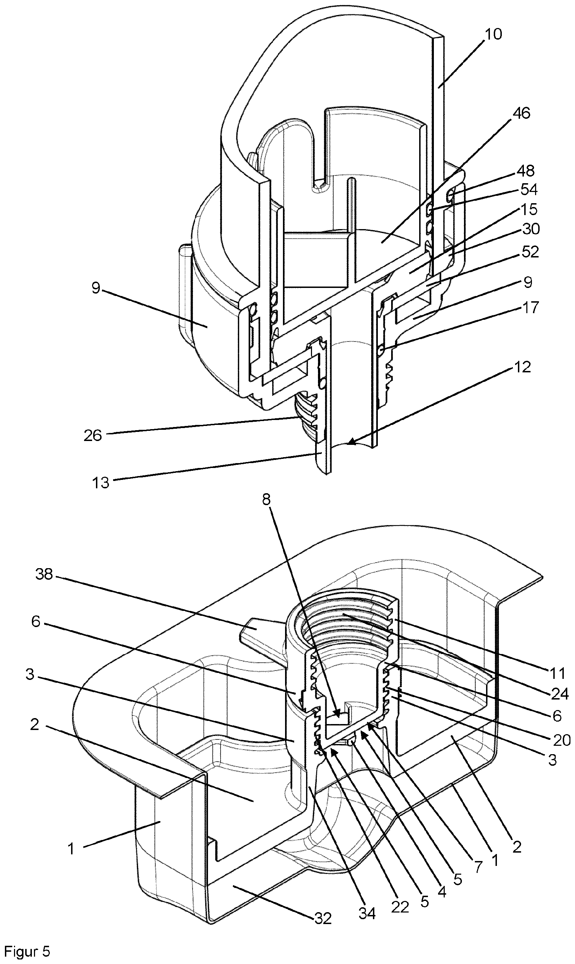

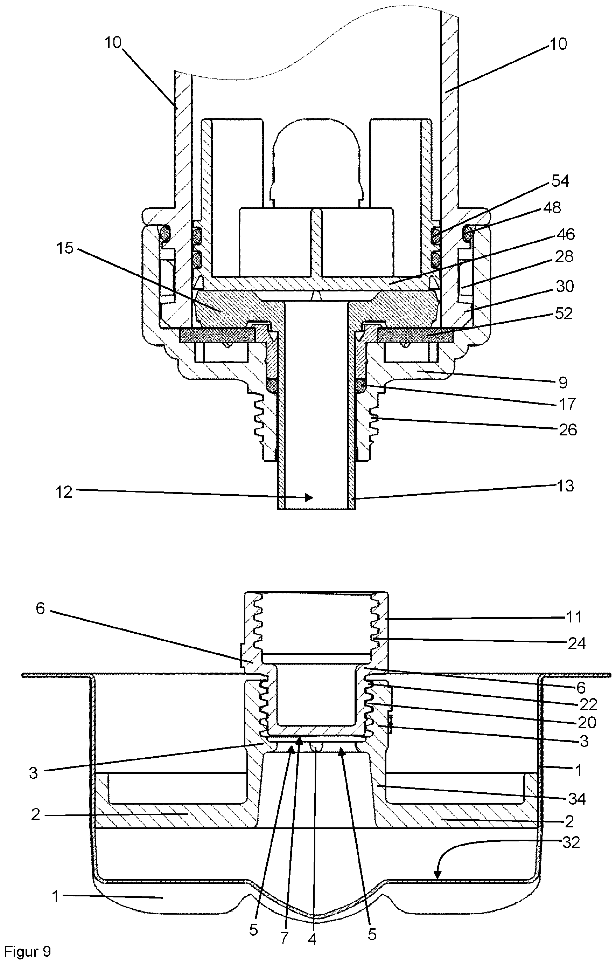

Eventuell verbliebene dünne Verbindungen reißen oder brechen ohne weiteres ab. Diese Situation ist in Figur 5 und Figur 9 dargestellt.

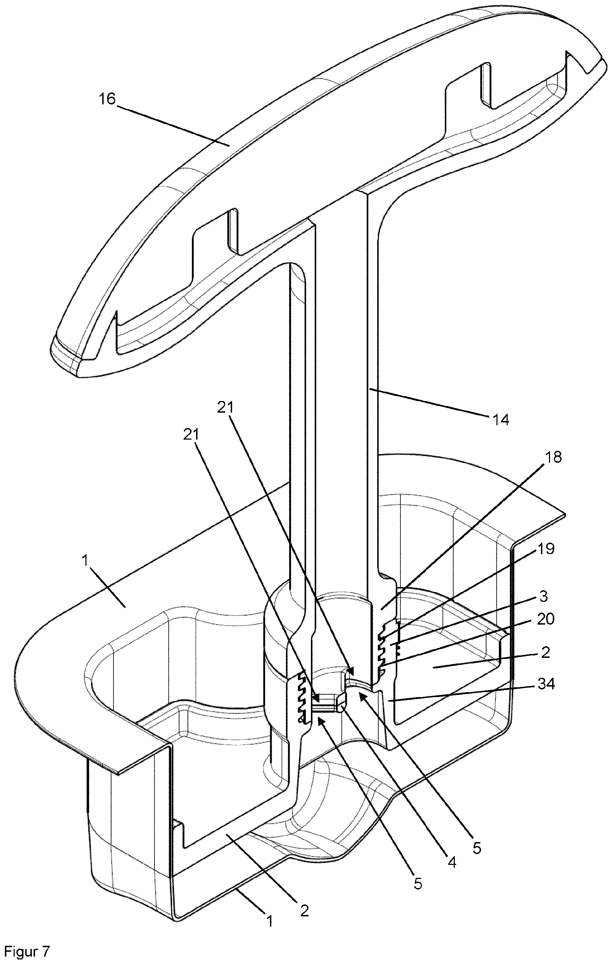

Wenn Knochenzementteig 50 im Überschuss in die Gießform gefüllt wurde oder die Höhe der Kniespacer-Komponente zu groß ist, kann zur Einstellung der gewünschten Höhe der zu erzeugenden Kniespacer-Komponente das Griffstück 14 in das Innengewinde 20 des Ventilsitzes 3 geschraubt werden. Dabei bildet auch der Ventilkörper 18 mit dem Ventilsitz 3 ein neues Ventil. Durch das Einschrauben des Griffstücks 14 bis zum Anschlag wird das Ventil in die geöffnete Stellung gebracht. Diese Situation ist in den Figuren 6 und 7 dargestellt.

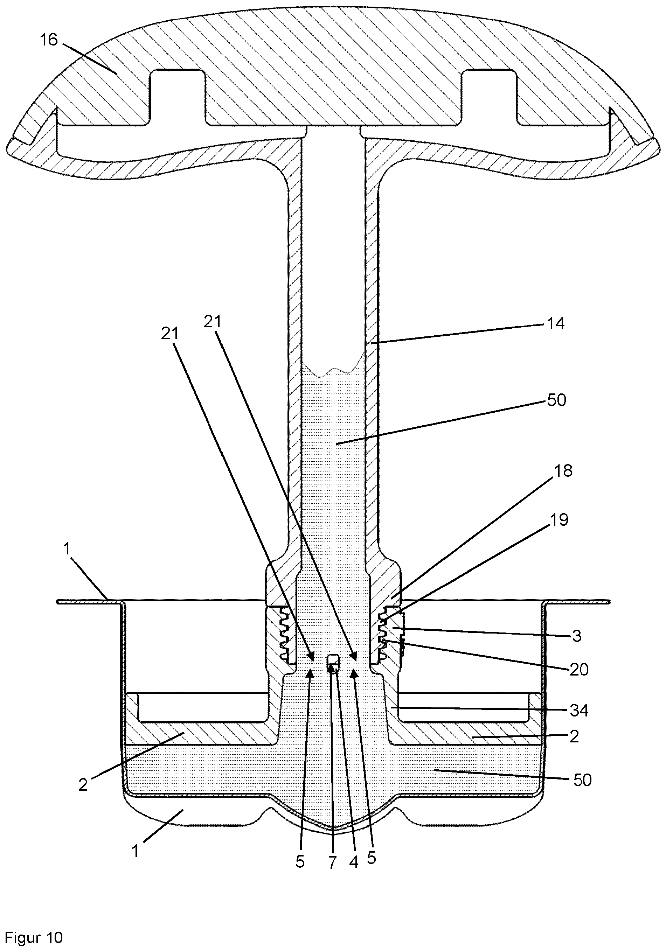

Durch ein Verringern der Höhe des Innenraums der Gießform durch Einschieben des Stempels 2 in die trogförmige Form 1 wird ein Teil des Knochenzementteigs 50 aus der Gießform heraus und durch die beiden ersten Durchführungen 5 und die beiden zweiten Durchführungen 21 hindurch in den Auffangbehälter des Griffstücks 14 gedrückt. Dies ist in Figur 10 dargestellt. Das Griffstück 14 wird nun um eine Vierteldrehung (um 90°) gedreht und damit die zwei zweiten Öffnungen 21 gegen die beiden ersten Öffnungen 5 verdreht und somit das Ventil geschlossen. Dabei wird der Knochenzementteig 50 im Ventil abgeschert oder weitgehend abgeschert.

In diesem Zustand kann der Knochenzementteig 50 in der Gießform ausgehärtet werden. Anschließend wird die so geformte Tibia-Komponente aus der Gießform entnommen. Ein durch den Ventilsitz 3 und die ersten Durchgänge 5 verursachter Abguss kann abgeschnitten und entfernt werden. Die Oberfläche der Tibia-Komponente kann poliert werden und/oder beschichtet werden, beispielsweise mit Antibiotika.

Anstatt einer Gießform zum Formen einer Tibia-Komponente kann ohne weiteres auch eine Gießform zum Formen einer Femur-Komponente verwendet werden.

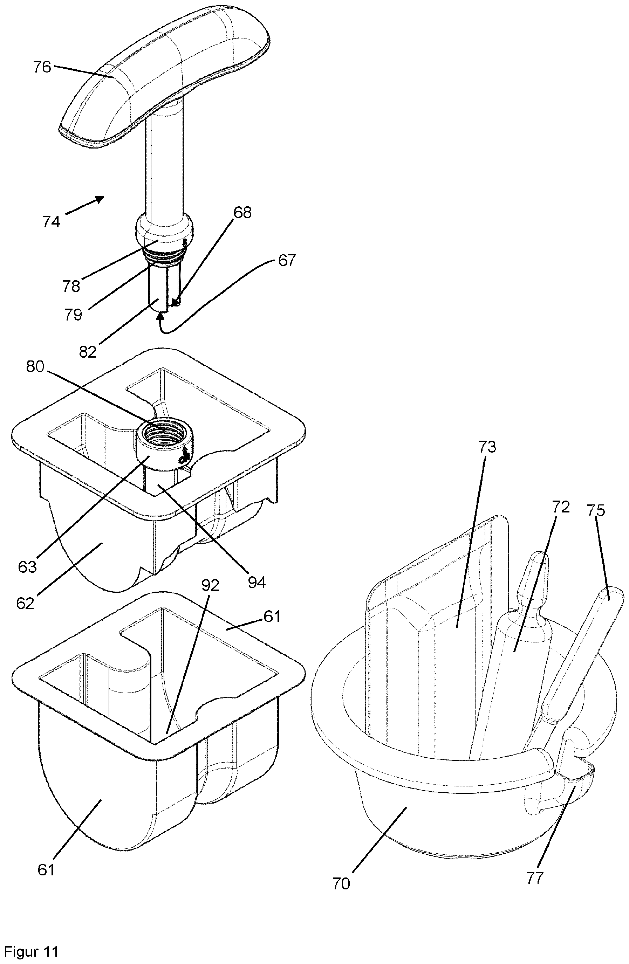

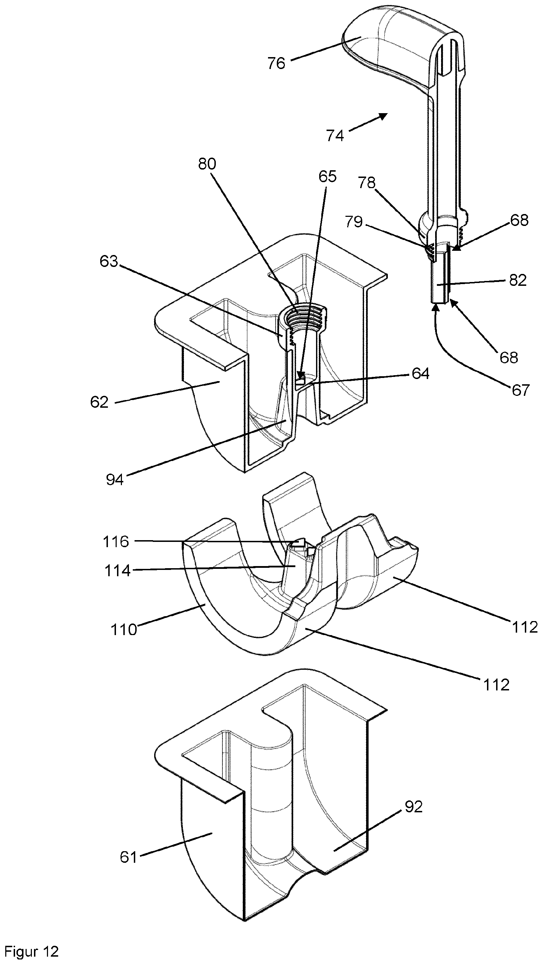

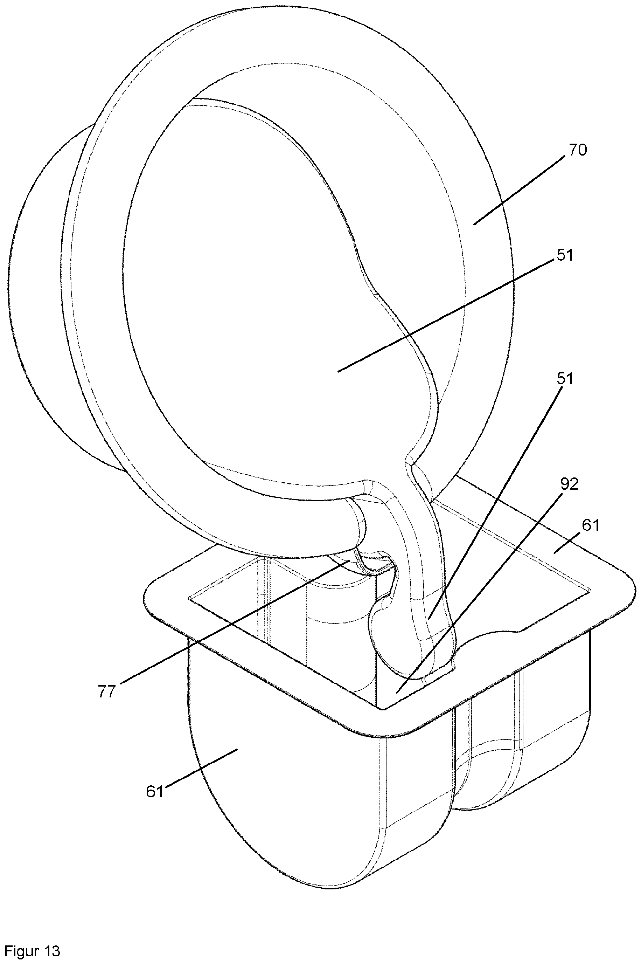

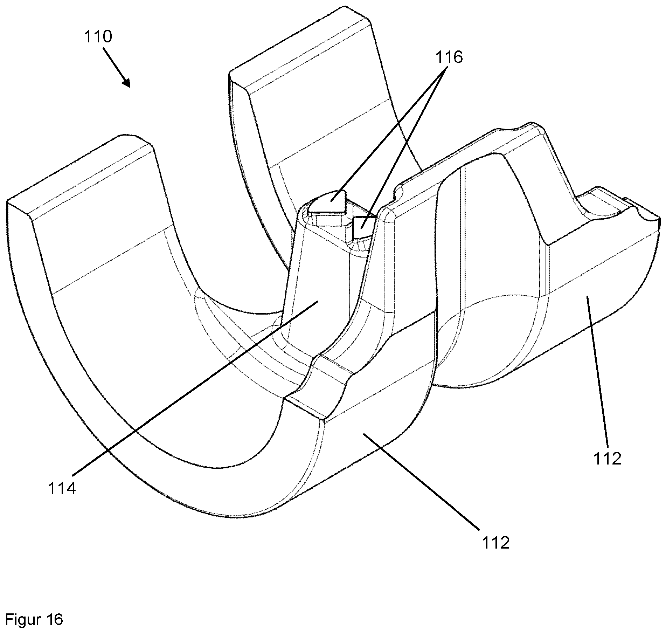

In den Figuren 11 bis 15 sind Abbildungen eines zweiten Ausführungsbeispiels einer erfindungsgemäßen Vorrichtung zur Herstellung einer Kniespacer-Komponente 110 in Form einer Femur-Komponente für einen Kniespacer und Teile der Vorrichtung in verschiedenen Ansichten gezeigt. Die Figur 16 zeigt die Kniespacer-Komponente 110, die mit einer solchen zweiten erfindungsgemäßen Vorrichtung hergestellt wurde, als Resultat eines erfindungsgemäßen Verfahrens, dessen Verfahrensschritte chronologisch in den Figuren 13 bis 16 gezeigt sind.

Die zweite erfindungsgemäße Vorrichtung ist zur Herstellung einer Femur-Komponente eines Kniespacers geeignet und vorgesehen. Die Vorrichtung umfasst eine Gießform, die sich aus zwei Teilen zusammensetzt. Die Gießform kann eine trogförmige Form 61 und einen Stempel 62 aufweisen. Der Stempel 62 kann in die trogförmige Form 61 eingesetzt werden und kann in die trogförmigen Form 61 eingeschoben und vorzugsweise auch wieder herausgezogen werden. Die trogförmige Form 61 kann kostengünstig aus Kunststofffolie gefertigt werden. Die Kunststofffolie kann mehrere Schichten aufweisen. An einer Seite des Stempels 62 kann eine Öffnung zum Durchfließen von Knochenzementteig 51 ausgeformt sein, die durch eine zylindrische Wandung des Stempels 62 begrenzt sein kann. In dieser Öffnung kann ein Ventilsitz 63 angeordnet sein. Der Ventilsitz 63 kann mit dem Stempel 62 der Gießform fest verbunden sein oder sogar einteilig ausgeführt sein, so wie das in den Figuren 11 bis 15 dargestellt ist.

Der Ventilsitz 63 kann als ein Hohlzylinder ausgeführt sein, der auf einer in Richtung der Öffnung im Stempel 62 ausgerichteten Kopfseite 64 bis auf zwei erste Durchführungen 65 geschlossen ist. Die beiden ersten Durchführungen 65 können viertelkreisförmig geformt sein und können vorzugsweise um 180° bezüglich der Zylinderachse des Ventilsitzes 63 gegeneinander verdreht beziehungsweise versetzt angeordnet sein.

Im Inneren des Ventilsitzes 63 kann ein gegen den Ventilsitz 63 axial verdrehbarer Ventilkörper 78 angeordnet sein oder werden. Der Ventilkörper 78 kann eine in Richtung der Kopfseite 64 des Ventilsitzes 63 ausgerichtete Dichtfläche 67 beziehungsweise Oberfläche aufweisen, die durch einen Verschlussstifts 82 zum Verschließen der zwei ersten Durchführungen 65 begrenzt sein kann.

In dem Ventilkörper 78 können zwei zweite Durchführungen 68 angeordnet sein. Die beiden zweiten Durchführungen 68 können analog den ersten Durchführungen 65 viertelkreisförmig geformt sein und können vorzugsweise um 180° bezüglich der Zylinderachse des Ventilkörpers 78 gegeneinander verdreht angeordnet sein. Der Ventilsitz 63 und der Ventilkörper 78 bilden zusammen ein Ventil der Vorrichtung.

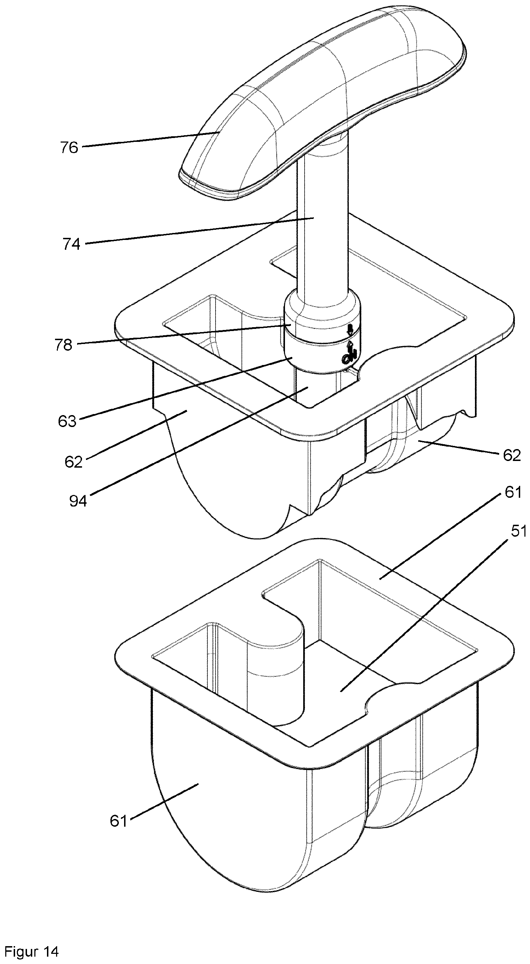

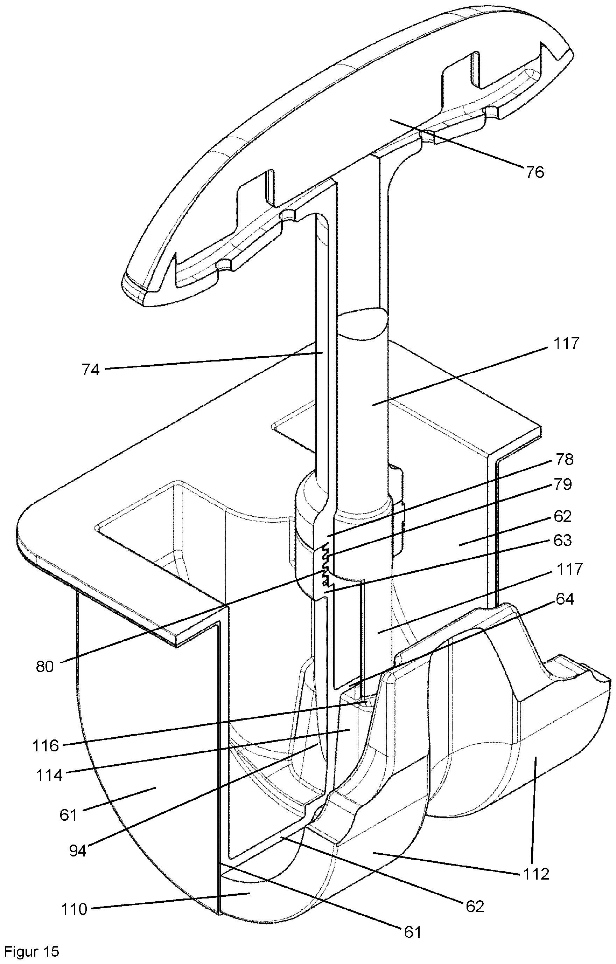

Die Vorrichtung kann ein Griffstück 74 aufweisen, das an einem Ende einen Griff 76 formt (siehe Figuren 11, 12, 14 und 15). Am gegenüberliegenden Ende des Griffstücks 74 kann der Ventilkörper 78 mit einem Außengewinde 79 angeordnet sein. Der Ventilkörper 78 kann mittels des Griffstücks 74 in dem Ventilsitz 63 und gegen den Ventilsitz 63 axial verdrehbar angeordnet sein beziehungsweise werden (siehe Figuren 14 und 15). Der Ventilkörper 78 kann den in Richtung der Kopfseite 64 des Ventilsitzes 63 ausgerichteten Verschlussstift 82 aufweisen, der die Dichtfläche 67 des Ventilkörpers 78 bildet. Der Ventilkörper 78 kann in den Ventilsitz 63 eingeschraubt oder eingesteckt sein.

Das Griffstück 74 kann im inneren Hohl ausgeführt sein. Der Hohlraum im Inneren des Griffstücks 74 kann mit den beiden zweiten Durchführungen 68 des Griffstücks 74 verbunden sein. Dadurch kann der Hohlraum im Inneren des Griffstücks einen Auffangbehälter zur Aufnahme von überschüssigem Knochenzement 117 bilden, wobei der überschüssige Knochenzementteig 51 aus der Gießform durch die zwei zweiten Durchführungen 68 in den Auffangbehälter strömen kann, wenn das durch den Ventilkörper 78 und den Ventilsitz 63 gebildete Ventil in einer geöffneten Stellung ist, in der die beiden ersten Durchführungen 65 im Ventilsitz 63 für den Knochenzementteig 51 flüssigkeitsdurchlässig mit den beiden zweiten Durchführungen 68 verbunden sind beziehungsweise übereinanderliegend angeordnet sind.

Der Stempel 62 kann in die trogförmige Form 61 hineingeschoben werden. Durch Einstecken des Stempels 62 in die trogförmige Form 61 kann die Gießform nach außen verschlossen werden. Zwischen der trogförmigen Form 61 und dem Stempel 62 kann im ineinandergesteckten Zustand ein Spalt zur Entlüftung des Innenraums der Gießform vorhanden sein (in den Figuren 11 bis 15 nicht zu sehen). Durch den Spalt kann Luft oder Gas aus dem Inneren der geschlossenen Gießform entweichen, wenn ein Knochenzementteig 51 in die Gießform eingefüllt wird.

Der Ventilsitz 63 kann an seiner Innenseite ein Innengewinde 80 aufweisen. Das Außengewinde 79 des Griffstücks 74 passt zu dem Innengewinde 80 des Ventilsitzes 63, so dass der Ventilkörper 78 in den Ventilsitz 63 eingeschraubt werden kann.

Durch Einschrauben des Ventilkörpers 78 in den Ventilsitz 63 bis zum Anschlag können die ersten Durchführungen 65 und die zweiten Durchführungen 68 in Überdeckung zueinander gebracht werden. Das Ventil befindet sich dann im geöffneten Zustand. Ein Knochenzementteig 51 kann in diesem geöffneten Zustand durch die ersten Durchführungen 65 und durch die zweiten Durchführungen 68 aus der Gießform in den Auffangbehälter im Griffstück 74 fließen.

Durch eine Vierteldrehung (um 90°) des Ventilkörpers 78 gegen den Ventilsitz 63, das heißt durch ein Herausschrauben des Ventilkörpers 78 aus dem Ventilsitz 63, können die ersten Durchführungen 65 und die zweiten Durchführungen 68 gegeneinander versetzt werden, so dass die Dichtfläche 67 des Ventilkörpers 78 die ersten Durchführungen 65 des Ventilsitzes 63 abdeckt und die geschlossenen Bereiche der Kopfseite 64 des Ventilsitzes 63 die zweiten Durchführungen 68 des Ventilkörpers 78 abdeckt. Das Ventil befindet sich dann im geschlossenen Zustand. Durch den geringen Hub des Ventilkörpers 78 gegen den Ventilsitz 63 bei einer Vierteldrehung, ist der zwischen dem Ventilkörper 78 und dem Ventilsitz 63 entstehende Spalt so schmal (weniger als 1 mm breit), dass ein normaler und erst recht ein hochviskoser Knochenzementteig 51 nicht durch den Spalt dringen können. Dies gilt insbesondere, weil der Knochenzementteig 51 im Spalt von seiner eigentlichen Flussrichtung um 90° abgelenkt wird.

Das Innengewinde 80 des Ventilsitzes 63 und das Außengewinde 79 des Ventilkörpers 78 können alle den gleichen Drehsinn aufweisen, das heißt, dass alle diese Gewinde Rechtsgewinde oder Linksgewinde sind. Dadurch kann das Ventil durch Einschrauben des Griffstücks 74 in den Ventilsitz 63 geöffnet werden. Gleichzeitig dichtet der Ventilkörper 78 gegen den Ventilsitz 63 ab.

Die Gießform kann eine Bodenplatte 92 zum Formen einer Gleitfläche einer mit der Gießform geformten Kniespacer-Komponente 110 aufweisen. Für den vorliegenden Fall kann dies eine Gleitfläche 112 von Strukturen der Femur-Komponente sein, die die Kondylen eines Femurs nachbilden (siehe Figur 16). Die Kniespacer-Komponente 110 kann zudem einen Schaft 114 und am Schaft 114 einen Abguss 116 aufweisen. Die Bodenplatte 92 kann den Grund beziehungsweise den Boden der trogförmigen Form 61 bilden. Der Stempel 62 kann in Richtung der Bodenplatte 92 in die trogförmige Form 61 hineingeschoben werden. Dadurch kann ein in die Gießform gefüllter Knochenzementteig 51 gegen die Bodenplatte 92 der trogförmigen Form 61 gedrückt werden. Ferner kann die Gießform und insbesondere der Stempel 62 der Gießform ein Schaft-Formteil 94 zum Ausformen des Schafts 114 der Kniespacer-Komponente 110 aufweisen.

Der Knochenzementteig 51 kann in einem Mischbecher 70 (siehe Figuren 11 und 13) gemischt werden. Die Vorrichtung kann hierzu einen Monomerflüssigkeitsbehälter 72 enthaltend Monomerflüssigkeit und einen Knochenzementpulverbehälter 73 enthaltend Knochenzementpulver aufweisen. Der Knochenzementteig 51 kann in dem Mischbecher 70 aus dem Knochenzementpulver und der Monomerflüssigkeit gemischt werden. Zum Mischen des Knochenzementteigs 51 in dem Mischbecher 70 kann die Vorrichtung einen Spatel 75 oder ein anderes Mischwerkzeug aufweisen. Der Monomerflüssigkeitsbehälter 72 kann eine Glasampulle sein. Der Mischbecher 70 kann eine Tülle 77 zum Ausgießen des Knochenzementteigs 51 aufweisen. Der gemischte Knochenzementteig 51 kann aus dem Mischbecher 70 über die Tülle 77 des Mischbechers 70 in die trogförmige Form 61 gefüllt werden (siehe Figur 13).

Alternativ kann auch eine Knochenzementkartusche zum Herstellen und Einfüllen eines Knochenzementteigs in die trogförmige Form 61 oder in die Gießform analog zu dem ersten Ausführungsbeispiel nach den Figuren 1 bis 10 angewendet werden.

Der Ablauf eines erfindungsgemäßen Verfahrens wird im Folgenden anhand der Figuren 13 bis 16 anhand der zweiten erfindungsgemäßen Vorrichtung erläutert. Ein Knochenzementteig 51 kann in dem Mischbecher 70 gemischt werden. Hierzu wird das Knochenzementpulver aus dem Knochenzementpulverbehälter 73 und die Monomerflüssigkeit aus dem Monomerflüssigkeitsbehälter 72 in den Mischbecher 70 gefüllt und dort mit dem Spatel 75 oder mit einem anderen Hilfsmittel gemischt, bis der Knochenzementteig 51 erhalten wird. Anschließend wird der so gemischte Knochenzementteig 51 aus dem Mischbecher 70 über die Tülle 77 in die trogförmige Form 61 gefüllt (siehe Figur 13).

Zur Einstellung der gewünschten Höhe der zu erzeugenden Kniespacer-Komponente 110 kann nun das Griffstück 74 in das Innengewinde 80 des Ventilsitzes 63 geschraubt werden. Dabei bildet der Ventilkörper 78 mit dem Ventilsitz 63 ein Ventil. Durch das Einschrauben des Griffstücks 74 bis zum Anschlag wird das Ventil in die geöffnete Stellung gebracht. Diese Situation ist in Figur 14 dargestellt.

Durch ein Verringern der Höhe des Innenraums der Gießform durch Einschieben des Stempels 62 in die trogförmige Form 61 wird ein Teil des Knochenzementteigs 51 aus der Gießform heraus und durch die beiden ersten Durchführungen 65 und die beiden zweiten Durchführungen 68 hindurch in den Auffangbehälter des Griffstücks 74 gedrückt. Dies ist in Figur 15 dargestellt. Das Griffstück 74 wird nun um eine Vierteldrehung (um 90°) gedreht und damit die zwei zweiten Öffnungen 68 gegen die beiden ersten Öffnungen 65 verdreht und somit das Ventil geschlossen. Dabei wird der Knochenzementteig 51 im Ventil abgeschert oder weitgehend abgeschert. Hierdurch kann der in dem Auffangbehälter des Griffstücks 74 enthaltene überschüssige Knochenzement 117 von der Kniespacer-Komponente 110 beziehungsweise dem die Kniespacer-Komponente 110 bildenden Knochenzementteig 51 in der Gießform entfernt werden.

In diesem Zustand kann der Knochenzementteig 51 in der Gießform ausgehärtet werden. Anschließend wird die so geformte Kniespacer-Komponente 110 aus der Gießform entnommen (siehe Figur 16). Ein durch den Ventilsitz 63 und die ersten Durchgänge 65 verursachter Abguss 116 kann abgeschnitten und entfernt werden. Die Oberfläche der Kniespacer-Komponente 110 kann poliert werden und/oder beschichtet werden, beispielsweise mit Antibiotika.

Anstatt einer Gießform zum Formen einer Femur-Komponente kann ohne weiteres auch eine Gießform zum Formen einer Tibia-Komponente verwendet werden.

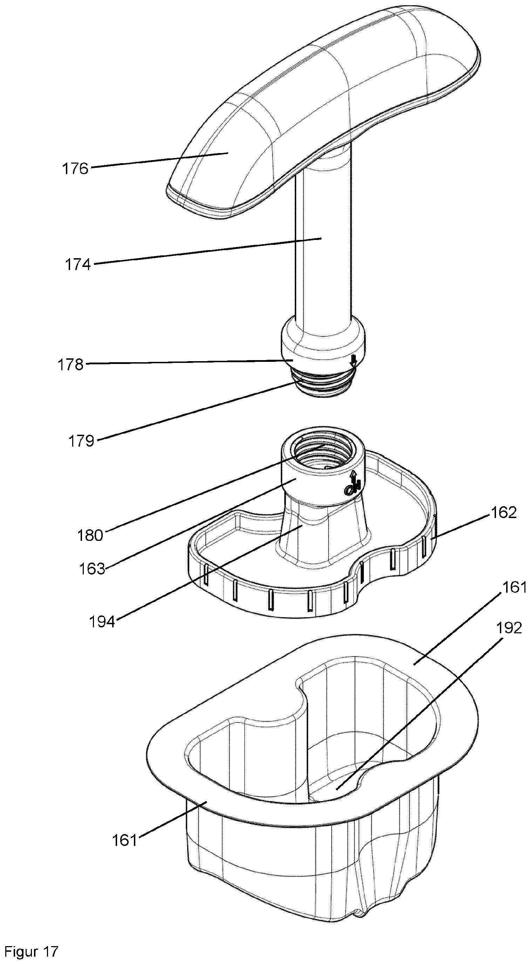

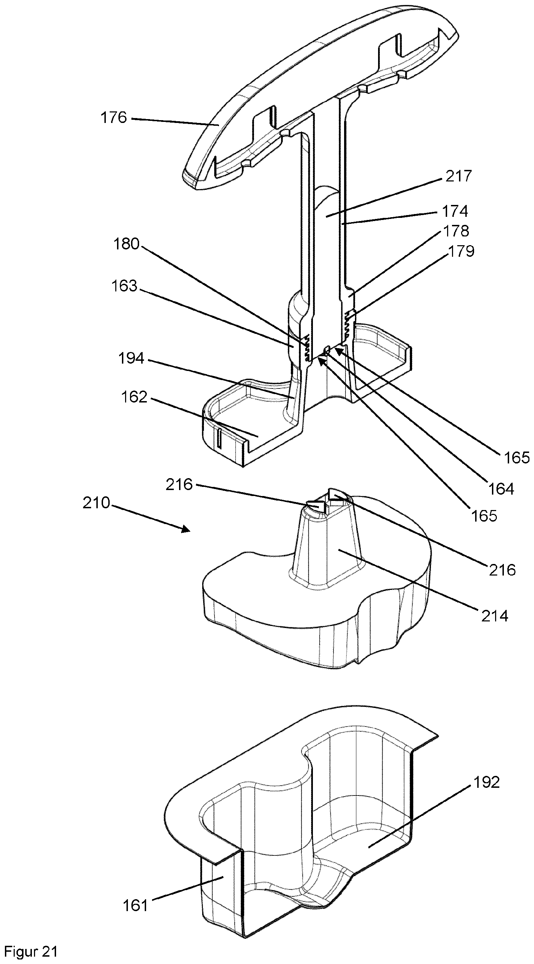

In den Figuren 17 bis 21 sind Abbildungen eines dritten Ausführungsbeispiels einer erfindungsgemäßen Vorrichtung zur Herstellung einer Kniespacer-Komponente 210 in Form einer Tibia-Komponente für einen Kniespacer und Teile der Vorrichtung in verschiedenen Ansichten gezeigt. Die Figur 22 zeigt die Kniespacer-Komponente 210 und einen entfernten überschüssigen Knochenzement 271, die mit einer solchen dritten erfindungsgemäßen Vorrichtung hergestellt wurde, als Resultat eines erfindungsgemäßen Verfahrens, dessen Verfahrensschritte chronologisch in den Figuren 13 bis 16 gezeigt sind.

Die dritte erfindungsgemäße Vorrichtung ist zur Herstellung einer Tibia-Komponente eines Kniespacers geeignet und vorgesehen. Die Vorrichtung umfasst eine Gießform, die sich aus zwei Teilen zusammensetzt. Die Gießform kann eine trogförmige Form 161 und einen Stempel 162 aufweisen. Der Stempel 162 kann in die trogförmige Form 161 eingesetzt werden und kann in die trogförmigen Form 161 eingeschoben und vorzugsweise auch wieder herausgezogen werden. Die trogförmige Form 161 kann kostengünstig aus Kunststofffolie gefertigt werden. Die Kunststofffolie kann mehrere Schichten aufweisen. An einer Seite des Stempels 162 kann eine Öffnung zum Durchfließen von Knochenzementteig (nicht gezeigt) ausgeformt sein, die durch eine zylindrische Wandung des Stempels 162 begrenzt sein kann. In dieser Öffnung kann ein Ventilsitz 163 angeordnet sein. Der Ventilsitz 163 kann mit dem Stempel 162 der Gießform fest verbunden sein oder sogar einteilig ausgeführt sein, so wie das in den Figuren 17 bis 21 dargestellt ist.

Der Ventilsitz 163 kann als ein Hohlzylinder ausgeführt sein, der auf einer in Richtung der Öffnung im Stempel 162 ausgerichteten Kopfseite 164 bis auf zwei erste Durchführungen 165 geschlossen ist. Die beiden ersten Durchführungen 165 können viertelkreisförmig geformt sein und können vorzugsweise um 180° bezüglich der Zylinderachse des Ventilsitzes 163 gegeneinander verdreht beziehungsweise versetzt angeordnet sein.

Im Inneren des Ventilsitzes 163 kann ein gegen den Ventilsitz 163 axial verdrehbarer Ventilkörper 178 angeordnet sein oder werden. Der Ventilkörper 178 kann eine in Richtung der Kopfseite 164 des Ventilsitzes 163 ausgerichtete Dichtfläche 167 beziehungsweise Oberfläche aufweisen, die zum Verschließen der zwei ersten Durchführungen 165 geeignet sein kann.

In dem Ventilkörper 178 können zwei zweite Durchführungen 168 angeordnet sein. Die beiden zweiten Durchführungen 168 können analog den ersten Durchführungen 165 viertelkreisförmig geformt sein und können vorzugsweise um 180° bezüglich der Zylinderachse des Ventilkörpers 178 gegeneinander verdreht angeordnet sein. Der Ventilsitz 163 und der Ventilkörper 178 bilden zusammen ein Ventil der Vorrichtung.

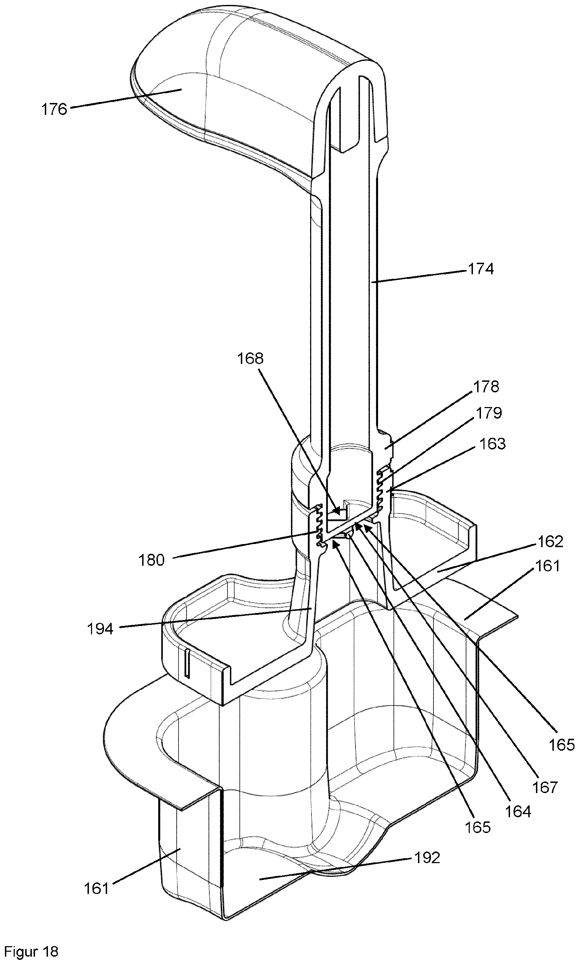

Die Vorrichtung kann ein Griffstück 174 aufweisen, das an einem Ende einen Griff 176 formt (siehe Figuren 17 bis 21). Am gegenüberliegenden Ende des Griffstücks 174 kann der Ventilkörper 178 mit einem Außengewinde 179 angeordnet sein. Der Ventilkörper 178 kann mittels des Griffstücks 174 in dem Ventilsitz 163 und gegen den Ventilsitz 163 axial verdrehbar angeordnet sein beziehungsweise werden (siehe Figuren 18 bis 21). Der Ventilkörper 178 kann in den Ventilsitz 163 eingeschraubt oder eingesteckt sein.

Das Griffstück 174 kann im inneren Hohl ausgeführt sein. Der Hohlraum im Inneren des Griffstücks 174 kann mit den beiden zweiten Durchführungen 168 des Griffstücks 174 verbunden sein. Dadurch kann der Hohlraum im Inneren des Griffstücks einen Auffangbehälter zur Aufnahme von überschüssigem Knochenzement 217 bilden, wobei der überschüssige Knochenzementteig aus der Gießform durch die zwei zweiten Durchführungen 168 in den Auffangbehälter strömen kann, wenn das durch den Ventilkörper 178 und den Ventilsitz 163 gebildete Ventil in einer geöffneten Stellung ist, in der die beiden ersten Durchführungen 165 im Ventilsitz 163 für den Knochenzementteig flüssigkeitsdurchlässig mit den beiden zweiten Durchführungen 168 verbunden sind beziehungsweise übereinanderliegend angeordnet sind.

Der Stempel 162 kann in die trogförmige Form 161 hineingeschoben werden. Durch Einstecken des Stempels 162 in die trogförmige Form 161 kann die Gießform nach außen verschlossen werden. Zwischen der trogförmigen Form 161 und dem Stempel 162 kann im ineinandergesteckten Zustand ein Spalt zur Entlüftung des Innenraums der Gießform vorhanden sein (in den Figuren 17 bis 21 nicht zu sehen). Durch den Spalt kann Luft oder Gas aus dem Inneren der geschlossenen Gießform entweichen, wenn ein Knochenzementteig in die Gießform eingefüllt wird.

Der Ventilsitz 163 kann an seiner Innenseite ein Innengewinde 180 aufweisen. Das Außengewinde 179 des Griffstücks 174 passt zu dem Innengewinde 180 des Ventilsitzes 163, so dass der Ventilkörper 178 in den Ventilsitz 163 eingeschraubt werden kann.

Durch Einschrauben des Ventilkörpers 178 in den Ventilsitz 163 bis zum Anschlag können die ersten Durchführungen 165 und die zweiten Durchführungen 168 in Überdeckung zueinander gebracht werden. Das Ventil befindet sich dann im geöffneten Zustand. Ein Knochenzementteig kann in diesem geöffneten Zustand durch die ersten Durchführungen 165 und durch die zweiten Durchführungen 168 aus der Gießform in den Auffangbehälter im Griffstück 174 fließen.

Durch eine Vierteldrehung (um 90°) des Ventilkörpers 178 gegen den Ventilsitz 163, das heißt durch ein Herausschrauben des Ventilkörpers 178 aus dem Ventilsitz 163, können die ersten Durchführungen 165 und die zweiten Durchführungen 168 gegeneinander versetzt werden, so dass die Dichtfläche 167 des Ventilkörpers 178 die ersten Durchführungen 165 des Ventilsitzes 163 abdeckt und die geschlossenen Bereiche der Kopfseite 164 des Ventilsitzes 163 die zweiten Durchführungen 168 des Ventilkörpers 178 abdeckt. Das Ventil befindet sich dann im geschlossenen Zustand. Durch den geringen Hub des Ventilkörpers 178 gegen den Ventilsitz 163 bei einer Vierteldrehung, ist der zwischen dem Ventilkörper 178 und dem Ventilsitz 163 entstehende Spalt so schmal (weniger als 1 mm breit), dass ein normaler und erst recht ein hochviskoser Knochenzementteig nicht durch den Spalt dringen können. Dies gilt insbesondere, weil der Knochenzementteig im Spalt von seiner eigentlichen Flussrichtung um 90° abgelenkt wird.

Das Innengewinde 180 des Ventilsitzes 163 und das Außengewinde 179 des Ventilkörpers 178 können alle den gleichen Drehsinn aufweisen, das heißt, dass alle diese Gewinde Rechtsgewinde oder Linksgewinde sind. Dadurch kann das Ventil durch Einschrauben des Griffstücks 174 in den Ventilsitz 163 geöffnet werden. Gleichzeitig dichtet der Ventilkörper 178 gegen den Ventilsitz 163 ab.

Die Gießform kann eine Bodenplatte 192 zum Formen einer Gleitfläche einer mit der Gießform geformten Kniespacer-Komponente 210 aufweisen. Für den vorliegenden Fall kann dies eine Gleitfläche 212 eines Tibia-Plateaus einer Tibia-Komponente sein (siehe Figur 22). Die Kniespacer-Komponente 210 kann zudem einen Schaft 214 und am Schaft 214 einen Abguss 216 aufweisen. Die Bodenplatte 192 kann den Grund beziehungsweise den Boden der trogförmigen Form 161 bilden. Der Stempel 162 kann in Richtung der Bodenplatte 192 in die trogförmige Form 161 hineingeschoben werden. Dadurch kann ein in die Gießform gefüllter Knochenzementteig gegen die Bodenplatte 192 der trogförmigen Form 161 gedrückt werden. Ferner kann die Gießform und insbesondere der Stempel 162 der Gießform ein Schaft-Formteil 194 zum Ausformen des Schafts 214 der Kniespacer-Komponente 210 aufweisen.

Der Ablauf eines erfindungsgemäßen Verfahrens wird im Folgenden anhand der Figuren 17 bis 22 anhand der dritten erfindungsgemäßen Vorrichtung erläutert. Ein Knochenzementteig wird in die trogförmige Form 161 gefüllt.

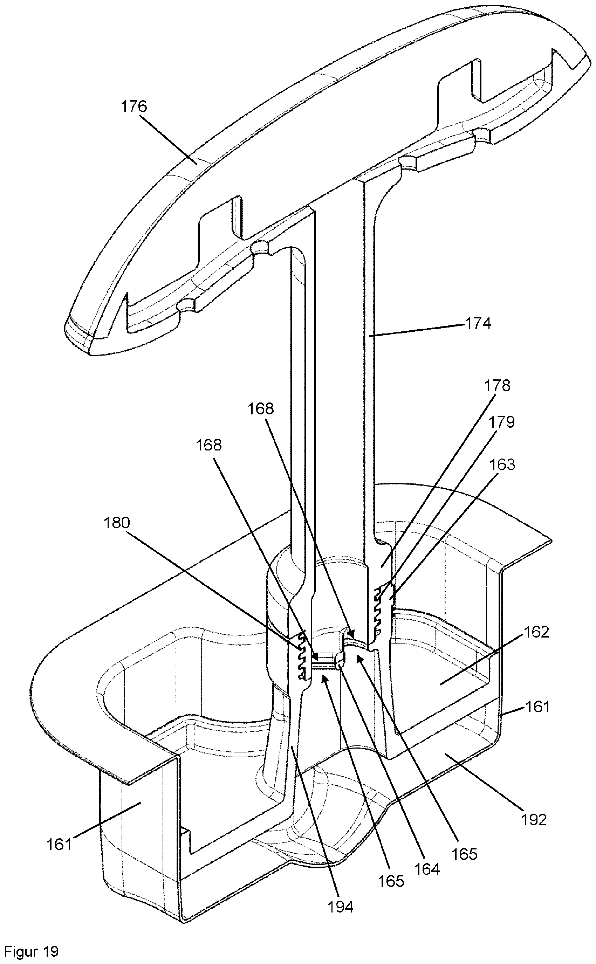

Zur Einstellung der gewünschten Höhe der zu erzeugenden Kniespacer-Komponente 210 kann nun das Griffstück 174 in das Innengewinde 180 des Ventilsitzes 163 geschraubt werden. Dabei bildet der Ventilkörper 178 mit dem Ventilsitz 163 ein Ventil. Diese Situation ist in Figur 18 dargestellt. Durch das Einschrauben des Griffstücks 174 bis zum Anschlag wird das Ventil in die geöffnete Stellung gebracht (siehe Figur 19).

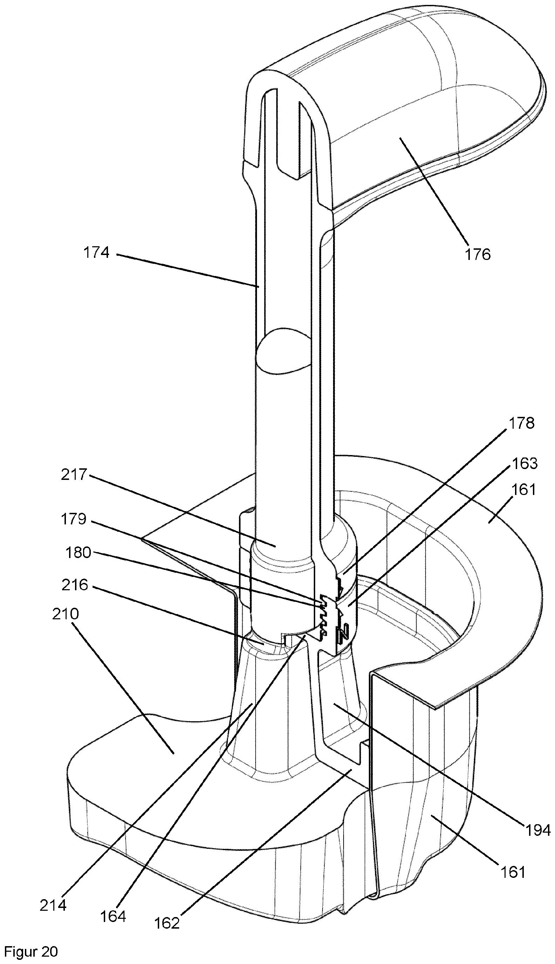

Durch ein Verringern der Höhe des Innenraums der Gießform durch Einschieben des Stempels 162 in die trogförmige Form 161 wird ein Teil des Knochenzementteigs aus der Gießform heraus und durch die beiden ersten Durchführungen 165 und die beiden zweiten Durchführungen 168 hindurch in den Auffangbehälter des Griffstücks 174 gedrückt. Dies ist in Figur 20 dargestellt. Das Griffstück 174 wird nun um eine Vierteldrehung (um 90°) gedreht und damit die zwei zweiten Öffnungen 168 gegen die beiden ersten Öffnungen 165 verdreht und somit das Ventil geschlossen. Dabei wird der Knochenzementteig im Ventil abgeschert oder weitgehend abgeschert. Hierdurch kann der in dem Auffangbehälter des Griffstücks 174 enthaltene überschüssige Knochenzement 217 von der Kniespacer-Komponente 210 beziehungsweise dem die Kniespacer-Komponente 210 bildenden Knochenzementteig in der Gießform entfernt werden.

In diesem Zustand kann der Knochenzementteig in der Gießform ausgehärtet werden. Anschließend wird die so geformte Kniespacer-Komponente 210 aus der Gießform entnommen (siehe Figur 22). Ein durch den Ventilsitz 163 und die ersten Durchgänge 165 verursachter Abguss 216 kann abgeschnitten und entfernt werden. Die Oberfläche der Kniespacer-Komponente 210 kann poliert werden und/oder beschichtet werden, beispielsweise mit Antibiotika.

Anstatt einer Gießform zum Formen einer Tibia-Komponente kann ohne weiteres auch eine Gießform zum Formen einer Femur-Komponente verwendet werden.

Die Figuren 23 bis 29 zeigen ein Ventil für eine erfindungsgemäße Vorrichtung zur Herstellung eines Kniespacers in geöffneter Stellung (Figuren 23 bis 25) und in geschlossener Stellung (Figuren 26 bis 29). Das Ventil entspricht den Ventilen der ersten erfindungsgemäßen Vorrichtung nach den Figuren 1 bis 10, der zweiten erfindungsgemäßen Vorrichtung nach den Figuren 11 bis 15 und der dritten erfindungsgemäßen Vorrichtung nach den Figuren 17 bis 21, ist aber auch mit anderen Gießformen zur Herstellung anderer Kniespacer und Kniespacer-Komponenten einsetzbar.

Das Ventil hat einen Ventilsitz 263, der in einer Gießform (nicht gezeigt) angeordnet werden oder einteilig mit einer Gießform (nicht gezeigt) ausgeführt sein kann. Der Ventilsitz 263 kann aber auch auf andere Weise mit einem Teil der Gießform fest verbunden sein oder auch einteilig mit der Gießform oder einem Teil der Gießform ausgeführt sein.

Der Ventilsitz 263 kann als ein Hohlzylinder ausgeführt sein, der auf einer Kopfseite 264 bis auf zwei erste Durchführungen 265 geschlossen ist. Die beiden ersten Durchführungen 265 können viertelkreisförmig geformt sein und können vorzugsweise um 180° bezüglich der Zylinderachse des Ventilsitzes 263 gegeneinander verdreht angeordnet sein. Im Inneren des Ventilsitzes 263 kann ein gegen den Ventilsitz 263 axial verdrehbarer Ventilkörper 266 angeordnet sein. Der Ventilkörper 266 kann eine in Richtung der Kopfseite 264 des Ventilsitzes 263 ausgerichtete Dichtfläche 267 beziehungsweise Oberfläche aufweisen. Der Ventilkörper 266 kann als abgestufter Hohlzylinder aufgebaut sein, dessen vorderer Teil in den Ventilsitz 263 eingeschraubt oder eingesteckt sein kann.

In der Dichtfläche 267 können zwei zweite Durchführungen 268 angeordnet sein. Die beiden zweiten Durchführungen 268 können analog den ersten Durchführungen 265 viertelkreisförmig geformt sein und können vorzugsweise um 180° bezüglich der Zylinderachse des Ventilkörpers 266 gegeneinander verdreht beziehungsweise versetzt angeordnet sein. Der Ventilsitz 263 und der Ventilkörper 266 bilden zusammen das Ventil einer erfindungsgemäßen Vorrichtung. In den Ventilkörper 266 kann ein Griffstück (nicht gezeigt) oder ein Adapterelement (nicht gezeigt) zum flüssigkeitsdichten Verbinden einer Knochenzementkartusche (nicht gezeigt) eingeschraubt werden. Der Ventilkörper 266 kann an seiner offenen Seite, die von der Dichtfläche 267 abgewandt ist, als Anschluss 271 zum Verbinden des Griffstücks oder eines Adapterelements ausgeformt sein.

Der Ventilsitz 263 kann an seiner Innenseite ein Innengewinde 280 aufweisen. An der der Dichtfläche 267 zugewandten vorderen Hälfte des Ventilkörpers 266 kann der Ventilkörper 266 an seiner Außenseite ein zum Innengewinde 280 des Ventilsitzes 263 passendes Außengewinde 282 aufweisen. Der Ventilkörper 266 kann mit seinem Außengewinde 282 in das Innengewinde 280 des Ventilsitzes 263 eingeschraubt werden.

Durch Einschrauben des Ventilkörpers 266 in den Ventilsitz 263 bis zum Anschlag können die ersten Durchführungen 265 und die zweiten Durchführungen 268 in Überdeckung zueinander gebracht werden. Das Ventil befindet sich dann im geöffneten Zustand. Ein Knochenzementteig kann in diesem geöffneten Zustand (siehe Figuren 23 bis 25) durch die ersten Durchführungen 265 und durch die zweiten Durchführungen 268 fließen. Durch eine Vierteldrehung (um 90°) des Ventilkörpers 266 gegen den Ventilsitz 263, das heißt, durch ein Herausschrauben des Ventilkörpers 266 aus dem Ventilsitz 263, können die ersten Durchführungen 265 und die zweiten Durchführungen 268 gegeneinander versetzt werden, so dass die Dichtfläche 267 des Ventilkörpers 266 die ersten Durchführungen 265 des Ventilsitzes 263 abdeckt und die geschlossenen Bereiche der Kopfseite 264 des Ventilsitzes 263 die zweiten Durchführungen 268 des Ventilkörpers 266 abdeckt. Das Ventil befindet sich dann im geschlossenen Zustand (siehe Figuren 26 bis 29). Durch den geringen Hub des Ventilkörpers 266 gegen den Ventilsitz 263 bei einer Vierteldrehung, ist der zwischen dem Ventilkörper 266 und dem Ventilsitz 263 entstehende Spalt 320 so schmal (weniger als 1 mm breit), dass ein normaler und erst recht ein hochviskoser Knochenzementteig nicht durch den Spalt 320 dringen können (siehe Figur 29). Dies gilt insbesondere, weil der Knochenzementteig im Spalt 320 von seiner eigentlichen Flussrichtung um 90° abgelenkt wird.

In der Rückseite des Ventilkörpers 266 kann im Anschluss 271 ein Innengewinde 284 angeordnet sein. Ein Griffstück (nicht gezeigt) oder ein Adapterelement (nicht gezeigt) kann so in den Anschluss 271 des Ventilkörpers 266 eingeschraubt werden. Das Innengewinde 280 des Ventilsitzes 263, das Außengewinde 282 des Ventilkörpers 266 und das Innengewinde 284 des Ventilkörpers 266 können alle den gleichen Drehsinn aufweisen, das heißt, dass alle diese Gewinde Rechtsgewinde oder Linksgewinde sind. Dadurch kann das Ventil durch Einschrauben eines Griffstücks oder eines Adapterelements in den Anschluss 271 und gleichsinniges Weiterdrehen des Griffstücks oder des Adapterelements geöffnet werden. Gleichzeitig dichtet auch der Ventilkörper 266 gegen den Ventilsitz 263 ab.

Des Weiteren kann ein Handgriff 298 am Ventilkörper 266 angeordnet sein. Der Ventilkörper 266 kann mit dem Handgriff 298 im Ventilsitz 263 gedreht werden. Dadurch kann das Ventil mit Hilfe des Handgriffs 298 manuell von außen vom geöffneten Zustand in den geschlossenen Zustand beziehungsweise vom geschlossenen Zustand in den geöffneten Zustand überführt werden, auch wenn eine Knochenzementkartusche an dem Anschluss 271 angeschlossen ist.

In den Figuren 30 bis 33 sind Abbildungen eines vierten Ausführungsbeispiels einer erfindungsgemäßen Vorrichtung zur Herstellung einer Kniespacer-Komponente 410 in Form einer Tibia-Komponente für einen Kniespacer und Teile der Vorrichtung in verschiedenen Ansichten gezeigt. Die Figur 33 zeigt unter anderem die Kniespacer-Komponente 410, die mit einer solchen vierten erfindungsgemäßen Vorrichtung hergestellt wurde, als Resultat eines erfindungsgemäßen Verfahrens, dessen Verfahrensschritte chronologisch in den Figuren 30 bis 33 gezeigt sind.

Die vierte erfindungsgemäße Vorrichtung ist zur Herstellung einer Tibia-Komponente eines Kniespacers geeignet und vorgesehen. Die Vorrichtung umfasst eine Gießform, die sich aus zwei Teilen zusammensetzt. Die Gießform kann eine trogförmige Form 361 und einen Stempel 362 aufweisen. Der Stempel 362 kann in die trogförmige Form 361 eingesetzt werden und kann in die trogförmigen Form 361 eingeschoben und vorzugsweise auch wieder herausgezogen werden. Die trogförmige Form 361 kann kostengünstig aus Kunststofffolie gefertigt werden. Die Kunststofffolie kann mehrere Schichten aufweisen. An einer Seite des Stempels 362 kann eine Öffnung zum Durchfließen von Knochenzementteig (nicht gezeigt) ausgeformt sein, die durch eine zylindrische Wandung des Stempels 362 begrenzt sein kann. In dieser Öffnung kann ein Ventilsitz 363 angeordnet sein. Der Ventilsitz 363 kann mit dem Stempel 362 der Gießform fest verbunden sein oder sogar einteilig ausgeführt sein, so wie das in den Figuren 30 bis 33 dargestellt ist.

Der Ventilsitz 363 kann als ein Hohlzylinder ausgeführt sein, der auf einer in Richtung der Öffnung im Stempel 362 ausgerichteten Kopfseite 364 bis auf zwei erste Durchführungen 365 geschlossen ist. Die beiden ersten Durchführungen 365 können viertelkreisförmig geformt sein und können vorzugsweise um 180° bezüglich der Zylinderachse des Ventilsitzes 363 gegeneinander verdreht beziehungsweise versetzt angeordnet sein.

Im Inneren des Ventilsitzes 363 kann ein gegen den Ventilsitz 363 axial verdrehbarer Ventilkörper 178 angeordnet sein oder werden. Der Ventilkörper 178 kann eine in Richtung der Kopfseite 364 des Ventilsitzes 363 ausgerichtete Dichtfläche 167 beziehungsweise Oberfläche aufweisen, die zum Verschließen der zwei ersten Durchführungen 365 geeignet sein kann.

In dem Ventilkörper 178 können zwei zweite Durchführungen 168 angeordnet sein. Die beiden zweiten Durchführungen 168 können analog den ersten Durchführungen 365 viertelkreisförmig geformt sein und können vorzugsweise um 180° bezüglich der Zylinderachse des Ventilkörpers 178 gegeneinander verdreht angeordnet sein. Der Ventilsitz 363 und der Ventilkörper 178 bilden zusammen ein Ventil der Vorrichtung.

Die Vorrichtung kann ein Griffstück 174 aufweisen, das an einem Ende einen Griff 176 formt (siehe Figuren 30 bis 33). Das Griffstück 174 des vierten Ausführungsbeispiels nach den Figuren 30 bis 33 ist gleich dem Griffstück 174 des dritten Ausführungsbeispiels nach den Figuren 17 bis 21 ausgeführt. Daher wurden die gleichen Bezugszeichen verwendet. Am gegenüberliegenden Ende des Griffstücks 174 kann der Ventilkörper 178 ein Außengewinde 179 aufweisen. Der Ventilkörper 178 kann mittels des Griffstücks 174 in dem Ventilsitz 363 und gegen den Ventilsitz 363 axial verdrehbar angeordnet sein beziehungsweise werden (siehe Figuren 31 und 32). Der Ventilkörper 178 kann in den Ventilsitz 363 eingeschraubt oder eingesteckt sein.

Das Griffstück 174 kann im inneren Hohl ausgeführt sein. Der Hohlraum im Inneren des Griffstücks 174 kann mit den beiden zweiten Durchführungen 168 des Griffstücks 174 verbunden sein. Dadurch kann der Hohlraum im Inneren des Griffstücks einen Auffangbehälter zur Aufnahme von überschüssigem Knochenzement bilden, wobei der überschüssige Knochenzementteig aus der Gießform durch die zwei zweiten Durchführungen 168 in den Auffangbehälter strömen kann, wenn das durch den Ventilkörper 178 und den Ventilsitz 363 gebildete Ventil in einer geöffneten Stellung ist, in der die beiden ersten Durchführungen 365 im Ventilsitz 363 für den Knochenzementteig flüssigkeitsdurchlässig mit den beiden zweiten Durchführungen 168 verbunden sind beziehungsweise übereinanderliegend angeordnet sind.

Der Stempel 362 kann in die trogförmige Form 361 hineingeschoben werden. Durch Einstecken des Stempels 362 in die trogförmige Form 361 kann die Gießform nach außen verschlossen werden. Zwischen der trogförmigen Form 361 und dem Stempel 362 kann im ineinandergesteckten Zustand ein Spalt zur Entlüftung des Innenraums der Gießform vorhanden sein (in den Figuren 30 bis 33 nicht zu sehen). Durch den Spalt kann Luft oder Gas aus dem Inneren der geschlossenen Gießform entweichen, wenn ein Knochenzementteig in die Gießform eingefüllt wird.

Der Ventilsitz 363 kann an seiner Innenseite ein Innengewinde 380 aufweisen. Das Außengewinde 179 des Griffstücks 174 passt zu dem Innengewinde 380 des Ventilsitzes 363, so dass der Ventilkörper 178 in den Ventilsitz 363 eingeschraubt werden kann.

Durch Einschrauben des Ventilkörpers 178 in den Ventilsitz 363 bis zum Anschlag können die ersten Durchführungen 365 und die zweiten Durchführungen 168 in Überdeckung zueinander gebracht werden. Das Ventil befindet sich dann im geöffneten Zustand. Ein Knochenzementteig kann in diesem geöffneten Zustand durch die ersten Durchführungen 365 und durch die zweiten Durchführungen 168 aus der Gießform in den Auffangbehälter im Griffstück 174 fließen.

Durch eine Vierteldrehung (um 90°) des Ventilkörpers 178 gegen den Ventilsitz 363, das heißt durch ein Herausschrauben des Ventilkörpers 178 aus dem Ventilsitz 363, können die ersten Durchführungen 365 und die zweiten Durchführungen 168 gegeneinander versetzt werden, so dass die Dichtfläche 167 des Ventilkörpers 178 die ersten Durchführungen 365 des Ventilsitzes 363 abdeckt und die geschlossenen Bereiche der Kopfseite 364 des Ventilsitzes 363 die zweiten Durchführungen 168 des Ventilkörpers 178 abdeckt. Das Ventil befindet sich dann im geschlossenen Zustand. Durch den geringen Hub des Ventilkörpers 178 gegen den Ventilsitz 363 bei einer Vierteldrehung, ist der zwischen dem Ventilkörper 178 und dem Ventilsitz 363 entstehende Spalt so schmal (weniger als 1 mm breit), dass ein normaler und erst recht ein hochviskoser Knochenzementteig nicht durch den Spalt dringen können. Dies gilt insbesondere, weil der Knochenzementteig im Spalt von seiner eigentlichen Flussrichtung um 90° abgelenkt wird.

Das Innengewinde 380 des Ventilsitzes 363 und das Außengewinde 179 des Ventilkörpers 178 können alle den gleichen Drehsinn aufweisen, das heißt, dass alle diese Gewinde Rechtsgewinde oder Linksgewinde sind. Dadurch kann das Ventil durch Einschrauben des Griffstücks 174 in den Ventilsitz 363 geöffnet werden. Gleichzeitig dichtet der Ventilkörper 178 gegen den Ventilsitz 363 ab.

Die Gießform kann eine Bodenplatte 392 zum Formen einer Gleitfläche einer mit der Gießform geformten Kniespacer-Komponente 410 aufweisen. Für den vorliegenden Fall kann dies eine Gleitfläche eines Tibia-Plateaus einer Tibia-Komponente sein (siehe Figur 33). Die Kniespacer-Komponente 410 kann einen Abguss 416 aufweisen. die Kniespacer-Komponente 410 nach dem vierten Ausführungsbeispiel weist im Gegensatz zu den anderen Ausführungsbeispielen 1 bis 3 keinen Schaft auf. Die Bodenplatte 392 kann den Grund beziehungsweise den Boden der trogförmigen Form 361 bilden. Der Stempel 362 kann in Richtung der Bodenplatte 392 in die trogförmige Form 361 hineingeschoben werden. Dadurch kann ein in die Gießform gefüllter Knochenzementteig gegen die Bodenplatte 392 der trogförmigen Form 361 gedrückt werden.

Der Ablauf eines erfindungsgemäßen Verfahrens läuft analog den beispielhaft erläuterten Verfahren der Ausführungsbeispiele zwei und drei ab.

Der Knochenzementteig kann dabei in einem Mischbecher 70 (siehe Figur 30) gemischt werden. Die Vorrichtung kann hierzu einen Monomerflüssigkeitsbehälter 72 enthaltend Monomerflüssigkeit und einen Knochenzementpulverbehälter 73 enthaltend Knochenzementpulver aufweisen. Der Knochenzementteig kann in dem Mischbecher 70 aus dem Knochenzementpulver und der Monomerflüssigkeit gemischt werden. Zum Mischen des Knochenzementteigs in dem Mischbecher 70 kann die Vorrichtung einen Spatel 75 oder ein anderes Mischwerkzeug aufweisen. Der Monomerflüssigkeitsbehälter 72 kann eine Glasampulle sein. Der Mischbecher 70 kann eine Tülle 77 zum Ausgießen des Knochenzementteigs aufweisen. Der gemischte Knochenzementteig kann aus dem Mischbecher 70 über die Tülle 77 des Mischbechers 70 in die trogförmige Form 361 gefüllt werden.

Alternativ kann auch eine Knochenzementkartusche zum Herstellen und Einfüllen eines Knochenzementteigs in die trogförmige Form 361 oder in die Gießform analog zu dem ersten Ausführungsbeispiel nach den Figuren 1 bis 10 angewendet werden.

Anstatt einer Gießform zum Formen einer Tibia-Komponente kann ohne weiteres auch eine Gießform zum Formen einer Femur-Komponente verwendet werden.

### Bezugszeichenliste

- 1, 61, 161, 361: Trogförmige Form / Gießform
- 2, 62, 162, 362: Stempel / Gießform
- 3, 63, 163, 263, 363: Ventilsitz
- 4, 64, 164, 264, 364: Kopfseite
- 5, 65, 165, 265, 365: Durchführung
- 6, 266: Ventilkörper
- 7, 67, 167, 267: Dichtfläche
- 8, 68, 168, 268: Durchführung
- 9: Adapterelement
- 10: Knochenzementkartusche
- 11, 271: Anschluss
- 12: Austragsöffnung
- 13: Austragsrohr
- 14, 74, 174: Griffstück
- 15: Mischer
- 16, 76, 176: Griff
- 17: Dichtung
- 18, 78, 178: Ventilkörper
- 19, 79, 179: Außengewinde
- 20, 80, 180, 280, 380: Innengewinde
- 21: Durchführung
- 22, 182, 282: Außengewinde
- 24, 184, 284: Innengewinde
- 26: Außengewinde
- 28: Rastmittel
- 30: Gegenrastung
- 32, 92, 192, 392: Bodenplatte zum Formen einer Gleitfläche
- 34, 94, 194: Schaft-Formteil
- 38, 298: Handgriff
- 44: Vakuumanschluss
- 46: Kolben
- 48: Dichtung
- 50, 51: Knochenzementteig
- 52: Porenscheibe
- 54: Dichtung
- 70: Mischbecher
- 72: Monomerflüssigkeitsbehälter
- 73: Knochenzementpulverbehälter
- 75: Spatel
- 77: Tülle
- 82: Verschlussstift
- 110: Kniespacer-Komponente / Femur-Komponente
- 112, 212: Gleitfläche
- 114,214: Schaft
- 116, 216, 416: Abguss
- 117, 217: Überschüssiger Knochenzement
- 210, 410: Kniespacer-Komponente / Tibia-Komponente
- 316: Vorsprung
- 320: Spalt

## Patentansprüche

1. Vorrichtung zum Herstellen einer Kniespacer-Komponente (110, 210, 410) durch Aushärten von Knochenzementteig (50, 51), wobei die Kniespacer-Komponente (110, 210, 410) dazu vorgesehen ist, im medizinischen Bereich einen Teil eines Kniegelenks umfassend eine artikulierende Oberfläche des Kniegelenks temporär zu ersetzen, die Vorrichtung aufweisend
eine Gießform zum Formen der Kniespacer-Komponente (110, 210, 410) aus Knochenzementteig (50, 51);
einen Ventilsitz (3, 63, 163, 263, 363), der mit der Gießform verbunden ist, wobei der Ventilsitz (3, 63, 163, 263, 363) eine bereichsweise geschlossene Kopfseite (4, 64, 164, 264, 364) mit mindestens einer ersten Durchführung (5, 65, 165, 265, 365) aufweist, wobei die mindestens eine erste Durchführung (5, 65, 165, 265, 365) in die Gießform hinein mündet;
einen Ventilkörper (6, 18, 78, 178, 266, 366), der drehbar gegen den Ventilsitz (3, 63, 163, 263, 363) gelagert ist und der eine Dichtfläche (7, 67, 167, 267) aufweist, wobei die Dichtfläche (7, 67, 167, 267) in Richtung der bereichsweise geschlossenen Kopfseite (4, 64, 164, 264, 364) des Ventilsitzes (3, 63, 163, 263, 363) ausgerichtet ist, wobei in der Dichtfläche (7, 67, 167, 267) mindestens eine zweite Durchführung (8, 21, 68, 168, 268) angeordnet ist;
wobei der Ventilsitz (3, 63, 163, 263, 363) und der Ventilkörper (6, 18, 78, 178, 266, 366) zusammen ein Ventil bilden, wobei das Ventil durch Drehen des Ventilkörpers (6, 18, 78, 178, 266, 366) gegen den Ventilsitz (3, 63, 163, 263, 363) reversibel in eine geöffnete Stellung und reversibel in eine geschlossene Stellung überführbar ist, wobei in der geöffneten Stellung des Ventils die mindestens eine erste Durchführung (5, 65, 165, 265, 365) des Ventilsitzes (3, 63, 163, 263, 363) und die mindestens eine zweite Durchführung (8, 21, 68, 168, 268) des Ventilkörpers (6, 18, 78, 178, 266, 366) zumindest bereichsweise übereinanderliegen und eine für Knochenzementteig (50, 51) durchlässige Verbindung durch das Ventil bereitstellen, wobei in der geschlossenen Stellung des Ventils die mindestens eine erste Durchführung (5, 65, 165, 265, 365) des Ventilsitzes (3, 63, 163, 263, 363) durch die Dichtfläche (7, 67, 167, 267) des Ventilkörpers (6, 18, 78, 178, 266, 366) abgedeckt ist, wobei die Gießform in der geschlossenen Stellung des Ventils für Knochenzementteig (50, 51) flüssigkeitsdicht verschlossen ist.

2. Vorrichtung nach Anspruch 1, wobei
der Ventilsitz (3, 63, 163, 263, 363) nicht verdrehbar gegen die Gießform mit der Gießform verbunden ist, bevorzugt der Ventilsitz (3, 63, 163, 263, 363) fest und/oder starr mit der Gießform verbunden ist oder einteilig mit der Gießform ausgeführt ist, und/oder
das Ventil manuell bedienbar ist, bevorzugt manuell von außerhalb der Vorrichtung manuell bedienbar ist, wobei besonders bevorzugt der Ventilkörper (6, 18, 78, 178, 266, 366) manuell gegen den Ventilsitz (3, 63, 163, 263, 363) drehbar ist und durch die Drehung das Ventil von der geschlossenen Stellung in die geöffnete Stellung und von der geöffneten Stellung in die geschlossene Stellung überführbar ist.

3. Vorrichtung nach einem der vorangehenden Ansprüche, wobei
die mindestens eine erste Durchführung (5, 65, 165, 265, 365) des Ventilsitzes (3, 63, 163, 263, 363) in der geschlossenen Stellung des Ventils mit der Dichtfläche (7, 67, 167, 267) des Ventilkörpers (6, 18, 78, 178, 266, 366) abgedeckt ist, wobei bevorzugt die bereichsweise geschlossene Kopfseite (4, 64, 164, 264, 364) des Ventilsitzes (3, 63, 163, 263, 363) und die Dichtfläche (7, 67, 167, 267) des Ventilkörpers (6, 18, 78, 178, 266, 366) maximal 2 mm voneinander beabstandet sind, besonders bevorzugt maximal 1 mm voneinander beabstandet sind, ganz besonders bevorzugt maximal 0,5 mm voneinander beabstandet sind, und/oder
der Ventilkörper (6, 18, 78, 178, 266, 366) um eine Drehachse gegen den Ventilsitz (3, 63, 163, 263, 363) drehbar gelagert ist, wobei die Drehachse senkrecht zur Dichtfläche (7, 67, 167, 267) des Ventilkörpers (6, 18, 78, 178, 266, 366) verläuft oder wobei die Drehachse entlang einer Rotationssymmetrieachse der Dichtfläche (7, 67, 167, 267) des Ventilkörpers (6, 18, 78, 178, 266, 366) verläuft.

4. Vorrichtung nach einem der vorangehenden Ansprüche, wobei
der Ventilkörper (6, 18, 78, 178, 266, 366) einen Anschluss (11, 271) zum flüssigkeitsdichten Verbinden einer Knochenzementkartusche (10) oder eines Griffstücks der Vorrichtung aufweist oder mit einem solchen Anschluss (11, 271) fest verbunden ist, wobei vorzugsweise
die Vorrichtung ein Adapterelement (9) aufweist, das mit einer Knochenzementkartusche (10) verbunden ist oder verbindbar ist, wobei das Adapterelement (9) lösbar und formschlüssig mit dem Anschluss (11, 271) verbunden oder verbindbar ist, so dass ein Innenraum einer Knochenzementkartusche (10) über das Adapterelement (9) für Knochenzementteig (50, 51) durchlässig mit der mindestens einen zweiten Durchführung (8, 21, 68, 168, 268) im Ventilkörper (6, 18, 78, 178, 266, 366) verbunden oder verbindbar ist, und/oder
der Anschluss (11, 271) zum flüssigkeitsdichten Verbinden einer Knochenzementkartusche (10) oder eines Griffstücks (14, 74, 174) ein Innengewinde (24, 84, 184) im Ventilkörper (6, 18, 78, 178, 266, 366) oder ein Außengewinde am Ventilkörper (6, 18, 78, 178, 266, 366) umfasst, wobei vorzugsweise ein Adapterelement (9) der Knochenzementkartusche (10) oder an der Knochenzementkartusche (10) ein zu dem Innengewinde (24, 84, 184) oder zu dem Außengewinde passendes Gegengewinde (26) aufweist oder das Griffstück (14, 74, 174) der Vorrichtung ein zu dem Innengewinde (24, 84, 184) oder zu dem Außengewinde passendes Gegengewinde (19, 79, 179) aufweist.

5. Vorrichtung nach einem der vorangehenden Ansprüche, wobei
die Vorrichtung eine Knochenzementkartusche (10) zum Mischen von Knochenzement-Ausgangskomponenten und zum Austragen von gemischtem Knochenzementteig (50, 51) aus der Knochenzementkartusche (10) heraus aufweist, bevorzugt eine Knochenzementkartusche (10) zum Mischen von Polymethylmethacrylat-Knochenzement-Ausgangskomponenten und zum Austragen von gemischtem Polymethylmethacrylat-Knochenzementteig (50, 51) aus der Knochenzementkartusche (10) heraus aufweist, wobei besonders bevorzugt die Knochenzementkartusche (10) die Knochenzement-Ausgangskomponenten zur Herstellung des Knochenzementteigs (50, 51) in voneinander getrennten Bereichen beinhaltet, und/oder
die Vorrichtung ein Griffstück (14, 74, 174) aufweist, das mit dem Ventilkörper (6, 18, 78, 178, 266, 366) verbindbar ist und mit dem der Ventilkörper (6, 18, 78, 178, 266, 366) gegen den Ventilsitz (3, 63, 163, 263, 363) drehbar ist, wobei bevorzugt das Griffstück (14, 74, 174) einen Hohlraum zur Aufnahme von Knochenzementteig (50, 51) aufweist, der mit der mindestens einen zweiten Durchführung (8, 21, 68, 168, 268) flüssigkeitsdurchlässig verbunden ist, und/oder
die Summe aller freien Öffnungen der mindestens einen ersten Durchführung (5, 65, 165, 265, 365) in der geschlossenen Kopfseite (4, 64, 164, 264, 364) höchstens so groß ist wie die geschlossene Oberfläche der Kopfseite (4, 64, 164, 264, 364) und dass die Summe aller freien Öffnungen der mindestens einen zweiten Durchführung (8, 21, 68, 168, 268) in der Dichtfläche (7, 67, 167, 267) höchstens so groß ist wie die geschlossene Oberfläche der Dichtfläche (7, 67, 167, 267), und
der Ventilsitz (3, 63, 163, 263, 363) ein Innengewinde (20, 80, 180) an der Innenseite aufweist und der Ventilkörper (6, 18, 78, 178, 266, 366) ein passendes Außengewinde (22, 82, 182) an der Außenseite aufweist, so dass der Ventilkörper (6, 18, 78, 178, 266, 366) in den Ventilsitz (3, 63, 163, 263, 363) schraubbar ist.

6. Vorrichtung nach einem der vorangehenden Ansprüche, wobei
die Gießform eine trogförmige Form (1, 61, 161, 361) mit einem Hohlraum und einen Stempel (2, 62, 162, 362) aufweist,
wobei der Stempel (2, 62, 162, 362) in den Hohlraum der trogförmigen Form (1, 61, 161, 361) einsetzbar oder eingesetzt ist und der Stempel (2, 62, 162, 362) in dem Hohlraum axial verschiebbar ist, und
wobei vorzugsweise die trogförmige Form (1, 61, 161, 361) einen Hohlraumboden (32, 92, 192, 392) aufweist, wobei der Hohlraumboden (32, 92, 192, 392) die Kontur einer oder mehrerer artikulierender Gleitflächen (112, 212) der Kniespacer-Komponente (110, 210, 410) bildet, und
der Ventilsitz (3, 63, 163, 263, 363) in dem Stempel (2, 62, 162, 362) angeordnet ist, vorzugsweise am Ende eines einen Schaft der Kniespacer-Komponente (110, 210, 410) formenden Teils des Stempels (2, 62, 162, 362) angeordnet ist.

7. Vorrichtung nach einem der vorangehenden Ansprüche, wobei
die mindestens eine erste Durchführung (5, 65, 165, 265, 365) in der bereichsweise geschlossenen Kopfseite (4, 64, 164, 264, 364) die gleiche Größe und Gestalt hat wie die mindestens eine zweite Durchführung (8, 21, 68, 168, 268) in der Dichtfläche (7, 67, 167, 267) und/oder
die mindestens eine erste Durchführung (5, 65, 165, 265, 365) in der bereichsweise geschlossenen Kopfseite (4, 64, 164, 264, 364) zwei erste Durchführungen (5, 65, 165, 265, 365) sind und die mindestens eine zweite Durchführung (8, 21, 68, 168, 268) in der Dichtfläche (7, 67, 167, 267) zwei zweite Durchführungen (8, 21, 68, 168, 268) sind, wobei bevorzugt die zwei ersten Durchführungen (5, 65, 165, 265, 365) in gegenüberliegend angeordneten Kreissegmentvierteln bezüglich der Drehachse des Ventilkörpers (6, 18, 78, 178, 266, 366) im Ventilsitz (3, 63, 163, 263, 363) angeordnet sind und die zwei zweiten Durchführungen (8, 21, 68, 168, 268) in gegenüberliegend angeordneten Kreissegmentvierteln bezüglich der Drehachse des Ventilkörpers (6, 18, 78, 178, 266, 366) in der Dichtfläche (7, 67, 167, 267) angeordnet sind.

8. Vorrichtung nach einem der vorangehenden Ansprüche, wobei an der Dichtfläche (7, 67, 167, 267) des Ventilkörpers (6, 18, 78, 178, 266, 366) ein Bund angeordnet ist, der sich auf einem Rand des Ventilsitzes (3, 63, 163, 263, 363) abstützt oder an der bereichsweise geschlossenen Kopfseite (4, 64, 164, 264, 364) des Ventilsitzes (3, 63, 163, 263, 363) ein Bund angeordnet ist, der sich auf einem Rand des Ventilkörpers (6, 18, 78, 178, 266, 366) abstützt, und/oder an dem Ventilkörper (6, 18, 78, 178, 266, 366) ein Griff (16, 38, 76, 176, 298) befestigt oder befestigbar ist, der zumindest eine radiale Erstreckung bezüglich der Drehachse des Ventilkörpers (6, 18, 78, 178, 266, 366) aufweist,
wobei vorzugsweise der Griff (16, 38, 76, 176, 298) an einer der Dichtfläche gegenüberliegenden Seite des Ventilkörpers befestigt oder befestigbar ist, wobei besonders bevorzugt sich der Ventilkörper (6, 18, 78, 178, 266, 366) maximal um 90° gegen den Ventilsitz (3, 63, 163, 263, 363) drehen lässt, und/oder der Ventilkörper (6, 18, 78, 178, 266, 366) und der Ventilsitz (3, 63, 163, 263, 363) aus Kunststoff gefertigt sind, insbesondere aus thermoplastischem Kunststoff gefertigt sind, wobei bevorzugt der Ventilsitz (3, 63, 163, 263, 363) mit der Gießform einteilig ausgeführt ist.

9. Vorrichtung nach einem der vorangehenden Ansprüche, wobei
ein Rastelement an der Dichtfläche des Ventilkörpers (6, 18, 78, 178, 266, 366) angeordnet ist und an der Kopfseite (4, 64, 164, 264, 364) des Ventilsitzes (3, 63, 163, 263, 363) ein Gegenrastelement angeordnet ist, wobei das Rastelement mit dem Gegenrastelement in Eingriff bringbar ist, wobei das Rastelement am Ventilkörper (6, 18, 78, 178, 266, 366) so positioniert ist, dass ein Drehen oder ein Herausdrehen des Ventilkörpers (6, 18, 78, 178, 266, 366) aus dem Ventilsitz (3, 63, 163, 263, 363) auf einen Drehwinkel von maximal 90° begrenzt ist, wenn der Ventilkörper (6, 18, 78, 178, 266, 366) maximal in den Ventilsitz (3, 63, 163, 263, 363) eingesteckt oder eingeschraubt ist, und/oder
die Gießform zweitteilig oder mehrteilig ist, wobei zwischen zumindest zwei der Teile der zusammengesetzten Gießform ein Spalt vorhanden ist, durch den Luft oder Gas aus dem Inneren der Gießform entweichen kann, und/oder
die Vorrichtung einen Auffangbehälter aufweist, wobei das Ventil auf der von der Gießform abgewandten Seite mit dem Auffangbehälter zur Aufnahme von durch das Ventil in der geöffneten Stellung aus der Gießform austretenden überschüssigem Knochenzementteig (50, 51) verbunden oder verbindbar ist oder das Ventil einteilig mit dem Auffangbehälter ausgeführt ist.

10. Verfahren zur Herstellung einer Kniespacer-Komponente (110, 210, 410) zum temporären Ersetzen eines Teils eines Kniegelenks umfassend eine artikulierende Oberfläche des Kniegelenks, wobei das Verfahren mit einer Vorrichtung nach einem der Ansprüche 1 bis 9 durchgeführt wird, das Verfahren aufweisend die folgenden chronologischen Schritte:
A) Flüssigkeitsdichtes Verbinden einer Knochenzementkartusche (10) mit der Vorrichtung;
B) Einpressen von Knochenzementteig (50, 51) aus der Knochenzementkartusche (10) durch das Ventil in der geöffneten Stellung hindurch in die Gießform;
C) Drehen des Ventilkörpers (6, 18, 78, 178, 266, 366) gegen den Ventilsitz (3, 63, 163, 263, 363) und dadurch Überführen des Ventils in die geschlossene Stellung und Abscheren des Knochenzementteigs (50, 51) an der mindestens einen ersten Durchführung (5, 65, 165, 265, 365) in der bereichsweise geschlossenen Kopfseite (4, 64, 164, 264, 364) des Ventilsitzes (3, 63, 163, 263, 363) durch die Drehung des Ventilkörpers (6, 18, 78, 178, 266, 366) gegen den Ventilsitz (3, 63, 163, 263, 363);
D) Lösen der Knochenzementkartusche (10) von der Gießform;
E) Aushärten des Knochenzementteigs (50, 51) in der Gießform; und
F) Entnehmen der so geformten und ausgehärteten Kniespacer-Komponente (110, 210, 410) aus der Gießform.

11. Verfahren nach Anspruch 10, wobei nach Schritt D) und vor Schritt E) die folgenden Zwischenschritte erfolgen:
D2) Flüssigkeitsdichtes Verbinden einer neuen Knochenzementkartusche (10) mit der Vorrichtung, wobei in der neuen Knochenzementkartusche (10) Knochenzementteig (50, 51) oder Ausgangskomponenten zur Herstellung des Knochenzementteigs (50, 51) enthalten ist oder sind;
D3) Drehen des Ventilkörpers (6, 18, 78, 178, 266, 366) gegen den Ventilsitz (3, 63, 163, 263, 363) und dadurch Überführen des Ventils in die geöffnete Stellung;
D4) Einpressen des Knochenzementteigs (50, 51) aus der neuen Knochenzementkartusche (10) durch das Ventil in der geöffneten Stellung hindurch in die Gießform;
D5) Drehen des Ventilkörpers (6, 18, 78, 178, 266, 366) gegen den Ventilsitz (3, 63, 163, 263, 363) und dadurch Überführen des Ventils in die geschlossene Stellung und Abscheren des Knochenzementteigs (50, 51) an der mindestens einen ersten Durchführung (5, 65, 165, 265, 365) in der bereichsweise geschlossenen Kopfseite (4, 64, 164, 264, 364) des Ventilsitzes (3, 63, 163, 263, 363) durch die Drehung des Ventilkörpers (6, 18, 78, 178, 266, 366) gegen den Ventilsitz (3, 63, 163, 263, 363); und
D6) Lösen der neuen Knochenzementkartusche (10) von der Gießform;
wobei vorzugsweise die Schritte D2) bis D6) einmal oder mehrfach mit jeweils neuen Knochenzementkartuschen (10), die Knochenzementteig (50, 51) oder dessen Ausgangskomponenten enthalten, wiederholt werden, bis die Gießform vollständig oder nach Bedarf mit Knochenzementteig (50, 51) gefüllt ist.

12. Verfahren nach einem der Ansprüche 10 oder 11, wobei
zum flüssigkeitsdichten Verbinden der Knochenzementkartusche (10) und/oder der neuen Knochenzementkartusche (10) diese mit einem Anschluss (11, 271) an dem Ventilkörper (6, 18, 78, 178, 266, 366) der Vorrichtung verbunden wird, wobei die Knochenzementkartusche (10) oder die neue Knochenzementkartusche (10) in den Anschluss (11, 271) hineingedreht oder eingeschraubt wird und zum Lösen der Knochenzementkartusche (10) und/oder der neuen Knochenzementkartusche (10) von dem Anschluss (11, 271) die Knochenzementkartusche (10) oder die neue Knochenzementkartusche (10) aus dem Anschluss (11, 271) herausgedreht oder herausgeschraubt wird, und/oder
das Einpressen des Knochenzementteigs (50, 51) aus der Knochenzementkartusche (10) oder der neuen Knochenzementkartusche (10) durch Eindrücken eines Kolbens (46) in einen Innenraum der Knochenzementkartusche (10) erfolgt, und/oder die Gießform eine trogförmige Form (1, 61, 161, 361) und einen Stempel (2, 62, 162, 362) aufweist, wobei vor Schritt E) der Stempel (2, 62, 162, 362) in die trogförmige Form (1, 61, 161, 361) gepresst wird und dadurch die Kniespacer-Komponente (110, 210, 410) aus dem Knochenzementteig (50, 51) in der Gießform geformt wird, wobei bevorzugt beim Einpressen des Stempels (2, 62, 162, 362) in die trogförmige Form (1, 61, 161, 361) ein Teil des Knochenzementteigs (50, 51) durch das Ventil in der geöffneten Stellung aus der Gießform herausgedrückt wird und wobei besonders bevorzugt der Teil des Knochenzementteigs (50, 51) in einen Auffangbehälter gedrückt wird, der mit dem zumindest einen zweiten Durchgang des Ventils verbunden ist.

13. Verfahren zur Herstellung einer Kniespacer-Komponente (110, 210, 410) zum temporären Ersetzen eines Teils eines Kniegelenks umfassend eine artikulierende Oberfläche des Kniegelenks, wobei das Verfahren mit einer Vorrichtung nach einem der Ansprüche 1 bis 9 durchgeführt wird, das Verfahren aufweisend die folgenden chronologischen Schritte:
A) Herstellen eines Knochenzementteigs (50, 51);
B) Einfüllen eines gemischten Knochenzementteigs (50, 51) in die Gießform;
C) Komprimieren der Gießform und dadurch Herausdrücken eines Teils des Knochenzementteigs (50, 51) aus der Gießform durch das Ventil in der geöffneten Stellung hindurch;
D) Drehen des Ventilkörpers (6, 18, 78, 178, 266, 366) gegen den Ventilsitz (3, 63, 163, 263, 363) und dadurch Überführen des Ventils in die geschlossene Stellung und Abscheren des Knochenzementteigs (50, 51) an der mindestens einen ersten Durchführung (5, 65, 165, 265, 365) in der bereichsweise geschlossenen Kopfseite (4, 64, 164, 264, 364) des Ventilsitzes (3, 63, 163, 263, 363) durch die Drehung des Ventilkörpers (6, 18, 78, 178, 266, 366) gegen den Ventilsitz (3, 63, 163, 263, 363);
E) Aushärten des Knochenzementteigs (50, 51) in der Gießform; und
F) Entnehmen der so geformten und ausgehärteten Kniespacer-Komponente (110, 210, 410) aus der Gießform.

14. Verfahren nach Anspruch 13, wobei zwischen den Schritten B) und C) ein Schritt B1) erfolgt:
Verschließen der Gießform bis auf das Ventil, und/oder
die Gießform eine trogförmige Form (1, 61, 161, 361) und einen Stempel (2, 62, 162, 362) aufweist, wobei in Schritt C) der Stempel (2, 62, 162, 362) in die trogförmige Form (1, 61, 161, 361) gepresst wird und dadurch die Kniespacer-Komponente (110, 210, 410) aus dem Knochenzementteig (50, 51) in der Gießform geformt wird, wobei vorzugsweise beim Einpressen des Stempels (2, 62, 162, 362) in die trogförmige Form (1, 61, 161, 361) ein Teil des Knochenzementteigs (50, 51) durch das Ventil in der geöffneten Stellung aus der Gießform herausgedrückt wird.

15. Verfahren nach einem der Ansprüche 10 bis 14, wobei
vor Schritt B) der Knochenzementteig (50, 51) in der Knochenzementkartusche (10) aus einer Monomerflüssigkeit und aus einem Zementpulver gemischt wird, wobei bevorzugt gegebenenfalls vor Schritt D3), vorzugsweise vor Schritt D2), der Knochenzementteig (50, 51) in der neuen Knochenzementkartusche (10) aus einer Monomerflüssigkeit und aus einem Zementpulver gemischt wird, und/oder
das Drehen des Ventilkörpers (6, 18, 78, 178, 266, 366) gegen den Ventilsitz (3, 63, 163, 263, 363) durch ein Schrauben des Ventilkörpers (6, 18, 78, 178, 266, 366) in dem Ventilsitz (3, 63, 163, 263, 363) erfolgt oder durch ein manuelles Drehen des Ventilkörpers (6, 18, 78, 178, 266, 366) gegen Ventilsitz (3, 63, 163, 263, 363) erfolgt, wobei bevorzugt das manuelle Drehen durch Bedienen eines Griff (16, 38, 76, 176, 298) am Ventilkörper (6, 18, 78, 178, 266, 366) erfolgt.

## Claims

1. A device for producing a knee spacer component (110, 210, 410) by curing bone cement paste (50, 51), wherein the knee spacer component (110, 210, 410) is provided in the medical field for temporarily replacing part of a knee joint comprising an articulating surface of the knee joint, the device having
a casting mold for molding the knee spacer component (110, 210, 410) from bone cement paste (50, 51);
a valve seat (3, 63, 163, 263, 363) which is connected to the casting mold, wherein the valve seat (3, 63, 163, 263, 363) has a regionally closed head side (4, 64, 164, 264, 364) with at least one first feed-through (5, 65, 165, 265, 365), wherein the at least one first feed-through (5, 65, 165, 265, 365) opens into the casting mold;
a valve body (6, 18, 78, 178, 266, 366) which is mounted so as to be rotatable relative to the valve seat (3, 63, 163, 263, 363) and which has a sealing face (7, 67, 167, 267), wherein the sealing face (7, 67, 167, 267) is oriented in the direction of the regionally closed head side (4, 64, 164, 264, 364) of the valve seat (3, 63, 163, 263, 363), wherein at least one second feed-through (8, 21, 68, 168, 268) is arranged in the sealing face (7, 67, 167, 267);
wherein the valve seat (3, 63, 163, 263, 363) and the valve body (6, 18, 78, 178, 266, 366) together form a valve, wherein the valve is reversibly transferable into an open position and a closed position by rotation of the valve body (6, 18, 78, 178, 266, 366) relative to the valve seat (3, 63, 163, 263, 363), wherein, in the open position of the valve, the at least one first feed-through (5, 65, 165, 265, 365) of the valve seat (3, 63, 163, 263, 363) and the at least one second feed-through (8, 21, 68, 168, 268) of the valve body (6, 18, 78, 178, 266, 366) are located above one another at least in places and provide a connection through the valve which is permeable to bone cement paste (50, 51), wherein, in the closed position of the valve, the at least one first feed-through (5, 65, 165, 265, 365) of the valve seat (3, 63, 163, 263, 363) is covered by the sealing face (7, 67, 167, 267) of the valve body (6, 18, 78, 178, 266, 366), wherein, in the closed position of the valve, the casting mold is closed in liquid-tight manner for bone cement paste (50, 51).

2. The device according to Claim 1, wherein
the valve seat (3, 63, 163, 263, 363) is connected to the casting mold so as not to be rotatable relative to the casting mold, preferably the valve seat (3, 63, 163, 263, 363) is connected fixedly and/or rigidly to the casting mold or is formed as one part with the casting mold, and/or
the valve is manually operable, preferably manually operable from outside the device, wherein the valve body (6, 18, 78, 178, 266, 366) is particularly preferably manually rotatable relative to the valve seat (3, 63, 163, 263, 363) and the valve is transferable by rotation from the closed position into the open position and from the open position into the closed position.

3. The device according to any one of the preceding claims, wherein,
in the closed position of the valve, the at least one first feed-through (5, 65, 165, 265, 365) of the valve seat (3, 63, 163, 263, 363) is covered by the sealing face (7, 67, 167, 267) of the valve body (6, 18, 78, 178, 266, 366), wherein the regionally closed head side (4, 64, 164, 264, 364) of the valve seat (3, 63, 163, 263, 363) and the sealing face (7, 67, 167, 267) of the valve body (6, 18, 78, 178, 266, 366) are preferably spaced apart from one another by a maximum of 2 mm, particularly preferably by a maximum of 1 mm and very particularly preferably by a maximum of 0.5 mm, and/or
the valve body (6, 18, 78, 178, 266, 366) is mounted so as to be rotatable about an axis of rotation relative to the valve seat (3, 63, 163, 263, 363), wherein the axis of rotation extends perpendicular to the sealing face (7, 67, 167, 267) of the valve body (6, 18, 78, 178, 266, 366) or wherein the axis of rotation extends along an axis of rotational symmetry of the sealing face (7, 67, 167, 267) of the valve body (6, 18, 78, 178, 266, 366).

4. The device according to any one of the preceding claims, wherein
the valve body (6, 18, 78, 178, 266, 366) has a port (11, 271) for liquid-tight connection of a bone cement cartridge (10) or of a handle of the device or is firmly connected to such a port (11, 271), wherein preferably
the device has an adapter element (9) which is connected or connectable to a bone cement cartridge (10), wherein the adapter element (9) is detachably and interlockingly connected or connectable to the port (11, 271), such that an interior of a bone cement cartridge (10) is connected or connectable permeably for bone cement paste (50, 51) via the adapter element (9) to the at least one second feed-through (8, 21, 68, 168, 268) in the valve body (6, 18, 78, 178, 266, 366), and/or
the port (11, 271) comprises, for liquid-tight connection of a bone cement cartridge (10) or a handle (14, 74, 174), an inner thread (24, 84, 184) in the valve body (6, 18, 78, 178, 266, 366) or an outer thread on the valve body (6, 18, 78, 178, 266, 366), wherein an adapter element (9) of the bone cement cartridge (10) or on the bone cement cartridge (10) preferably has a mating thread (26) matching the inner thread (24, 84, 184) or the outer thread, or the handle (14, 74, 174) of the device has a mating thread (19, 79, 179) matching the inner thread (24, 84, 184) or the outer thread.

5. The device according to any one of the preceding claims, wherein
the device has a bone cement cartridge (10) for mixing bone cement starting components and for delivering mixed bone cement paste (50, 51) from the bone cement cartridge (10) and preferably has a bone cement cartridge (10) for mixing polymethyl methacrylate bone cement starting components and for delivering mixed polymethyl methacrylate bone cement paste (50, 51) from the bone cement cartridge (10), wherein the bone cement cartridge (10) particularly preferably contains the bone cement starting components for producing the bone cement paste (50, 51) in mutually separate regions, and/or
the device has a handle (14, 74, 174) which is connectable to the valve body (6, 18, 78, 178, 266, 366) and with which the valve body (6, 18, 78, 178, 266, 366) is rotatable relative to the valve seat (3, 63, 163, 263, 363), wherein the handle (14, 74, 174) preferably has a cavity for receiving bone cement paste (50, 51), which cavity is connected to the at least one second feed-through (8, 21, 68, 168, 268) in liquid-permeable manner, and/or
the sum of all free openings of the at least one first feed-through (5, 65, 165, 265, 365) in the closed head side (4, 64, 164, 264, 364) is at most as large as the closed surface of the head side (4, 64, 164, 264, 364) and
the sum of all free openings of the at least one second feed-through (8, 21, 68, 168, 268) in the sealing face (7, 67, 167, 267) is at most as large as the closed surface of the sealing face (7, 67, 167, 267) and
the valve seat (3, 63, 163, 263, 363) has an inner thread (20, 80, 180) on the inside and the valve body (6, 18, 78, 178, 266, 366) has a matching outer thread (22, 82, 182) on the outside, such that the valve body (6, 18, 78, 178, 266, 366) is able to be screwed into the valve seat (3, 63, 163, 263, 363).

6. The device according to any one of the preceding claims, wherein
the casting mold has a trough-shaped mold (1, 61, 161, 361) with a cavity and a punch (2, 62, 162, 362),
wherein the punch (2, 62, 162, 362) is insertable or inserted into the cavity of the trough-shaped mold (1, 61, 161, 361) and the punch (2, 62, 162, 362) is axially displaceable in the cavity, and
wherein the trough-shaped mold (1, 61, 161, 361) preferably has a cavity bottom (32, 92, 192, 392), wherein the cavity bottom (32, 92, 192, 392) forms the contour of one or more articulating sliding surfaces (112, 212) of the knee spacer component (110, 210, 410), and
the valve seat (3, 63, 163, 263, 363) is arranged in the punch (2, 62, 162, 362), preferably is arranged at the end of a part of the punch (2, 62, 162, 362) which shapes a stem of the knee spacer component (110, 210, 410).

7. The device according to any one of the preceding claims, wherein
the at least one first feed-through (5, 65, 165, 265, 365) in the regionally closed head side (4, 64, 164, 264, 364) has the same size and shape as the at least one second feed-through (8, 21, 68, 168, 268) in the sealing face (7, 67, 167, 267) and/or
the at least one first feed-through (5, 65, 165, 265, 365) in the regionally closed head side (4, 64, 164, 264, 364) is two first feed-throughs (5, 65, 165, 265, 365) and the at least one second feed-through (8, 21, 68, 168, 268) in the sealing face (7, 67, 167, 267) is two second feed-throughs (8, 21, 68, 168, 268), wherein the two first feed-throughs (5, 65, 165, 265, 365) are preferably arranged in the valve seat (3, 63, 163, 263, 363) in quadrants arranged opposingly with regard to the axis of rotation of the valve body (6, 18, 78, 178, 266, 366) and the two second feed-throughs (8, 21, 68, 168, 268) are arranged in the sealing face (7, 67, 167, 267) in quadrants arranged opposingly with regard to the axis of rotation of the valve body (6, 18, 78, 178, 266, 366).

8. The device according to any one of the preceding claims, wherein
a collar is arranged on the sealing face (7, 67, 167, 267) of the valve body (6, 18, 78, 178, 266, 366), which collar rests on an edge of the valve seat (3, 63, 163, 263, 363) or a collar is arranged on the regionally closed head side (4, 64, 164, 264, 364) of the valve seat (3, 63, 163, 263, 363), which collar rests on an edge of the valve body (6, 18, 78, 178, 266, 366), and/or
a handgrip (16, 38, 76, 176, 298) is fastened or fastenable to the valve body (6, 18, 78, 178, 266, 366), which handgrip has at least one radial extent with regard to the axis of rotation of the valve body (6, 18, 78, 178, 266, 366),
wherein the handgrip (16, 38, 76, 176, 298) is preferably fastened or fastenable to an opposite side of the valve body from the sealing face, wherein the valve body (6, 18, 78, 178, 266, 366) may particularly preferably be rotated by a maximum of 90° relative to the valve seat (3, 63, 163, 263, 363), and/or
the valve body (6, 18, 78, 178, 266, 366) and the valve seat (3, 63, 163, 263, 363) are fabricated of plastics, in particular of thermoplastics, wherein the valve seat (3, 63, 163, 263, 363) is preferably formed as one part with the casting mold.

9. The device according to any one of the preceding claims, wherein
a latch element is arranged on the sealing face of the valve body (6, 18, 78, 178, 266, 366) and a mating latch element is arranged on the head side (4, 64, 164, 264, 364) of the valve seat (3, 63, 163, 263, 363) wherein the latch element is able to be brought into engagement with the mating latch element, wherein the latch element is positioned on the valve body (6, 18, 78, 178, 266, 366) such that rotation or unscrewing of the valve body (6, 18, 78, 178, 266, 366) from the valve seat (3, 63, 163, 263, 363) is limited to an angle of rotation of a maximum of 90° when the valve body (6, 18, 78, 178, 266, 366) is maximally put or screwed into the valve seat (3, 63, 163, 263, 363), and/or the casting mold is in two parts or multiple parts, wherein a gap through which air or gas can escape from the interior of the casting mold is present between at least two of the parts of the assembled casting mold, and/or
the device has a collecting vessel, wherein the valve is connected or connectable on the side remote from the casting mold to the collecting vessel for receiving excess bone cement paste (50, 51) which emerges from the casting mold through the valve in the open position or the valve is formed as one part with the collecting vessel.

10. A method for producing a knee spacer component (110, 210, 410) for temporarily replacing part of a knee joint comprising an articulating surface of the knee joint, wherein the method is carried out with a device according to any one of Claims 1 to 9, the method having the following chronological steps:
A) connecting a bone cement cartridge (10) to the device in liquid-tight manner;
B) injecting bone cement paste (50, 51) from the bone cement cartridge (10) through the valve in the open position into the casting mold;
C) rotating the valve body (6, 18, 78, 178, 266, 366) relative to the valve seat (3, 63, 163, 263, 363) and so transferring the valve into the closed position and shearing off the bone cement paste (50, 51) at the at least one first feed-through (5, 65, 165, 265, 365) in the regionally closed head side (4, 64, 164, 264, 364) of the valve seat (3, 63, 163, 263, 363) by rotation of the valve body (6, 18, 78, 178, 266, 366) relative to the valve seat (3, 63, 163, 263, 363);
D) detaching the bone cement cartridge (10) from the casting mold;
E) curing the bone cement paste (50, 51) in the casting mold; and
F) removing the resultant molded and cured knee spacer component (110, 210, 410) from the casting mold.

11. The method according to Claim 10, wherein
the following intermediate steps proceed after step D) and before step E):
D2) connecting a new bone cement cartridge (10) to the device in liquid-tight manner, wherein bone cement paste (50, 51) or starting components for producing the bone cement paste (50, 51) is/are present in the new bone cement cartridge (10);
D3) rotating the valve body (6, 18, 78, 178, 266, 366) relative to the valve seat (3, 63, 163, 263, 363) and so transferring the valve into the open position;
D4) injecting bone cement paste (50, 51) from the new bone cement cartridge (10) through the valve in the open position into the casting mold;
D5) rotating the valve body (6, 18, 78, 178, 266, 366) relative to the valve seat (3, 63, 163, 263, 363) and so transferring the valve into the closed position and shearing off the bone cement paste (50, 51) at the at least one first feed-through (5, 65, 165, 265, 365) in the regionally closed head side (4, 64, 164, 264, 364) of the valve seat (3, 63, 163, 263, 363) by rotation of the valve body (6, 18, 78, 178, 266, 366) relative to the valve seat (3, 63, 163, 263, 363); and
D6) detaching the new bone cement cartridge (10) from the casting mold;
wherein steps D2) to D6) are preferably repeated once or multiple times with in each case new bone cement cartridges (10) which contain bone cement paste (50, 51) or the starting components thereof until the casting mold is filled completely or as required with bone cement paste (50, 51).

12. The method according to either one of Claims 10 or 11, wherein,
for liquid-tight connection of the bone cement cartridge (10) and/or the new bone cement cartridge (10), said cartridge is connected to a port (11, 271) on the valve body (6, 18, 78, 178, 266, 366) of the device, wherein the bone cement cartridge (10) or the new bone cement cartridge (10) is rotated or screwed into the port (11, 271) and, for detaching the bone cement cartridge (10) and/or the new bone cement cartridge (10) from the port (11, 271), the bone cement cartridge (10) or the new bone cement cartridge (10) is rotated out of or unscrewed from the port (11, 271), and/or injection of the bone cement paste (50, 51) from the bone cement cartridge (10) or the new bone cement cartridge (10) proceeds by pushing a piston (46) into an interior of the bone cement cartridge (10), and/or
the casting mold has a trough-shaped mold (1, 61, 161, 361) and a punch (2, 62, 162, 362), wherein, before step E), the punch (2, 62, 162, 362) is pressed into the trough-shaped mold (1, 61, 161, 361) and as a result the knee spacer component (110, 210, 410) is molded from the bone cement paste (50, 51) in the casting mold, wherein, preferably on pressing the punch (2, 62, 162, 362) into the trough-shaped mold (1, 61, 161, 361), part of the bone cement paste (50, 51) is expelled from the casting mold through the valve in the open position and wherein particularly preferably the part of the bone cement paste (50, 51) which is pressed into a collecting vessel is that associated with the at least one second passage of the valve.

13. A method for producing a knee spacer component (110, 210, 410) for temporarily replacing part of a knee joint comprising an articulating surface of the knee joint, wherein the method is carried out with a device according to any one of Claims 1 to 9, the method having the following chronological steps:
A) producing a bone cement paste (50, 51);
B) filling a mixed bone cement paste (50, 51) into the casting mold;
C) compressing the casting mold and so expelling part of the bone cement paste (50, 51) from the casting mold through the valve in the open position;
D) rotating the valve body (6, 18, 78, 178, 266, 366) relative to the valve seat (3, 63, 163, 263, 363) and so transferring the valve into the closed position and shearing off the bone cement paste (50, 51) at the at least one first feed-through (5, 65, 165, 265, 365) in the regionally closed head side (4, 64, 164, 264, 364) of the valve seat (3, 63, 163, 263, 363) by rotation of the valve body (6, 18, 78, 178, 266, 366) relative to the valve seat (3, 63, 163, 263, 363);
E) curing the bone cement paste (50, 51) in the casting mold; and
F) removing the resultant molded and cured knee spacer component (110, 210, 410) from the casting mold.

14. The method according to Claim 13, wherein
a step B1) proceeds between steps B) and C):
closing the casting mold apart from the valve, and/or
the casting mold has a trough-shaped mold (1, 61, 161, 361) and a punch (2, 62, 162, 362), wherein, in step C), the punch (2, 62, 162, 362) is pressed into the trough-shaped mold (1, 61, 161, 361) and as a result the knee spacer component (110, 210, 410) is molded from the bone cement paste (50, 51) in the casting mold, wherein, preferably on pressing the punch (2, 62, 162, 362) into the trough-shaped mold (1, 61, 161, 361), part of the bone cement paste (50, 51) is expelled from the casting mold through the valve in the open position.

15. The method according to any one of Claims 10 to 14, wherein
the bone cement paste (50, 51) is mixed before step B) in the bone cement cartridge (10) from a monomer liquid and a cement powder, wherein, optionally before step D3) and preferably before step D2), the bone cement paste (50, 51) is preferably mixed in the new bone cement cartridge (10) from a monomer liquid and a cement powder, and/or
the valve body (6, 18, 78, 178, 266, 366) is rotated relative to the valve seat (3, 63, 163, 263, 363) by screwing the valve body (6, 18, 78, 178, 266, 366) in the valve seat (3, 63, 163, 263, 363) or by manually rotating the valve body (6, 18, 78, 178, 266, 366) relative to the valve seat (3, 63, 163, 263, 363), wherein manual turning preferably proceeds by operation of a handgrip (16, 38, 76, 176, 298) on the valve body (6, 18, 78, 178, 266, 366).

## Revendications

1. Dispositif de fabrication d'un composant d'espaceur de genou (110, 210, 410) par le durcissement de pâte de ciment osseux (50, 51), le composant d'espaceur de genou (110, 210, 410) étant conçu pour, dans le domaine médical, remplacer temporairement une partie d'une articulation de genou comprenant une surface articulaire de l'articulation de genou, le dispositif présentant
un moule de coulée pour la formation du composant d'espaceur de genou (110, 210, 410) à partir de pâte de ciment osseux (50, 51) ;
un siège de soupape (3, 63, 163, 263, 363), lequel est connecté au moule de coulée, le siège de soupape (3, 63, 163, 263, 363) présentant un côté de tête (4, 64, 164, 264, 364) fermé par zones comprenant au moins une première conduite (5, 65, 165, 265, 365), l'au moins une première conduite (5, 65, 165, 265, 365) débouchant à l'intérieur du moule de coulée ;
un corps de soupape (6, 18, 78, 178, 266, 366), lequel est monté tournant par rapport au siège de soupape (3, 63, 163, 263, 363) et lequel présente une surface d'étanchéité (7, 67, 167, 267), la surface d'étanchéité (7, 67, 167, 267) étant orientée dans la direction du côté de tête (4, 64, 164, 264, 364) fermé par zones du siège de soupape (3, 63, 163, 263, 363), au moins une deuxième conduite (8, 21, 68, 168, 268) étant disposée dans la surface d'étanchéité (7, 67, 167, 267) ;
le siège de soupape (3, 63, 163, 263, 363) et le corps de soupape (6, 18, 78, 178, 266, 366) formant ensemble une soupape, la soupape pouvant, par la rotation du corps de soupape (6, 18, 78, 178, 266, 366) par rapport au siège de soupape (3, 63, 163, 263, 363), être transférée de manière réversible dans une position ouverte et de manière réversible dans une position fermée, dans la position ouverte de la soupape l'au moins une première conduite (5, 65, 165, 265, 365) du siège de soupape (3, 63, 163, 263, 363) et l'au moins une deuxième conduite (8, 21, 68, 168, 268) du corps de soupape (6, 18, 78, 178, 266, 366) étant superposées au moins par zones et fournissant une connexion à travers la soupape pouvant être traversée par la pâte de ciment osseux (50, 51), dans la position fermée de la soupape l'au moins une première conduite (5, 65, 165, 265, 365) du siège de soupape (3, 63, 163, 263, 363) étant couverte par la surface d'étanchéité (7, 67, 167, 267) du corps de soupape (6, 18, 78, 178, 266, 366), le moule de coulée étant, dans la position fermée de la soupape, fermé de manière étanche aux liquides pour la pâte de ciment osseux (50, 51).

2. Dispositif selon la revendication 1,
le siège de soupape (3, 63, 163, 263, 363) étant connecté au moule de coulée de manière non rotative par rapport au moule de coulée, le siège de soupape (3, 63, 163, 263, 363) étant de préférence connecté de manière fixe et/ou rigide au moule de coulée ou réalisé d'une seule pièce avec le moule de coulée et/ou
la soupape pouvant être actionnée manuellement, pouvant de préférence être actionnée manuellement depuis l'extérieur du dispositif, le corps de soupape (6, 18, 78, 178, 266, 366) pouvant de manière particulièrement préférée tourner manuellement par rapport au siège de soupape (3, 63, 163, 263, 363) et la soupape pouvant être transférée, par la rotation, de la position fermée à la position ouverte et de la position ouverte à la position fermée.

3. Dispositif selon l'une quelconque des revendications précédentes,
l'au moins une première conduite (5, 65, 165, 265, 365) du siège de soupape (3, 63, 163, 263, 363) étant, dans la position fermée de la soupape, recouverte par la surface d'étanchéité (7, 67, 167, 267) du corps de soupape (6, 18, 78, 178, 266, 366), le côté de tête (4, 64, 164, 264, 364) fermé par zones du siège de soupape (3, 63, 163, 263, 363) et la surface d'étanchéité (7, 67, 167, 267) du corps de soupape (6, 18, 78, 178, 266, 366) étant de préférence espacés l'un de l'autre d'au maximum 2 mm, de manière particulièrement préférée espacés l'un de l'autre d'au maximum 1 mm, de manière tout particulièrement préférée espacés l'un de l'autre d'au maximum 0,5 mm et/ou
le corps de soupape (6, 18, 78, 178, 266, 366) étant monté de manière rotative autour d'un axe de rotation par rapport au siège de soupape (3, 63, 163, 263, 363), l'axe de rotation s'étendant perpendiculaire à la surface d'étanchéité (7, 67, 167, 267) du corps de soupape (6, 18, 78, 178, 266, 366) ou l'axe de rotation s'étendant le long d'un axe de symétrie en rotation de la surface d'étanchéité (7, 67, 167, 267) du corps de soupape (6, 18, 78, 178, 266, 366).

4. Dispositif selon l'une quelconque des revendications précédentes,
le corps de soupape (6, 18, 78, 178, 266, 366) présentant un raccord (11, 271) pour la connexion étanche aux liquides d'une cartouche de ciment osseux (10) ou d'une poignée du dispositif ou étant connecté de manière fixe à un tel raccord (11, 271), le dispositif présentant de préférence un élément adaptateur (9), lequel est ou peut être connecté à une cartouche de ciment osseux (10), l'élément adaptateur (9) étant ou pouvant être connecté de manière amovible et par liaison de forme au raccord (11, 271), de sorte qu'un espace intérieur d'une cartouche de ciment osseux (10) soit ou puisse être connecté par le biais de l'élément adaptateur (9) de manière perméable pour la pâte de ciment osseux (50, 51) à l'au moins une deuxième conduite (8, 21, 68, 168, 268) dans le corps de soupape (6, 18, 78, 178, 266, 366) et/ou
le raccord (11, 271) pour la connexion étanche aux liquides d'une cartouche de ciment osseux (10) ou d'une poignée (14, 74, 174) comprenant un filetage interne (24, 84, 184) dans le corps de soupape (6, 18, 78, 178, 266, 366) ou un filetage externe sur le corps de soupape (6, 18, 78, 178, 266, 366), un élément adaptateur (9) de la cartouche de ciment osseux (10) ou sur la cartouche de ciment osseux (10) présentant de préférence un contre-filetage (26) correspondant au filetage interne (24, 84, 184) ou au filetage externe ou la poignée (14, 74, 174) du dispositif présentant un contre-filetage (19, 79, 179) correspondant au filetage interne (24, 84, 184) ou au filetage externe.

5. Dispositif selon l'une quelconque des revendications précédentes,
le dispositif présentant une cartouche de ciment osseux (10) pour le mélange de composants initiaux de ciment osseux et pour la distribution de pâte de ciment osseux (50, 51) mélangée hors de la cartouche de ciment osseux (10), présentant de préférence une cartouche de ciment osseux (10) pour le mélange de composants initiaux de ciment osseux de poly(méthacrylate de méthyle) et pour la distribution de pâte de ciment osseux de poly(méthacrylate de méthyle) (50, 51) mélangée hors de la cartouche de ciment osseux (10), la cartouche de ciment osseux (10) contenant de manière particulièrement préférée les composants initiaux de ciment osseux pour la fabrication de la pâte de ciment osseux (50, 51) dans des zones séparées les unes des autres et/ou
le dispositif présentant une poignée (14, 74, 174), laquelle peut être connectée au corps de soupape (6, 18, 78, 178, 266, 366) et au moyen de laquelle le corps de soupape (6, 18, 78, 178, 266, 366) peut être tourné par rapport au siège de soupape (3, 63, 163, 263, 363), la poignée (14, 74, 174) présentant de préférence une cavité pour la réception de pâte de ciment osseux (50, 51), laquelle est connectée de manière perméable aux liquides à l'au moins une deuxième conduite (8, 21, 68, 168, 268) et/ou la somme de toutes les ouvertures libres de l'au moins une première conduite (5, 65, 165, 265, 365) dans le côté de tête (4, 64, 164, 264, 364) fermé étant au maximum de la même taille que la surface fermée du côté de tête (4, 64, 164, 264, 364) et
que la somme de toutes les ouvertures libres de l'au moins une deuxième conduite (8, 21, 68, 168, 268) dans la surface d'étanchéité (7, 67, 167, 267) étant au maximum de la même taille que la surface fermée de la surface d'étanchéité (7, 67, 167, 267) et
le siège de soupape (3, 63, 163, 263, 363) présentant un filetage interne (20, 80, 180) sur la face interne et le corps de soupape (6, 18, 78, 178, 266, 366) présentant un filetage externe (22, 82, 182) correspondant sur la face externe, de sorte que le corps de soupape (6, 18, 78, 178, 266, 366) puisse être vissé dans le siège de soupape (3, 63, 163, 263, 363).

6. Dispositif selon l'une quelconque des revendications précédentes,
le moule de coulée présentant un moule en forme d'auge (1, 61, 161, 361) comprenant une cavité et un piston (2, 62, 162, 362),
le piston (2, 62, 162, 362) étant ou pouvant être inséré dans la cavité du moule en forme d'auge (1, 61, 161, 361) et le piston (2, 62, 162, 362) pouvant être déplacé axialement dans la cavité et
le moule en forme d'auge (1, 61, 161, 361) présentant de préférence un fond de cavité (32, 92, 192, 392), le fond de cavité (32, 92, 192, 392) formant le contour d'une ou plusieurs surfaces de glissement (112, 212) articulaires du composant d'espaceur de genou (110, 210, 410) et
le siège de soupape (3, 63, 163, 263, 363) étant disposé dans le piston (2, 62, 162, 362), de préférence à l'extrémité d'une partie du piston (2, 62, 162, 362) formant une tige du composant d'espaceur de genou (110, 210, 410).

7. Dispositif selon l'une quelconque des revendications précédentes,
l'au moins une première conduite (5, 65, 165, 265, 365) ayant dans le côté de tête (4, 64, 164, 264, 364) fermé par zones les mêmes taille et forme que l'au moins une deuxième conduite (8, 21, 68, 168, 268) dans la surface d'étanchéité (7, 67, 167, 267) et/ou
l'au moins une première conduite (5, 65, 165, 265, 365) étant deux premières conduites (5, 65, 165, 265, 365) dans le côté de tête (4, 64, 164, 264, 364) fermé par zones et l'au moins une deuxième conduite (8, 21, 68, 168, 268) étant deux deuxièmes conduites (8, 21, 68, 168, 268) dans la surface d'étanchéité (7, 67, 167, 267), les deux premières conduites (5, 65, 165, 265, 365) étant de préférence disposées dans le siège de soupape (3, 63, 163, 263, 363) dans des quarts d'arc de cercle disposés l'un en face de l'autre par rapport à l'axe de rotation du corps de soupape (6, 18, 78, 178, 266, 366) et les deux deuxièmes conduites (8, 21, 68, 168, 268) étant disposées dans la surface d'étanchéité (7, 67, 167, 267) dans des quarts d'arc de cercle disposés l'un en face de l'autre par rapport à l'axe de rotation du corps de soupape (6, 18, 78, 178, 266, 366).

8. Dispositif selon l'une quelconque des revendications précédentes,
un épaulement étant disposé sur la surface d'étanchéité (7, 67, 167, 267) du corps de soupape (6, 18, 78, 178, 266, 366), lequel s'appuie sur un bord du siège de soupape (3, 63, 163, 263, 363) ou un épaulement étant disposé sur le côté de tête (4, 64, 164, 264, 364) fermé par zones du siège de soupape (3, 63, 163, 263, 363), lequel s'appuie sur un bord du corps de soupape (6, 18, 78, 178, 266, 366) et/ou
une poignée (16, 38, 76, 176, 298) étant ou pouvant être fixée sur le corps de soupape (6, 18, 78, 178, 266, 366), laquelle présente au moins une étendue radiale par rapport à l'axe de rotation du corps de soupape (6, 18, 78, 178, 266, 366), la poignée (16, 38, 76, 176, 298) étant ou pouvant de préférence être fixée sur une face du corps de soupape faisant face à la surface d'étanchéité, le corps de soupape (6, 18, 78, 178, 266, 366) pouvant de manière particulièrement préférée être tourné d'au maximum 90° par rapport au siège de soupape (3, 63, 163, 263, 363) et/ou
le corps de soupape (6, 18, 78, 178, 266, 366) et le siège de soupape (3, 63, 163, 263, 363) étant fabriqués en plastique, en particulier fabriqués en plastique thermoplastique, le siège de soupape (3, 63, 163, 263, 363) étant de préférence réalisé d'une seule pièce avec le moule de coulée.

9. Dispositif selon l'une quelconque des revendications précédentes,
un élément d'encliquetage étant disposé sur la surface d'étanchéité du corps de soupape (6, 18, 78, 178, 266, 366) et un élément de contre-encliquetage étant disposé sur le côté de tête (4, 64, 164, 264, 364) du siège de soupape (3, 63, 163, 263, 363), l'élément d'encliquetage pouvant être mis en prise avec l'élément de contre-encliquetage, l'élément d'encliquetage étant positionné sur le corps de soupape (6, 18, 78, 178, 266, 366) de telle façon qu'une extraction par rotation ou un dévissage du corps de soupape (6, 18, 78, 178, 266, 366) hors du siège de soupape (3, 63, 163, 263, 363) est limité à un angle de rotation d'au maximum 90° lorsque le corps de soupape (6, 18, 78, 178, 266, 366) est inséré ou vissé au maximum dans le siège de soupape (3, 63, 163, 263, 363) et/ou le moule de coulée étant en deux parties ou en plusieurs parties, une fente existant entre au moins deux des parties du moule de coulée composé, à travers laquelle de l'air ou du gaz peut s'échapper de l'intérieur du moule de coulée et/ou
le dispositif présentant un récipient collecteur, la soupape étant ou pouvant être connectée au récipient collecteur sur le côté opposé au moule de coulée pour la réception de la pâte de ciment osseux (50, 51) supplémentaire sortant du moule de coulée par la soupape en position ouverte ou la soupape étant réalisée d'une seule pièce avec le récipient collecteur.

10. Procédé de fabrication d'un composant d'espaceur de genou (110, 210, 410) pour le remplacement temporaire d'une partie d'une articulation de genou comprenant une surface articulaire de l'articulation de genou, le procédé étant exécuté au moyen d'un dispositif selon l'une quelconque des revendications 1 à 9, le procédé présentant les étapes chronologiques suivantes :
A) connexion étanche aux liquides d'une cartouche de ciment osseux (10) au dispositif ;
B) injection de pâte de ciment osseux (50, 51) de la cartouche de ciment osseux (10) à travers la soupape dans la position ouverte dans le moule de coulée ;
C) rotation du corps de soupape (6, 18, 78, 178, 266, 366) par rapport au siège de soupape (3, 63, 163, 263, 363) et ainsi transfert de la soupape dans la position fermée et cisaillement de la pâte de ciment osseux (50, 51) au niveau de l'au moins une première conduite (5, 65, 165, 265, 365) dans le côté de tête (4, 64, 164, 264, 364) fermé par zones du siège de soupape (3, 63, 163, 263, 363) par la rotation du corps de soupape (6, 18, 78, 178, 266, 366) par rapport au siège de soupape (3, 63, 163, 263, 363) ;
D) détachement de la cartouche de ciment osseux (10) du moule de coulée ;
E) durcissement de la pâte de ciment osseux (50, 51) dans le moule de coulée ; et
F) extraction du composant d'espaceur de genou (110, 210, 410) ainsi formé et durci du moule de coulée.

11. Procédé selon la revendication 10,
les étapes intermédiaires suivantes étant effectuées après l'étape D) et avant l'étape E) :
D2) connexion étanche aux liquides d'une nouvelle cartouche de ciment osseux (10) au dispositif, de la pâte de ciment osseux (50, 51) ou des composants initiaux pour la fabrication de la pâte de ciment osseux (50, 51) étant contenue ou contenus dans la nouvelle cartouche de ciment osseux (10) ;
D3) rotation du corps de soupape (6, 18, 78, 178, 266, 366) par rapport au siège de soupape (3, 63, 163, 263, 363) et ainsi transfert de la soupape dans la position ouverte ;
D4) injection de la pâte de ciment osseux (50, 51) de la nouvelle cartouche de ciment osseux (10) à travers la soupape dans la position ouverte dans le moule de coulée ;
D5) rotation du corps de soupape (6, 18, 78, 178, 266, 366) par rapport au siège de soupape (3, 63, 163, 263, 363) et ainsi transfert de la soupape dans la position fermée et
cisaillement de la pâte de ciment osseux (50, 51) au niveau de l'au moins une première conduite (5, 65, 165, 265, 365) dans le côté de tête (4, 64, 164, 264, 364) fermé par zones du siège de soupape (3, 63, 163, 263, 363) par la rotation du corps de soupape (6, 18, 78, 178, 266, 366) par rapport au siège de soupape (3, 63, 163, 263, 363) ; et D6) détachement de la nouvelle cartouche de ciment osseux (10) du moule de coulée ;
les étapes D2) à D6) étant de préférence répétées une fois ou plusieurs fois, avec respectivement de nouvelles cartouches de ciment osseux (10), lesquelles contiennent de la pâte de ciment osseux (50, 51) ou ses composants initiaux, jusqu'à ce que le moule de coulée soit rempli complètement ou selon les besoins de pâte de ciment osseux (50, 51).

12. Procédé selon l'une quelconque des revendications 10 ou 11,
pour la connexion étanche aux liquides de la cartouche de ciment osseux (10) et/ou de la nouvelle cartouche de ciment osseux (10), celle-ci étant connectée à un raccord (11, 271) sur le corps de soupape (6, 18, 78, 178, 266, 366) du dispositif, la cartouche de ciment osseux (10) ou la nouvelle cartouche de ciment osseux (10) étant insérée par rotation ou vissée dans le raccord (11, 271) et, pour le détachement de la cartouche de ciment osseux (10) et/ou de la nouvelle cartouche de ciment osseux (10) du raccord (11, 271), la cartouche de ciment osseux (10) ou la nouvelle cartouche de ciment osseux (10) étant extraite par rotation ou dévissée du raccord (11, 271) et/ou
l'injection de la pâte de ciment osseux (50, 51) de la cartouche de ciment osseux (10) ou de la nouvelle cartouche de ciment osseux (10) étant effectuée par pression d'un piston (46) dans un espace intérieur de la cartouche de ciment osseux (10) et/ou
le moule de coulée présentant un moule en forme d'auge (1, 61, 161, 361) et un piston (2, 62, 162, 362), avant l'étape E) le piston (2, 62, 162, 362) étant pressé dans le moule en forme d'auge (1, 61, 161, 361) et ainsi le composant d'espaceur de genou (110, 210, 410) étant formé dans le moule de coulée à partir de la pâte de ciment osseux (50, 51), lors de la pression du piston (2, 62, 162, 362) dans le moule en forme d'auge (1, 61, 161, 361), une partie de la pâte de ciment osseux (50, 51) étant de préférence pressée hors du moule de coulée à travers la soupape en position ouverte et, de manière particulièrement préférée, la partie de la pâte de ciment osseux (50, 51) étant pressée dans un récipient collecteur, lequel est connecté à l'au moins un deuxième passage de la soupape.

13. Procédé de fabrication d'un composant d'espaceur de genou (110, 210, 410) pour le remplacement temporaire d'une partie d'une articulation de genou comprenant une surface articulaire de l'articulation de genou, le procédé étant exécuté au moyen d'un dispositif selon l'une quelconque des revendications 1 à 9, le procédé présentant les étapes chronologiques suivantes :
A) fabrication d'une pâte de ciment osseux (50, 51) ;
B) remplissage d'une pâte de ciment osseux (50, 51) mélangée dans le moule de coulée ;
C) compression du moule de coulée et ainsi pression d'une partie de la pâte de ciment osseux (50, 51) hors du moule de coulée à travers la soupape en position ouverte ;
D) rotation du corps de soupape (6, 18, 78, 178, 266, 366) par rapport au siège de soupape (3, 63, 163, 263, 363) et ainsi transfert de la soupape dans la position fermée et cisaillement de la pâte de ciment osseux (50, 51) au niveau de l'au moins une première conduite (5, 65, 165, 265, 365) dans le côté de tête (4, 64, 164, 264, 364) fermé par zones du siège de soupape (3, 63, 163, 263, 363) par la rotation du corps de soupape (6, 18, 78, 178, 266, 366) par rapport au siège de soupape (3, 63, 163, 263, 363) ;
E) durcissement de la pâte de ciment osseux (50, 51) dans le moule de coulée ; et
F) extraction du composant d'espaceur de genou (110, 210, 410) ainsi formé et durci du moule de coulée.

14. Procédé selon la revendication 13,
une étape B1) étant effectuée entre les étapes B) et C) :
fermeture du moule de coulée jusqu'à la soupape et/ou
le moule de coulée présentant un moule en forme d'auge (1, 61, 161, 361) et un piston (2, 62, 162, 362), dans l'étape C) le piston (2, 62, 162, 362) étant pressé dans le moule en forme d'auge (1, 61, 161, 361) et ainsi le composant d'espaceur de genou (110, 210, 410) étant formé dans le moule de coulée à partir de la pâte de ciment osseux (50, 51), lors de la pression du piston (2, 62, 162, 362) dans le moule en forme d'auge (1, 61, 161, 361), une partie de la pâte de ciment osseux (50, 51) étant de préférence pressée hors du moule de coulée à travers la soupape en position ouverte.

15. Procédé selon l'une quelconque des revendications 10 à 14,
avant l'étape B) la pâte de ciment osseux (50, 51) dans la cartouche de ciment osseux (10) étant mélangée à partir d'un liquide de monomère et d'une poudre de ciment, de préférence le cas échéant, avant l'étape D3), de préférence avant l'étape D2), la pâte de ciment osseux (50, 51) dans la nouvelle cartouche de ciment osseux (10) étant mélangée à partir d'un liquide de monomère et d'une poudre de ciment et/ou
la rotation du corps de soupape (6, 18, 78, 178, 266, 366) par rapport au siège de soupape (3, 63, 163, 263, 363) étant effectuée par un vissage du corps de soupape (6, 18, 78, 178, 266, 366) dans le siège de soupape (3, 63, 163, 263, 363) ou par une rotation manuelle du corps de soupape (6, 18, 78, 178, 266, 366) par rapport au siège de soupape (3, 63, 163, 263, 363), la rotation manuelle étant de préférence effectuée par l'actionnement d'une poignée (16, 38, 76, 176, 298) sur le corps de soupape (6, 18, 78, 178, 266, 366).
